# EUROPEAN PATENT SPECIFICATION

(11) **EP 2 575 433 B1**
(45) Date of publication and mention of the grant of the patent: **07.09.2016**
(21) Application number: 11729247.4
(22) Date of filing: 31.05.2011
(51) Int. Cl.: A23L 7/25, A01H 5/10, C12C 1/18, C12C 12/00, A23L 2/38, A23L 7/20, C12N 9/02, C12N 15/82, C12N 9/10

(54) **ENERGY SAVING BREWING METHOD**
ENERGIE SPARENDES BRAU VERFAHREN
PROCÉDÉ DE BRASSAGE ÉCONOMIQUE EN ÉNERGIE

(30) Priority: 03.06.2010 DK 201070243
(43) Date of publication of application: 10.04.2013
(73) Proprietor: Carlsberg Breweries A/S, 1799 Copenhagen V (DK); Heineken Supply Chain B.V., 1017 ZD Amsterdam (NL)
(72) Inventor: KNUDSEN, Søren, DK-2760 Måløv (DK); RIIS, Preben, DK-3500 Værløse (DK); SKADHAUGE, Birgitte, DK-3460 Birkerød (DK); BECH, Lene, Mølskov, DK-2765 Smørum (DK); OLSEN, Ole, DK-2300 Copenhagen S (DK)
(74) Representative: Høiberg A/S
(86) International application number: PCT/DK2011/050186
(87) International publication number: WO 2011/150933

(56) References cited:
- WO-A1-2004/011591
- WO-A2-2010/063288
- WO-A2-2010/075860
- US-A1- 2005 204 437
- US-A1- 2009 041 890
- ROUCK G DE ET AL: "Sufficient formation and removal of dimethyl sulfide (DMS) without classic wort boiling", BREWING SCIENCE,, vol. 63, 1 January 2001 (2001-01-01), pages 31-40, XP009147905, ISSN: 1613-2041
- HIROTA N ET AL: "Brewing performance of malted lipoxygenase-1 null barley and effect on the flavor stability of beer", CEREAL CHEMISTRY, AMERICAN ASSOCIATION OF CEREAL CHEMISTS. MINNEAPOLIS, US, vol. 83, no. 3, 1 May 2006 (2006-05-01), pages 250-254, XP009124704, ISSN: 0009-0352, DOI: DOI:10.1094/CC-83-0250
- HOTZEL H ET AL: "Recovery and characterization of residual DNA from beer as a prerequisite for the detection of genetically modified ingredients", EUROPEAN FOOD RESEARCH AND TECHNOLOGY, SPRINGER VERLAG, HEIDELBERG, DE, vol. 209, no. 3-4, 1 January 1999 (1999-01-01), pages 192-196, XP002635202, ISSN: 1438-2377, DOI: 10.1007/S002170050478

## Description

### FIELD OF INVENTION

The present invention relates to energy saving methods for preparing barley based beverages, for example malt based beverages, such as beer. The invention furthermore relates to barley plants useful in the disclosed methods. In particular, the invention describes barley plants with combined traits of null-lipoxygenase-1 (null-LOX-1), null-lipoxygenase-2 (null-LOX-2) and null-S-adenosylmethionine:methionine S-methyltransferase (also denoted null-S-methionine (Met)- S-methyltransferase or null-MMT) in one plant, i.e. a null-LOX-1-null-LOX-2-null-MMT barley plant (herein also interchangeably denoted double-null-LOX-null-MMT), which is particularly useful for the energy saving methods to prepare barley based beverages described herein.

### BACKGROUND OF INVENTION

### Malting facility and brewhouse

Barley - Hordeum vulgare, L. - is a diploid cereal that is widely grown in different climates for food and beverage production. Beverages based on said plant are produced in large quantities, employing highly energy consuming methods, for example in the malting and brewhouse facilities for kiln drying and wort boiling operations, respectively.

Malting usually involves steeping of barley kernels to promote germination, followed by kiln drying at elevated temperatures, which makes the process particularly energy consuming. Key objectives of kiln drying include: (i) germination termination; (ii) drying the germinated barley grains; (iii) enzyme denaturation, particularly lipase and LOX enzymes in wild-type barley; and (iv) conversion of the dimethyl sulphide (DMS) precursors (DMSP), principally consisting of S-methyl-Met (SMM), into volatile DMS [after kiln drying of a normal pale malt, the DMSP content is on average 4 ppm in dry weight (see Technology Brewing and Malting, Kunze, 2004, VLB Berlin, pp. 158-162)]. Certain enzyme activities are preserved during kiln drying (e.g. amylase, protease, etc).

In the brewhouse, there is in general terms consumed about half of the energy in the brewing process, corresponding to an energy load in the range 48,000-83,000 kJ/hL (Modern brewhouse technology, Brauwelt International 2004, p. 410-412). Most of the energy is consumed in the process of wort boiling, the goal of which is in general to provide: (i) coagulation of protein; (ii) inactivation of enzymes; (iii) wort sterilization; (iv) extraction of hop compounds; (v) isomerization of α-acids; and (vi) evaporation of unwanted volatile compounds, e.g. the sulphury and stale off-flavours DMS and trans-2-nonenal (T2N), respectively.

Wort is traditionally boiled for at least 50-60 min to allow for an overall evaporation of at least 10-15% (see Technology Brewing and Malting, Kunze, 2004, VLB Berlin, Chapter 11), but now often improved by technological means to 6-8%. It has also been attempted to reduce energy consumption even further, for example by minimizing evaporation to as little as 3-4%, combined with a stripping process that extracts unwanted volatile compounds into injected steam (see e.g. Bonacchelli et al., 2007). It is thought that the levels of unwanted, volatile compounds in wort makes it difficult to further reduce, or even eliminate, evaporation.

### T2N

T2N - a volatile C₉ alkenal with a boiling point of 88°C - was characterized in 1970 as the molecule conferring the cardboard-like off-flavour in beer (Jamieson and Gheluwe, 1970). Since the taste-threshold level for T2N in humans is extremely low, previously determined to be around 0.7 nM or 0.1 ppb (Meilgaard, 1975), products with even minute levels of the aldehyde are perceived as aged. However, levels of T2N are generally very low in fresh beer (Lermusieau et al., 1999), indicating that processes during ageing promote liberation of free T2N from corresponding adducts (Nyborg et al., 1999). A subsequent observation revealed correlation between the T2N potential of wort and formed free T2N after product storage (Kuroda et al., 2005).

Kiln drying and wort boiling represent separate processing steps that may be targets for manipulation in order to achieve reduced levels of T2N in barley-based beverages. While kiln drying at high temperatures confer inactivation of the enzymes involved in T2N formation, such as lipases and LOXs (see e.g. Technology Brewing and Malting, Kunze, 2004, VLB Berlin, p. 162), free T2N also can be removed by wort boiling.

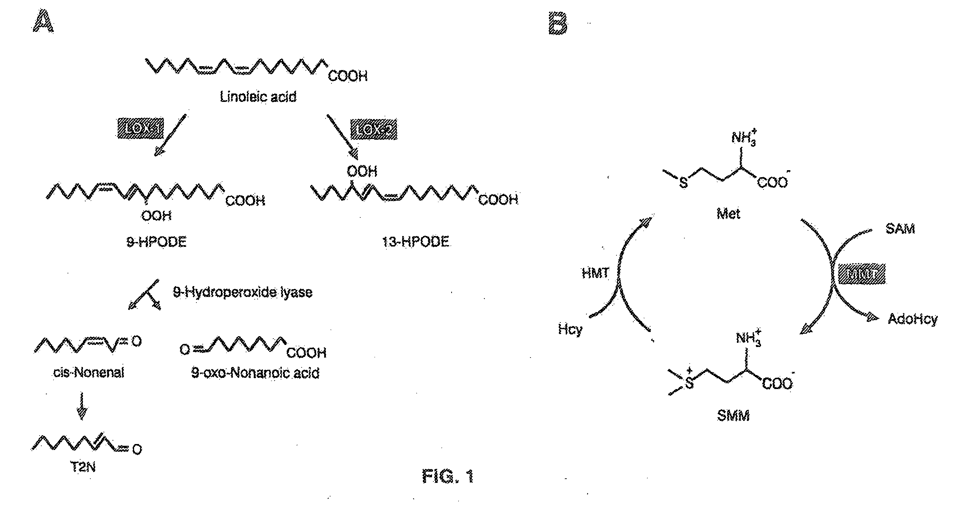

The barley kernel contains three LOX enzymes - known as LOX-1, LOX-2, and LOX-3 (van Mechelen et al., 1999). The major activity of LOX-1 catalyzes the formation of 9-hydroperoxy octadecadienoic acid (9-HPODE; see **FIG. 1A** for a partial overview of the LOX pathway) - a precursor of both T2N and trihydroxy octadecenoic acids (THAs) - from linoleic acid. LOX-2 mainly catalyzes the conversion of linoleic acid to 13-HPODE, which is further metabolized to hexanal, a C₆ aldehyde with a ∼0.4-ppm-high taste threshold (Meilgaard, supra). Given LOX-2's very little 9-HPODE-forming activity, several reports have noted that T2N is produced via a biochemical pathway involving conversion of linoleic acid to 9-HPODE, initially catalyzed by LOX-1, followed by cleavage of 9-HPODE through 9-hydroperoxide lyase action (see, for example, Kuroda et al., 2003, 2005; Noodermeer et al., 2001).

With regard to the above-mentioned properties of LOX-1, said enzyme is a useful target for inactivation in efforts to lower T2N levels in barley-based products, actually substantiated by the following two observations: (i) Kuroda et al. (2005) suggested a correlation between LOX-1 activity and the wort T2N potential, primarily because LOX-2 has been considered inferior with respect to formation of the T2N potential in said wort. However, there appears to be no correlation between the overall LOX activity in malt and the wort T2N potential; (ii) methods have been described to obtain reduced activity of LOX-1 in barley.

Several different barley plants have been developed that share the property of partially or totally reduced LOX-1 activity. For example, barley kernels and barley plants having a low LOX-1 activity were disclosed in PCT Application WO 02/053721 to Douma, A.C. et al., while WO 2005/087934 to Breddam, K. et al. focused on two different barley mutants deficient in LOX-1 activity - a splice site mutant and a mutant with a premature translational stop codon. In addition, EP 1609866 to Hirota, N. et al. described a barley plant with no LOX-1 activity, which was identified by screening a collection of barley landraces.

### DMS

In barley-based beverages - as also in many vegetables and foodstuffs, including tea, cocoa, milk, wines, spirits (such as rum), sweet corn, and numerous cooked vegetables - DMS adds prominent odour and flavour notes to the product. Depending on beer type, DMS levels typically can reach 150 ppb (150 µg/L), with said compound often contributing to undesirable "cooked vegetable" or "cabbage-like" flavours. In this regard, it is not only important that the sensory threshold is around 30-45 µg/L (Meilgaard, 1982), but also that the DMS-derived flavour remains unnoticed at levels <10 ppb.

The aforementioned kiln drying and wort boiling processing steps in beer production influence the levels of DMS in barley-based beverages, primarily because both of said processes may induce chemical conversion of SMM to DMS (see e.g. Technology Brewing and Malting, Kunze, 2004, VLB Berlin, p. 160). Due to the latter compound's boiling point of only 37-38°C, a major part of the DMS will simply evaporate to the atmosphere. However, when duration or vigor of wort boiling is inadequate to convert residual SMM, DMS may continue to form as the wort cools - with high probability of transfer to the beer.

SMM represents almost all, possibly all, of the DMSP pool in germinating barley kernels, synthesized by the action of functional components of the SMM cycle **(****FIG. 1 B)****.** Here, MMT catalyzes the transfer of a methyl group from S-adenosyl-Met (AdoMet) to Met, forming SMM. The latter compound can in turn serve as methyl donor for Met synthesis from homocysteine (Hcy), a reaction catalyzed by the enzyme Hcy-S-methyltransferase (HMT).

In the scientific literature, it has been considered an opportunity to regulate SMM synthesis by using, for example, antisense technology (McElroy and Jacobsen, 1995). However, no guidance was provided on relevant target genes to antisense. Despite that, it was expected that the likelihood of a positive outcome was questionable as large reductions in SMM levels could be harmful to barley growth and development. Alternative solutions for obtaining lower level of SMM were not discussed by McElroy and Jacobsen (supra). And also, as discussed in detail herein below, antisense technologies have not been successfully applied in barley to completely abolish gene expression.

Technological methods for reducing the level of DMS in beer have been developed. Thus, AU 38578/93 described a method of reducing DMS levels in malt, comprising steam treatment of said malt. In patent application US 2006/0057684 to Bisgaard-Frantzen, H. et al. was described brewing methods comprising heat treatment of mash at ≥70°C. And in U.S. Patent No. 5,242,694 to Reuther, H. was noted methods for preparing low-carbohydrate beer, wherein the methods comprise extensive boiling of wort followed by washing of said wort with carbon dioxide (CO₂). However, all of the aforementioned treatments are known to consume high levels of energy, possibly altering malt or wort characteristics.

### Mutant barley plants

Unfortunately no methods are available for preparing transgenic barley plants that completely lack expression of a given protein. In general for barley, application of antisense techniques lead to transgenic plants still expressing some of the protein in question (see for example Robbins et al. 1998; Stahl et al., 2004; Hansen et al., 2007). Also, effective methods for preparing specific mutations using chimeric RNA/DNA or site directed mutagenesis have not been developed for use in barley plants. In line with this, and despite intensive efforts, inventors of the present application remain unaware of any published example on successful oligonucleotide-directed gene targeting in barley. Although not pursued in barley, lida and Terada (2005) note that oligonucleotide-directed gene targeting has been tested in maize, tobacco and rice-but in all cases with the herbicide-resistance gene acetolactate synthase (ALS) as a target. According to the conclusion by lida and Terada (supra), it remains to be established whether the above-mentioned strategy, with appropriate modifications, is applicable to genes other than those directly selectable, such as the ALS genes. Although not yet substantiated, targeted mutagenesis using zinc-finger nucleases represents another tool that potentially could allow future investigations in basic plant biology or modifications in crop plants (Durai et al., 2005; Tzfira and White, 2005; Kumar et al., 2006). Also in this case, however, mutagenesis has not been pursued or successfully applied in barley.

Nonetheless, barley mutants may be prepared by random mutagenesis using irradiation or chemical treatment, such as incubating kernels for 12 h to over night with a solution of sodium azide (NaN₃). An example concerns barley kernels mutagenized through the use of NaN₃, and subsequently screened for high levels of free phosphate in an effort to screen for low-phytate mutants (Rasmussen and Hatzack, 1998); a total of 10 mutants out of 2,000 screened kernels were identified. Although far from always possible, finding a particular mutant after NaN₃ treatment is dependent on persistence and an effective screening method.

### Sustainability

In a world seeking solutions to its energy, environmental and food challenges, one focus of society is to limit or reduce atmospheric CO₂ concentrations - especially focusing on CO₂ emissions from industrial systems. The principal reason is that an increase in the concentration of a greenhouse gas causes a change in Earth's energy balance, with CO₂ being the largest single contributor. As a consequence of the widespread concern about climate change, and also based on economic rationales and constraints, breweries may play an active part by using energy as efficiently as possible, and by reducing greenhouse gas emissions from operations more effectively. Until now, the focal point has been on technological means to solve the above-mentioned issues on sustainability.

### SUMMARY OF INVENTION

Heat treatment methods have been described for reduction of LOX activity and DMS levels of DMS. Said treatment was generally undertaken during malting and/or preparation of wort, meaning that products of LOX activity were allowed to accumulate in barley until undertaking of the heat treatment. Analysis of barley revealed that significant amounts of products of LOX activity were present in barley, even prior to malting (Wackerbauer and Meyna, 2002). It is apparent that heat treatment methods are highly energy consuming.

In particular, the invention provides methods for preparing a barley based beverage with low levels of one or more off-flavours and precursors thereof (notably low levels of DMS and T2N and precursors thereof), wherein the method involves reduced energy input, the method comprising the steps of:
(i) providing a barley plant, or part thereof, wherein said barley plant comprises:
   (a) a first mutation that results in a total loss of functional LOX-1; and
   (b) a second mutation resulting in a total loss of functional LOX-2; and
   (c) a third mutation resulting in a total loss of functional MMT;
(ii) optionally malting at least part of said barley, thereby obtaining malted barley;
(iii) mashing said barley and/or malted barley and optionally additional adjuncts, thereby obtaining a wort;
(iv) heating said wort optionally in the presence of additional ingredient(s), wherein at the most 4% of the wort volume is evaporated, thereby obtaining heated wort;
(v) processing said heated wort into a beverage;
thereby preparing a barley derived beverage with low levels of one or more off-flavours and precursors thereof.

It is also an objective of the present invention to provide barley plants suitable for use in the disclosed methods. Thus, it is an objective of the invention to provide agronomically useful barley plants comprising all of the three different traits, i.e. a null-LOX-1-null-LOX-2-null-MMT barley plant (herein also denoted "double-null-LOX-null-MMT"). Selection of a useful barley plant concerns not only plant vigour, but also the combined lack of LOX-1, LOX-2 and MMT enzymic activities, utilizing biochemical assays as described in detail herein below. The barley plants according to the present invention may be introduced into any suitable breeding scheme, such as selfing, backcrossing, crossing to populations, and the like.

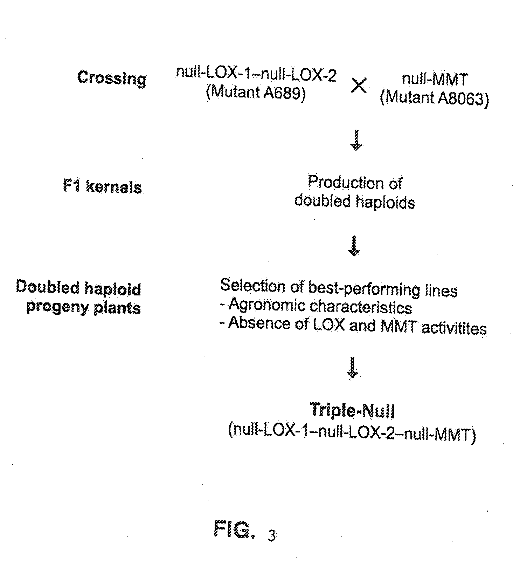

A way to accelerate the process of plant breeding comprises the initial multiplication of generated mutants by application of tissue culture and regeneration techniques. As described in Example 3 and schematically shown in FIG. 3, a traditional barley breeding scheme was employed to generate a barley plant with the double-null-LOX-null-MMT trait from a double-null-LOX barley plant and a null-MMT plant. Thus, another aspect is to provide cells, which upon growth and differentiation produce barley plants having the double-null-LOX-null-MMT trait. For example, breeding may involve traditional crossings, preparing fertile anther-derived, doubled haploid plants using tissue culturing such as anther culture or microspore culturings.

The present invention discloses that reduced energy input for kiln drying is achievable through application of null-LOX grains, because there is no need to inactivate endogenous LOX enzyme. The double-null-LOX-null-MMT mutant described by the present invention can be utilized for production of a raw material lacking the corresponding enzyme activities, making the mutant of interest to achieve lower energy consumption during kiln drying in the malting facility, but also in the brewhouse because of reduced heat input during wort boiling.

From a beer or beverage quality standpoint, there is also a need for a double-null-LOX-null-MMT raw material, in order to functionally eliminate or drastically reduce T2N and DMS levels in the products.

In addition to the above-mentioned, potential properties of a double-null-LOX-null-MMT malt, a corresponding barley could be useful for the production of off-flavour-low barley beer, here defined as beer produced through omission of the malting process, but instead providing a mash consisting of numerous external enzymes (e.g. Ondea Pro, an enzyme mixture produced by Novozymes). As addressed herein below in Example 7, it was surprising by the present inventors to find that wort produced by mashing unmalted double-null-LOX-null-MMT barley raw material contained very low levels of T2N and DMS off-flavours, while that from wild-type barley was surprisingly high in DMS levels despite that the barley kernels had not undergone germination. Accordingly, some DMS precursors (DMSP) must be present in the dry, mature barley kernel - a new, not-yet described property, which is explored in beer products of the instant application. Accordingly, double-null-LOX-null-MMT grains are useful in the production of barley-brewed wort and beer in order to minimize levels of T2N and DMS in fresh and aged beer products.

### DESCRIPTION OF DRAWINGS

**FIG. 1** presents simplified overviews of the barley biochemical pathways leading to formation of T2N **(A),** and SMM **(B).** Enzymes absent in Triple-Null barley are highlighted in black boxes. Enzyme abbreviations are those defined in the instant application. **FIG. 1** **B** shows selected components of the SMM cycle in which SMM is synthesized by methyl transfer from S-adenosylmethionine (SAM) to methionine (Met), catalyzed by the enzyme Met-*S*-methyltransferase (MMT). SMM can in turn serve as methyl donor for Met synthesis from homocysteine (Hcy), in a reaction catalyzed by the enzyme Hcy-S-methyltransferase (HMT). The illustration shows how the essentially irreversible reactions are connected. Each turn of the cycle is futile as it consumes and then regenerates two Mets while converting ATP to adenosine, PPi and Pi (not shown).

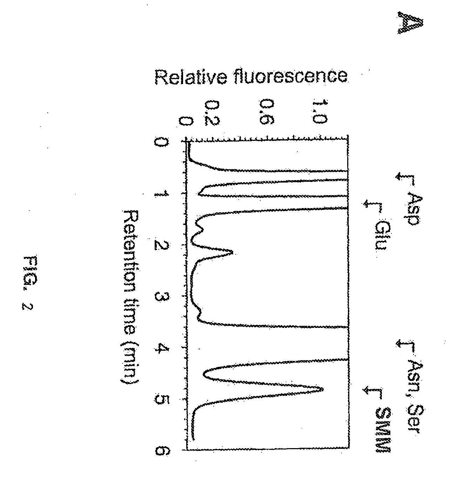

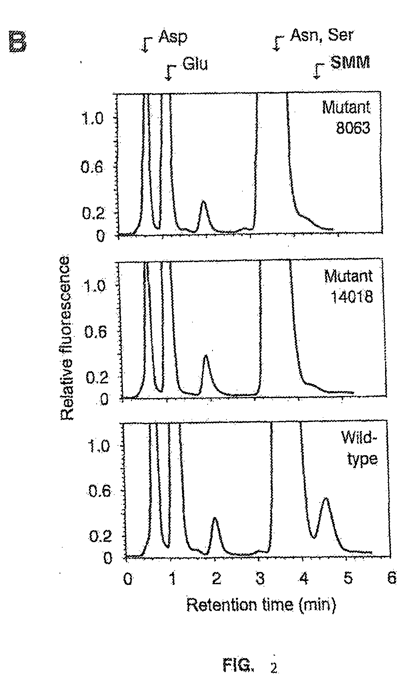

**FIG. 2** shows results of HPLC experiments to verify the null-MMT phenotype of Mutant 8063 and Mutant 14018. **(A)** An example on HPLC-based separation of an extract from shoots of cv. Prestige, showing elution of aspartic acid (Asp), glutamic acid (Glu), asparagine (Asn), serine (Ser) and SMM. Fluorescence of OPA-derivatized extracts of barley shoots were excited at 340 nm and emission measured at 450 nm. **(B)** HPLC-based separation of extracts from the indicated mutants and wild-type cv. Sebastian. Separation of components in a mutant extract provided a chromatogram without SMM-specific peaks.
**FIG. 3** illustrates the work flow from crossing of null-LOX-1-null-LOX-2 (double-null-LOX) and null-MMT barley plants to the Triple-Null barley.

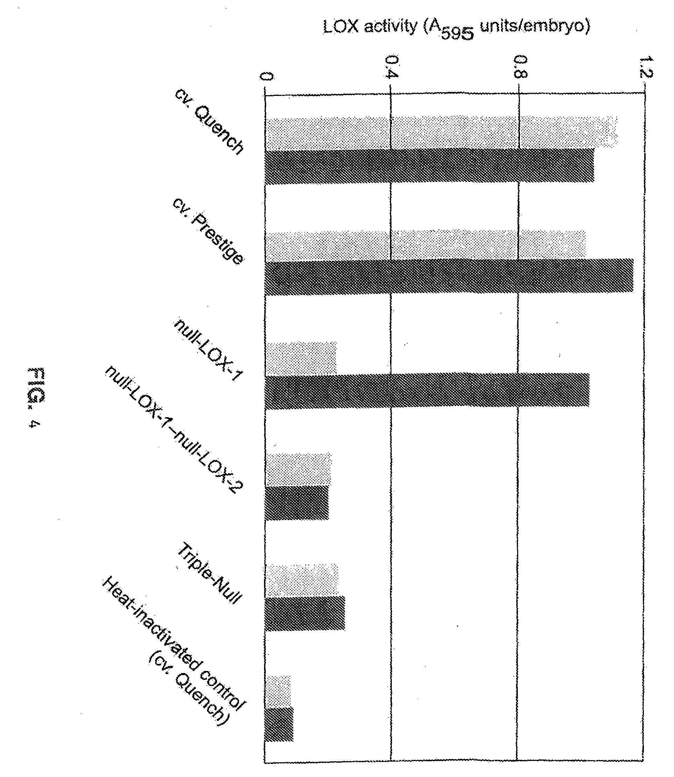

**FIG. 4** highlights LOX activities in samples derived from the indicated plants. Results of total LOX activity determinations in mature barley kernels are shown with grey bars, while those in black indicate LOX-2 activities in germinating embryos. Very low LOX activities were observed in both null-LOX-1-null-LOX-2 and Triple-Null plants.

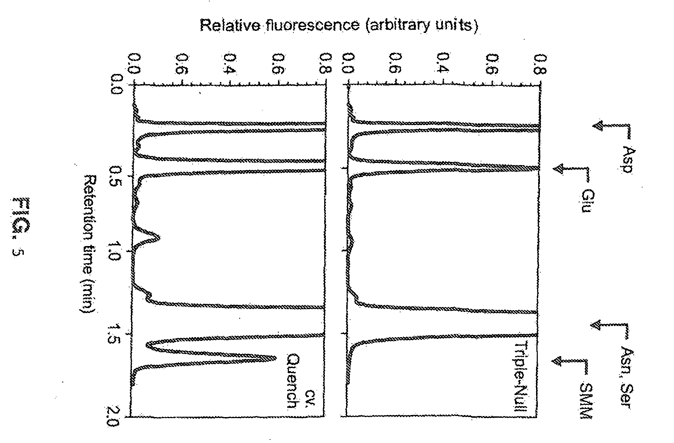

**FIG. 5** illustrates that germinated Triple-Null plants cannot synthesize SMM (upper panel in which the corresponding peak is absent in the UPLC chromatogram), while said compound is easily detectable as a corresponding chromatogram peak in an extract of germinating wild-type barley, cv. Quench (lower panel). The elution positions of selected amino acids are indicated.

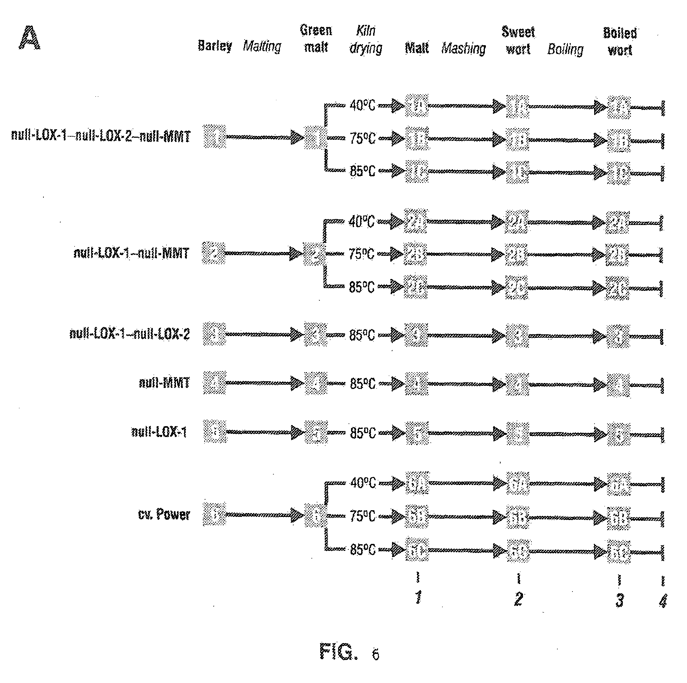

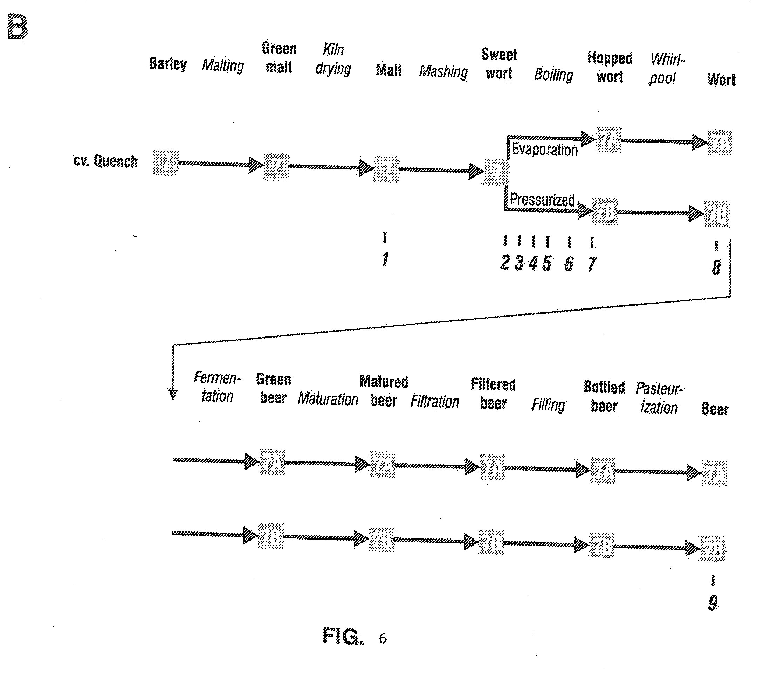

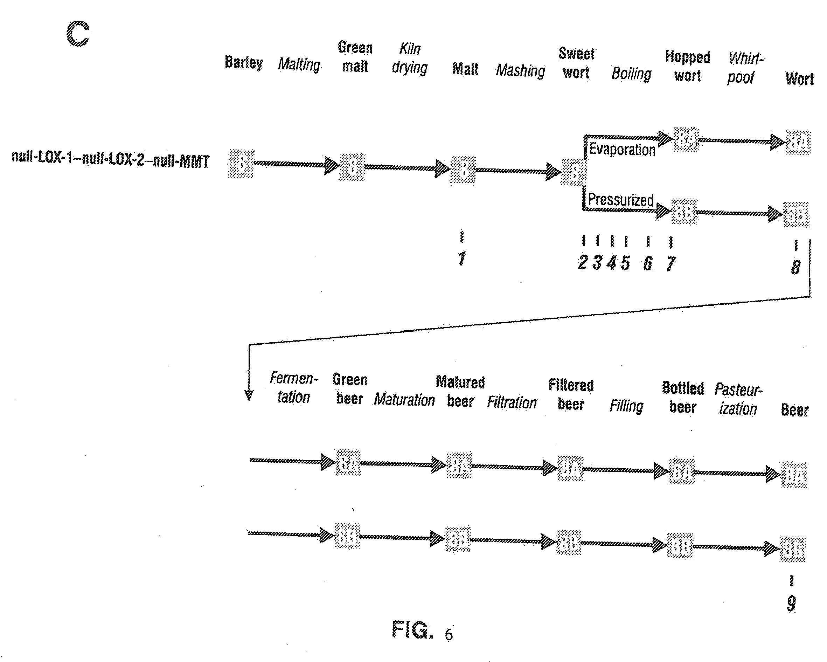

**FIG. 6** details graphically the workflow of micro-maltings and -mashings **(A),** in addition to that of pilot-maltings, -mashings and -brewings of kernels from wild-type barley **(B)** and the Triple-Null mutant (C). The flow of individual samples (marked in grey boxes) is illustrated with arrows. Start, intermediate and end products are marked in bold font type on top of the list; processes are in italics font type. In **(A),** numbers in italics below the flow list refer to sampling points for determination of levels of free T2N and its precursors (2 and 4), and DMSP and DMS (1, 2, 3), where measuring point 4 represents cooled-down, heated wort. For micro-mashing of barley flour, samples were measured at sampling points 2, 3 and 4. In **(B)** and **(C),** DMSP, DMS, T2N precursor and free T2N levels were determined at all sampling points.

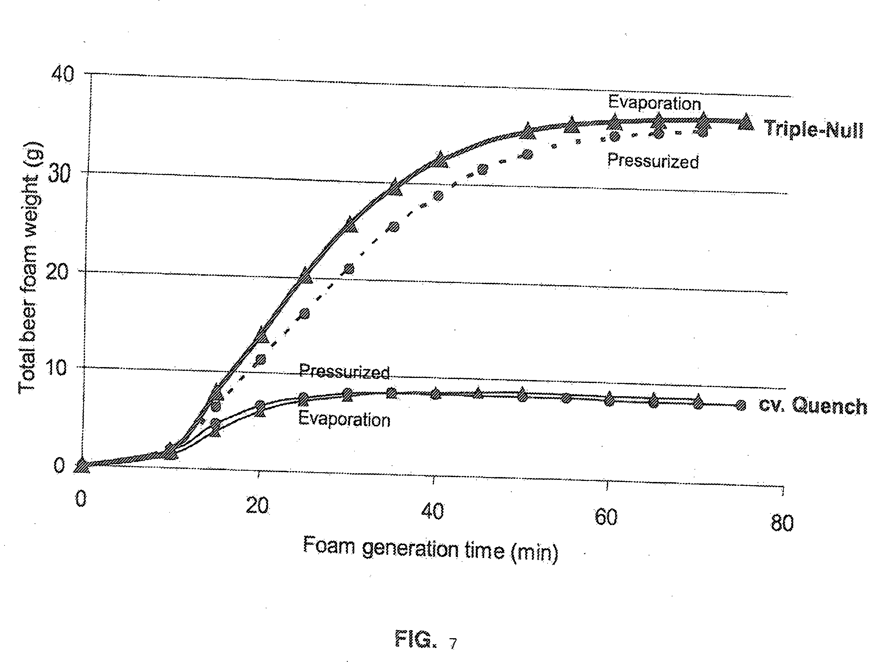

**FIG. 7** shows that beer made of Triple-Null malt generates around four times more beer foam than that of a beer brewed on malt of wild-type cv. Quench - irrespective of pressurized or normal wort boiling.

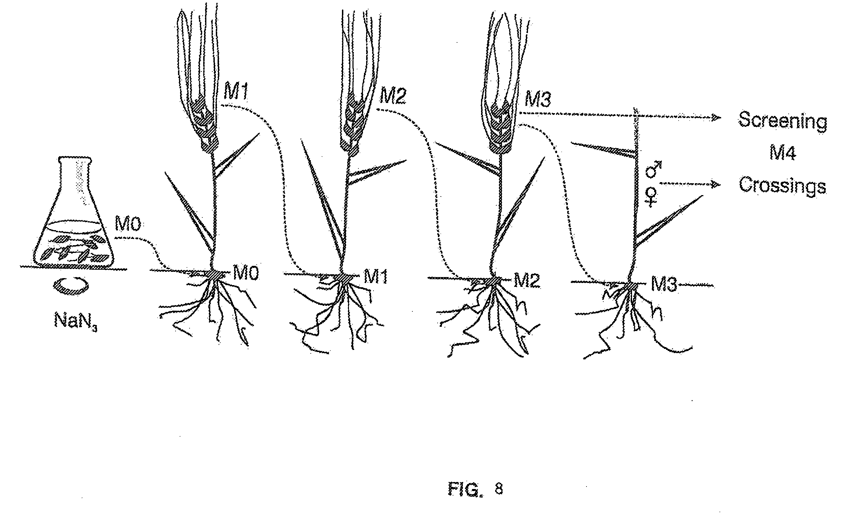

**FIG. 8** provides one example on how NaN₃-mutagenized barley kernels may be propagated. Kernels of generation M0 grow into plants that develop kernels of generation M1. These may be sown for development into M1 plants, which produce new kernels of generation M2. Next, M2 plants grow and set kernels of generation M3. Kernels of generation M3 may be allowed to germinate, for example for analysis of coleoptiles of the germinated M3 plants. Additionally, flowers derived from kernels of M3 plants may be used in crossings with barley lines or cultivars to obtain plants of generation M4. A similar figure is presented as FIG. 1A in PCT patent application WO 2005/087934 to Breddam, K. et al.

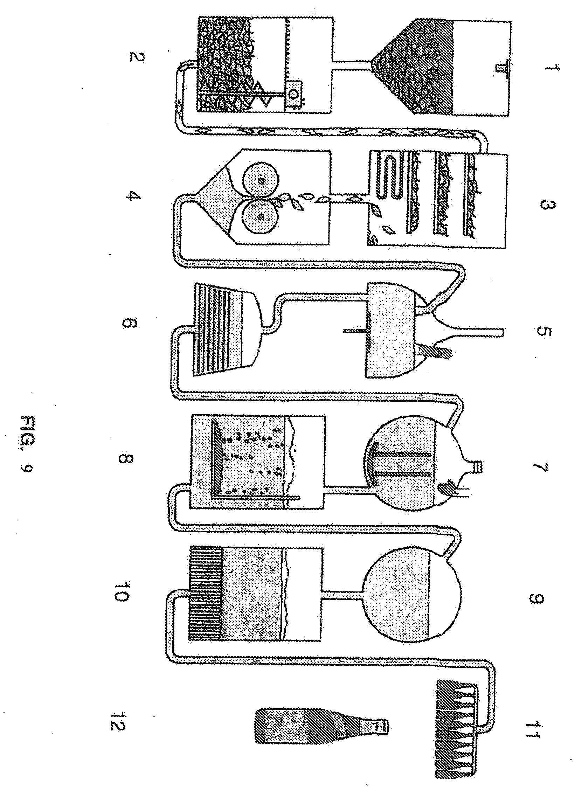

**FIG. 9** shows a simplified, schematic overview of a preferred beer production process, including steeping of barley grain (1), malting (2), kiln drying (3), milling of the dried malt (4), mashing (5), filtration (6), wort boiling in the presence of added hops (7), fermentation in the presence of yeast (8), beer maturation (9), beer filtration (10), packaging, such as the packaging into bottles, cans, or the like (11), and labeling (12). The individual processes can be grouped into sections comprising malt production (1-3), wort production (4-7), fermentation (8-9), and the preparation of the finished beer (10-12). Although a preferred method is illustrated, other methods may be envisaged that omit some of the depicted steps (filtration may, for example, be omitted, or hops may not be added - or additional steps may be added, such as addition of adjuncts, sugars, syrups, or carbonate).

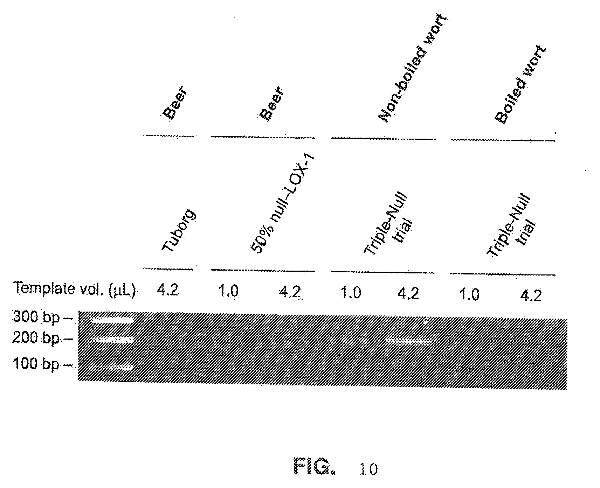

**FIG. 10** illustrates how to identify null-LOX-1 derived DNA fragments in beer (mixture comprising 50% null-LOX-1) and wort samples (Triple-Null) produced using null-LOX-1 raw materials, but not in beer produced using flour of a mixture of normal malt and wild-type barley (Tuborg). Template volume of the concluding PCR amplification is indicated.

### DETAILED DESCRIPTION OF THE INVENTION

### Definitions

In the description, figures, and tables that follow, a number of terms are used. In order to provide the specifications and claims, including the scope to be given such terms, the following definitions are provided:

As used herein, "a" can mean one or more, depending on the context in which it is used.

The term "agronomic trait" describes a phenotypic or genetic trait of a plant that contributes to the performance or economic value of said plant. Such traits include disease resistance, insect resistance, virus resistance, nematode resistance, drought tolerance, high salinity tolerance, yield, plant height, days to maturity, kernel grading (i.e. kernel size fractionation), kernel nitrogen content and the like.

The term "barley" in reference to the process of making barley based beverages, such as beer, particularly when used to describe the malting process, means barley kernels. In all other cases, unless otherwise specified, "barley" means the barley plant (Hordeum vulgare, L.), including any breeding line or cultivar or variety, whereas part of a barley plant may be any part of a barley plant, for example any tissue or cells.

In the process of "barley brewing", wort is prepared by incubating an extract of un-malted barley with an enzyme mixture that hydrolyzes the barley components. A wort produced by barley brewing may be denoted "barley wort", or "barley-brewed" wort.

By "disease resistance" is intended that the plants avoid disease symptoms, which are the outcome of plant-pathogen interactions. In this way, pathogens are prevented from causing plant diseases and the associated disease symptoms. Alternatively, the disease symptoms caused by the pathogen are minimized or reduced, or even prevented.

"DMSP" as used herein is an abbreviation for DMS precursor or DMS potential, i.e. the molecules that can be converted to DMS during the production of beverages. SMM represents the major part of, if not all, DMSP. The level of DMSP is defined herein as the quantity of DMS that can be generated from DMSP in the specified plant material, or product thereof, by boiling at alkaline conditions for 1 h. As defined herein, 1 ppb DMSP can be converted to 1 ppb DMS.

As used herein the term "double null-LOX" refers to a first mutation resulting in a total loss of functional LOX-1 and a second mutation resulting in a total loss of functional LOX-2. Thus, a "double null-LOX barley plant" is a barley plant comprising a first mutation resulting in a total loss of functional LOX-1 and a second mutation resulting in a total loss of functional LOX-2. Similarly, "double null-LOX kernels" are kernels having a first mutation resulting in a total loss of functional LOX-1 and a second mutation resulting in a total loss of functional LOX-2.

The term "double-null-LOX-null-MMT" refers to a first mutation resulting in a total loss of functional LOX-1 and a second mutation resulting in a total loss of functional LOX-2 and a third mutation resulting in a total loss of functional MMT. Thus, a "double-null-LOX-null-MMT barley plant" is a barley plant comprising a first mutation resulting in a total loss of functional LOX-1 and a second mutation resulting in a total loss of functional LOX-2 and a third mutation resulting in a total loss of functional MMT. Similarly, "double-null-LOX-null-MMT kernels" are kernels having a first mutation resulting in a total loss of functional LOX-1 and a second mutation resulting in a total loss of functional LOX-2 and a third mutation resulting in a total loss of functional MMT. An example of a useful double-null-LOX-null-MMT is denoted "Triple-Null" and described in the Examples herein below.

A "cereal" plant, as defined herein, is a member of the Graminae plant family, cultivated primarily for their starch-containing seeds or kernels. Cereal plants include, but are not limited to barley (Hordeum), wheat (Triticum), rice (Oryza), maize (Zea), rye (Secale), oat (Avena), sorghum (Sorghum), and Triticale, a rye-wheat hybrid.

By "encoding" or "encoded", in the context of a specified nucleic acid, is meant comprising the information for translation into the specified protein. A nucleic acid or polynucleotide encoding a protein may comprise non-translated sequences, e.g. introns, within translated regions of the nucleic acid, or may lack such intervening non-translated sequences, e.g. in cDNA. The information by which a protein is encoded is specified by the use of codons.

As used herein, "expression" in the context of nucleic acids is to be understood as the transcription and accumulation of sense mRNA or antisense RNA derived from a nucleic acid fragment. "Expression" used in the context of proteins refers to translation of mRNA into a polypeptide.

The term "gene" means the segment of DNA involved in producing a polypeptide chain; it includes regions preceding and following the coding region (promoter and terminator). Furthermore, plant genes generally consist of exons interrupted by introns. After transcription into RNA, the introns are removed by splicing to generate a mature messenger RNA (mRNA). The "splice sites" between exons and introns are typically determined by consensus sequences acting as splice signals for the splicing process, consisting of a deletion of the intron from the primary RNA transcript and a joining or fusion of the ends of the remaining RNA on either side of the excised intron. In some cases, alternate or different patterns of splicing can generate different proteins from the same single stretch of DNA. A native gene may be referred to as an "endogenous gene".

As used herein, "heterologous" in reference to a nucleic acid is a nucleic acid that originates from a foreign species, or, if from the same species, is substantially modified from its native form in composition and/or genomic locus by deliberate human intervention.

The term "germination" as used herein means the beginning or resumption of growth by a barley kernel in various compositions, such as normal soil as found in nature. Thus, a germinating embryo is an embryo undergoing germination. Germination can also take place in the soil of pots placed in growth chambers an the like, or for example take place on wet filter paper placed in standard laboratory Petri dishes or during malting (for example, in steep tanks or germination boxes of the malting factory). Germination is generally understood to include hydration of the kernels, swelling of the kernels and inducing growth of the embryo. Environmental factors affecting germination include moisture, temperature and oxygen level. Root and shoot development is observed.

The term "kernel" is defined to comprise the cereal caryopsis, also denoted internal seed, the lemma and palea. In most barley varieties, the lemma and palea adhere to the caryopsis and are a part of the kernel following threshing. However, naked barley varieties also occur. In these, the caryopsis is free of the lemma and palea and threshes out free as in wheat. The terms "kernel" and "grain" are used interchangeably herein.

"Grain development" refers to the period starting with fertilization of the egg cell by a pollen cell. During fertilization metabolic reserves - e.g. sugars, oligosaccharides, starch, phenolics, amino acids, and proteins - are deposited, with and without vacuole targeting, to various tissues in the kernel (grain) endosperm, testa, aleurone, and scutellum, thus leading to kernel (grain) enlargement, kernel (grain) filling, and ending with kernel (grain) desiccation.

The term "total loss of functional ..." refers to the lack of the given enzymatic activity. Thus a barley plant with a "total loss of functional LOX-1 and LOX-2 is a barley plant with no detectable LOX-1 and LOX-2 activities. In the context of the present invention, LOX-1 and LOX-2 activities are determined by an assay procedure determining the formation of 9-HPODE and 13-HPODE from linoleic acid, even though LOX-1 and LOX-2 enzymes may have other activities. Preferably, formation of 9-HPODE and 13-HPODE from linoleic acid is determined as described in Example 4 of international patent PCT/DK2009/050355 (published as WO 2010/075860), see below. The activity should be determined using protein extracts of germinated embryos. In the context of the present invention, generation of a chromatogram peak corresponding to less than 5%, preferably less than 3% of the 9-HPODE peak of the standard shown in **FIG. 5A** of international patent PCT/DK2009/050355 (published as WO 2010/075860), and/or a peak corresponding to less than 5%, preferably less than 3% of the 13-HPODE peak of the standard shown in **FIG. 5A** of international patent PCT/DK2009/050355 (published as WO 2010/075860), when using linoleic acid as substrate, is considered as no detectable LOX-1 and LOX-2 activity, when using the assay described in Example 4 of international patent PCT/DK2009/050355 (published as WO 2010/075860), see below. Molecular approaches to obtain a total loss of functional LOX comprise generation of mutations that either cause a total absence of transcripts for said enzyme, total absence of the corresponding encoded enzyme, or mutations that totally inactivate the encoded enzyme. Similarly, a barley plant with "total loss of functional MMT" refers to the lack of the MMT enzymatic activity, i.e. a barley plant with no detectable MMT activity when using the assay described in Example 2 herein below. Alternatively, MMT activity of a barley plant is determined by isolating MMT cDNA of said barley and determining whether the protein encoding by said cDNA is capable of catalyzing transfer of a methyl group from SAM to Met, thereby forming SMM.

The term "LOX-1 activity" refers to the enzymatic activity of the barley LOX-1 enzyme. Particularly, in the context of the present invention, "LOX-1 activity" is the enzyme-catalyzed dioxygenation of linoleic acid to 9-HPODE, and to a much lesser extent 13-HPODE. Even though the LOX-1 enzyme is capable of catalyzing other reactions, for the purpose of determining the activity of LOX-1 according to the present invention, only the 9- and 13-HPODE forming activities should be considered. **FIG. 1A** outlines the biochemical pathway wherein linoleic acid is converted to 9-HPODE.

The term "LOX-2 activity" refers to the enzymatic activity of the barley LOX-2 enzyme. Particularly, in the context of the present invention, "LOX-2 activity" is the enzyme-catalyzed dioxygenation of linoleic acid to 13-HPODE, and to a much lesser extent 9-HPODE. Even though the LOX-2 enzyme is capable of catalyzing other reactions, for the purpose of determining the activity of LOX-2 according to the present invention, only the 13- and 9-HPODE forming activities should be considered. **FIG. 1** **A** outlines the biochemical pathway wherein linoleic acid is converted to 13-HPODE.

The term "malt beverage" and the term "malt based beverage" refer to beverages prepared using malt, preferably beverages prepared by a method including a step of incubating malt with hot water. The terms are used interchangeably herein. Malt beverages may, for example, be beer or maltinas. Beer of the instant application may also be produced using "barley brewing" (cf. abovementioned definition).

The term "fermented malt beverage" refers to malt beverages, which have been fermented, i.e. incubated with yeast.

"Malting" is a special form of germination of barley kernels taking place under controlled environmental conditions - including, but not limited to steep tanks and germination boxes of the malting factory. In accordance with the process of the present invention, malting begins to occur during and/or after the barley kernels have been steeped. The malting process may be stopped by drying of the barley kernels, for example, in a kiln drying process. Kiln drying is usually performed at elevated temperatures, but an advantage of the present invention is that kiln drying may be performed at lower temperatures. In case that the malt has not been kiln dried, it is denoted "green malt". A malt composition prepared from double-null-LOX-null-MMT barley is understood to comprise double-null-LOX-null-MMT malt, such as pure double null-LOX-null-MMT malt, or any blend of malt comprising double null-LOX-null-MMT malt. Preferably, said composition is prepared only from double-null-LOX-null-MMT malt. Malt may be processed, for example, by milling and thus referred to as "milled malt" or "flour".

"Mashing" is the incubation of milled malt in water. Mashing is preferably performed at a specific temperature, and in a specific volume of water. The temperature and volume of water are of importance, as these affect the rate of decrease of enzyme activity derived from the malt, and hence especially the amount of starch hydrolysis that can occur; protease action may also be of importance. Mashing can occur in the presence of adjuncts, which is understood to comprise any carbohydrate source other than malt, such as, but not limited to, barley (including e.g. double-null-LOX-null-MMT barley), barley syrups, or maize, or rice - either as whole kernels or processed products like grits, syrups or starch. All of the aforementioned adjuncts may be used principally as an additional source of extract (syrups are typically dosed during wort heating). The requirements for processing of the adjunct in the brewery depend on the state and type of adjunct used, and in particular on the starch gelatinization or liquefaction temperatures. If the gelatinization temperature is above that for normal malt saccharification, then starch is gelatinized and liquefied before addition to the mash.

The term "MMT activity" refers to the enzymatic activity of the barley methionine S-methyltransferase enzyme. In the context of the present invention, "MMT activity" is the MMT-catalyzed methylation at the sulfur atom of Met to yield SMM. Even though the MMT enzyme may be capable of catalyzing other reactions, for the purpose of determining the activity of MMT according to the present invention, only the SMM-forming activity should be considered. **FIG. 1** **B** outlines the biochemical reactions wherein Met is converted to SMM by methylation.

"Mutations" include deletions, insertions, substitutions, transversions, and point mutations in the coding and noncoding regions of a gene. Deletions may be of the entire gene, or of only a portion of the gene, wherein the noncoding region preferably is either the promoter region, or the terminator region, or introns. Point mutations may concern changes of one base or one base pair, and may result in stop codons, frameshift mutations or amino acid substitutions. With reference to **FIG. 8** herein-which presents an overview on how grains of mutated barley may be propagated in a breeding program - grains of generation M3, and directly propagated grains thereof, or of any subsequent generation, including the plants thereof, may be termed "raw mutants". Further, still with reference to **FIG. 8** herein, the term "breeding line" refers to grains of generation M4, and any subsequent generation, including the plants thereof, which may be the result of a cross to a cultivar plant, or the result of a cross to another breeding line with a separate, specific trait.

The term "null-LOX" refers to the presence of a mutation in a LOX-encoding gene, causing a total loss of function of the encoded LOX enzyme (either LOX-1 or LOX-2 or both LOX-1 and LOX-2). Mutations that generate premature termination (nonsense) codons in a gene encoding LOX represent only one mechanism by which total loss of functional LOX can be obtained. Molecular approaches to obtain total loss of functional LOX comprise the generation of mutations that cause a total absence of transcripts for said enzyme, or mutations that cause total inactivation of the encoded enzyme. "Null-LOX" with reference to a plant refers to a plant having a mutation resulting in a total loss of functional LOX enzyme.

As used herein, the term "null-MMT" refers to a total loss of functional S-adenosylmethionine : methionine S-methyltransferase enzyme (also denoted methionine S-methyl transferase enzyme). Thus, a "null-MMT barley plant" is a barley plant comprising a mutation in the gene encoding MMT that results in a total loss of functional MMT. Similarly, "null-MMT kernels" are kernels comprising a mutation in the gene encoding MMT, resulting in a total loss of functional MMT.

"Operably linked" is a term used to refer to the association of two or more nucleic acid fragments on a single polynucleotide, so that the function of one is affected by the other. For example, a promoter is operably linked with a coding sequence when it is capable of affecting the expression of that coding sequence, i.e. that the coding sequence is under the transcriptional control of the promoter. Coding sequences can be operably linked to regulatory sequences in sense or antisense orientation.

"PCR" or "polymerase chain reaction" is well known by those skilled in the art as a technique used for the amplification of specific DNA segments (U.S. Pat. Nos. 4,683,195 and 4,800,159 to Mullis, K.B. et al.).

"Plant" or "plant material" includes plant cells, plant protoplasts, and plant cell tissue cultures from which barley plants can be regenerated - including plant calli, and plant cells that are intact in plants, or parts of plants, such as embryos, pollen, ovules, flowers, kernels, leaves, roots, root tips, anthers, or any part or product of a plant. Plant material may in one embodiment be plant cells from which no barley plants can be regenerated.

By the term "plant product" is meant a product resulting from the processing of a plant or plant material. Said plant product may thus, for example, be malt, wort, a fermented or non-fermented beverage, a food, or a feed product.

As used herein, "recombinant" in reference to a protein is a protein that originates from a foreign species, or, if from the same species, is substantially modified from its native form in composition by deliberate human intervention.

A "specialist beer taste panel" within the meaning of the present application is a panel of specialists extensively trained in tasting and describing beer flavours, with special focus on aldehydes, papery taste, old taste, esters, higher alcohols, fatty acids and sulfur components. Although a number of analytical tools exist for evaluating flavour components, the relative significance of flavour-active components are difficult to assess analytically. However, such complex properties can be evaluated by taste specialists. Their continuous training includes tasting and evaluation of standard beer samples.

By the term "splice site" is meant the boundaries between exons and introns of a gene. Thus, a splice site may be the border going from exon to intron (called a "donor site"), or the border separating intron from exon (denoted "acceptor site"). A splice site in plants typically comprises consensus sequences. The 5' end of an intron, in general, consists of a conserved GT dinucleotide (GU in the mRNA), and the 3' end of an intron usually consists of a conserved dinucleotide AG. The 5' splice site of an intron thus comprises the 5' end of an intron, and the 3' splice site comprises the 3' end of an intron. Preferably, within the context of the present invention, the splice site of an intron is either the 5' splice site consisting of the most 5' dinucleotides of the intron (which in general is GT), or the 3' splice site consisting of the most 3' dinucleotides of the intron (which in general is AG).

Unless otherwise noted, "T2N" means trans-2-nonenal (T2N) in the free form. T2N is sometimes also referred to as 2-E-nonenal.

By the term "T2N potential" is described the chemical substances which have the capacity to release T2N, or be converted into T2N, in one or more reactions. In the present context, the T2N potential is defined as the concentration of T2N released into a solution, e.g. wort or beer, during incubation for 2 h at 100°C, pH 4.0. In practical terms, the starting T2N concentration is determined, after which the solution is incubated for 2 h at 100°C, pH 4.0, followed by determination of the T2N concentration. The difference between the starting and the end T2N concentration is denoted the T2N potential. The thermal, acidic treatment causes liberation of T2N from the T2N potential, e.g. from "T2N adducts", the latter term used to describe T2N conjugated to one or more substances, including, but not limited to protein(s), sulphite, cellular debris, cell walls, or the like. In general, T2N adducts per se are not sensed by humans as off-flavours. However, T2N released from said T2N adducts may give rise to an off-flavour.

"Tissue culture" indicates a composition comprising isolated cells of the same or different types, or a collection of such cells organized into parts of a plant - including, for example, protoplasts, calli, embryos, pollen, anthers, and the like.

As used herein, "transgenic" includes reference to a cell that has been modified by the introduction of a heterologous nucleic acid, or that the cell is derived from a cell so modified. Thus, for example, transgenic cells express genes that are not found in an identical form within the native form of the cell, or express native genes that are otherwise abnormally expressed, under-expressed, or not expressed at all as a result of deliberate human intervention. The term "transgenic" as used herein in reference to plants, particularly barley plants, does not encompass the alteration of the cell by methods of traditional plant breeding - e.g. NaN₃-based mutagenesis, or by naturally occurring events without deliberate human invention.

"Wild barley", Hordeum vulgare ssp. spontaneum, is considered the progenitor of contemporary cultivated forms of barley. The transition of barley from a wild to a cultivated state is thought to have coincided with domestication of the plant into "barley landraces". These are genetically more closely related to modern cultivars than wild barley.

The term "wild-type" barley refers to a conventionally generated barley plant. Preferably, the term refers to the barley plant from which the barley plants of the instant invention have been derived, i.e the parental plants. Wild-type barley kernels are generally available from, for example, seed companies as "cultivars" or "varieties"-i.e. those genetically similar kernels that are listed by national plant breeding organizations. Despite the availability of several null-LOX-1 barley cultivars (e.g. cvs. Chamonix and Charmay), but for the purpose of a better understanding of the instant invention, all null-LOX-1, null-LOX-2, double-null-LOX and double-null-LOX-null-MMT plants are herein considered mutant plants, and not wild-type plants. The notations "cultivar" and "variety" are used interchangeably herein.

By the term "wort" is meant a liquid extract of malt, such as milled malt, or green malt, or milled green malt. In barley brewing, wort may also be prepared by incubating an extract of un-malted barley with an enzyme mixture that hydrolyzes the barley components. In addition to said malt or barley-derived extracts, the liquid extract may be prepared from malt and additional components, such as additional starch-containing material partly converted into fermentable sugars. The wort is in general obtained by mashing, optionally followed by "sparging", in a process of extracting residual sugars and other compounds from spent grains after mashing with hot water. Sparging is typically conducted in a lauter tun, a mash filter, or another apparatus to allow separation of the extracted water from spent grains. The wort obtained after mashing is generally referred to as "first wort", while the wort obtained after sparging is generally referred to as the "second wort". If not specified, the term wort may be first wort, second wort, or a combination of both. During conventional beer production, wort is boiled together with hops, however the present invention provides methods for reducing boiling or avoiding boiling of wort. Wort without hops, may also be referred to as "sweet wort", whereas wort boiled/heated with hops may be referred to as "boiled wort".

### Methods for preparing a barley based beverage

The present invention relates to methods for preparing a barley based beverage with low levels of off-flavours and precursors thereof, wherein the methods involve reduced energy input. The off-flavours are as described herein below, but preferably the off-flavours are T2N and DMS. The methods involve use of a barley plant, which preferably is a double-null-LOX-null-MMT barley plant. Such barley plants are described in more detail herein below.

According to the present invention, the method in general comprises a step of malting said double-null-LOX-null-MMT barley plant, although in some embodiments of the invention, the barley based beverage is prepared using unmalted barley. Malting is described in more detail in the section "Malting" herein below.

The method furthermore comprises a step of preparing wort by mashing a double-null-LOX-null-MMT barley or a double-null-LOX-null-MMT malt or a mixture thereof - optionally in the presence of additional adjuncts. Mashing is described in more detail in the section "Mashing" herein below.

The method also comprises a step of heating said wort optionally in the presence of additional ingredient(s), wherein at the most 4%, for example at the most 2% of the wort volume is evaporated, thereby obtained heated wort. This step is described in more detail herein in the section "Heating wort".

Finally, the method comprises processing heated wort into a beverage. That part of the method is described in more detail in the section "Preparation of beverages" herein below.

### Malting

By the term "malting" is to be understood germination of steeped barley kernels in a process taking place under controlled environmental conditions - for example, as illustrated in **FIG. 9****,** steps 2 and 3 - followed by a drying step. Said drying step may preferably be kiln drying of the germinated kernels at elevated temperatures.

Prior to drying, the steeped and germinated barley grains are referred to as "green malt", which may also represent a plant product according to the present invention.

This aforementioned sequence of malting events is important for the synthesis of numerous enzymes that cause grain modification, processes that principally depolymerize cell walls of the dead endosperm to mobilize the grain nutrients and activate other depolymerases. In the subsequent drying process, flavour and colour are generated due to chemical browning reactions.

Malting is a highly energy consuming process. Due to the need for high temperature, in particular kiln drying also is an energy consuming process. There are several objectives of kiln drying, including in particular: (i) drying the germinated barley kernels; (ii) stopping germination; (iii) denaturation of lipoxygenases in order to decrease levels of T2N and T2N potential; and (iv) generation of DMS from precursors and removal of DMS in order to decrease levels of DMS potential and DMS.

According to the present invention, kiln drying can be performed at low temperature, and still accomplishing the above-mentioned objectives. By employing a barley plant with loss of functional LOX-1 and LOX-2 there is no requirement for denaturation of lipoxygenases. By employing a barley plant with loss of functional MMT there is no requirement for decreasing the levels of DMS and DMS potential, because said levels are minute in such barley plants. Accordingly, barley grains may be dried and germination may be stopped even at low temperatures.

Thus malting according to the present invention preferably comprises the steps of:
(a) steeping double-null-LOX-null-MMT barley;
(b) germinating said barley; and
(c) drying, preferably by kiln drying, said barley.

Steeping may be performed by any conventional method known to the skilled person. One non-limiting example involves steeping at a temperature in the range of 10 to 25°C with alternating dry and wet conditions. During steeping, for example, the barley may be incubated wet for in the range of 30 min to 3 h followed by incubation dry for in the range of 30 min to 3 h and optionally repeating said incubation scheme in the range of 2 to 5 times. The final water content after steeping may, for example, be in the range of 40 to 50%.

Germination may be performed by any conventional method known to the skilled person. One non-limiting example involves germination at a temperature in the range of 10 to 25°C, optionally with changing temperature in the range of 1 to 4 h.

A non-limiting example of a suitable steeping and germination scheme is outlined in Example 9 herein below.

The kiln drying may be performed at conventional temperatures, such as at least 75°C, for example in the range of 80 to 90°C, such as in the range of 80 to 85°C. Thus, the malt may, for example be produced by any of the methods described by Briggs et al. (1981) and by Hough et al. (1982). However, any other suitable method for producing malt may also be used with the present invention, such as methods for production of specialty malts, including, but not limited to, methods of roasting the malt. Non-limiting examples are described in Examples 6 and 8 and 9.

Preferably, however, said kiln drying is performed at a low temperature, more preferably at a temperature below 80°C, yet more preferably at a temperature below 75°C, such as at temperature below 70°C, for example at a temperature below 65°C, such as at temperature below 60°C, for example at a temperature below 55°C, such as at temperature below 50°C, for example at a temperature below 45°C, such as at temperature below 41 °C. Thus, it is preferred that the temperature does not rise above 80°C, preferably does not rise above 75°C at any time during kiln drying.

In order to sufficiently dry the germinated barley kernels, the kiln drying time may increase if said drying is performed at a low temperature. Preferably, kiln drying is performed for a time sufficient to reduce the water content of the germinated kernels to less than 10%, preferably to less than 8%, more preferably to less than 6%. Thus, at a conventional kiln drying temperature of 85°C, kilning time may be in the range of 1 to 3 h, whereas kilning at a temperature in the range of 70 to 80°C may require a kilning time of in the range of 1 to 10 h; kilning at a temperature in the range of 50 to 70°C may require a kilning time of in the range of 3 to 50 h, whereas kilning at a temperature of below 50°C, for example in the range of 40 to 50°C, may require a kilning time of more than 40 h, such as in the range of 40 to 60 h, for example in the range of 45 to 52 h, such as 48 h.

In one aspect, the invention also relates to malt compositions prepared from double-null-LOX-null-MMT barley kernels by malting, preferably by malting as described herein directly above.

Said malt compositions comprise low levels of T2N and T2N potential even when prepared at the low kilning temperatures as described above. In particular, said malt compositions comprise low levels of T2N potential (and T2N precursors).

It has been described that LOX activity in barley kernels may be reduced by a soaking process, wherein barley may be subjected to high temperatures and/or lactic acid treatment. However, such soaking processes are also energy consuming. Furthermore, such treatment may have other adverse effects, such as reducing desirable enzymatic activities, e.g. phytase activity. In addition, such treatment only reduces LOX activity from the point when the heat treatment is undertaken and thus without effect on the prior accumulation of products derived from LOX activity.

In one embodiment according to the invention, the plant products are prepared using a method, wherein the barley kernels are not subjected to soaking at a temperature of at least 70°C. It is also preferred that the plant products according to the invention are prepared using a method, wherein the barley kernels are not subjected to soaking at a temperature of at least 57°C in the presence of lactic acid.

It is preferred that said malt compositions comprise less than 60%, preferably less than 50%, more preferably less than 40%, even more preferably less than 30%, for example less 20% T2N compared to a malt composition prepared in the same manner from a wild-type barley, preferably from cv. Power or from cv. Quench or from cv. Rosalina.

It is furthermore preferred that said malt compositions - even when kiln dried at a temperature of range of 70 to 80°C - comprise less than 60%, preferably less than 50%, more preferably less than 40%, even more preferably less than 30%, more preferably less than 20% T2N compared to a malt composition prepared in the same manner from a wild-type barley, preferably from cv. Power or from cv. Quench or from cv. Rosalina.

It is furthermore preferred that said malt compositions - even when kiln dried at a temperature of range of 50 to 70°C - comprise less than 60%, preferably less than 50%, more preferably less than 40%, even more preferably less than 30%, more preferably less than 20% T2N compared to a malt composition prepared in the same manner from a wild-type barley, preferably from cv. Power or from cv. Quench or from cv. Rosalina.

It is furthermore preferred that said malt compositions - even when kiln dried at a temperature of range of 40 to 50°C - comprise less than 60%, preferably less than 50%, more preferably less than 40%, even more preferably less than 30%, more preferably less than 20% T2N compared to a malt composition prepared in the same manner from a wild-type barley, preferably from cv. Power or cv. Quench or from cv. Rosalina.

In addition to the low levels of T2N and T2N potential, the malt according to the invention also comprises low levels of DMS and DMS precursor, even when prepared at the low kilning temperatures described above.

Interestingly, DMS is a rather volatile compound with a boiling point of 37°C-38°C (Imashuku, supra), and during malt production, for example during kiln drying, the composition is generally subjected to heat, such that substantial amounts of DMS evaporate. However, during cooling of a normal malt composition, more DMS may be generated from DMS precursors (DMSP). One major advantage of the present invention is that no, or only very little, DMSP (in particular SMM) is generated in the malt composition.

Methods for reducing DMS concentration in malt have been described. Many of these methods rely on highly energy consuming heat treatment of malt. Said heat treatment may simply be heating of malt, for example during kiln drying or it may involve volatization and/or removal of free DMS by application of steam. Thus, steam treatment of malt may reduce the levels of free DMS in malt, but again representing a process with high energy consumption. Furthermore, these methods mainly reduce the level of free DMS in malt, with only little effect on the level of SMM. In one embodiment, the malt compositions of the invention have only been subjected to limited treatment that involve volatizing and removing free DMS by steam, or alternatively have not been subjected to treatment that involve volatizing and removing free DMS using steam during kiln drying.

In one embodiment, it is preferred that the malt according to the invention has not been treated with a bromate salt, such as potassium bromate or calcium bromate.

The malt compositions of the invention preferably comprise at the most 3, preferably at the most 2, more preferably at the most 1, even more preferably at the most 0.5, such as at the most 0.2 µg/g free DMS. In addition, it is preferred that the malt compositions of the invention preferably comprises at the most 2, preferably at the most 1, more preferably at the most 0.5 µg/g, even more preferably at the most 0.2 µg/g DMSP. The concentration of DMSP, which preferably may be SMM, is here and elsewhere in this document indicated as the concentration of DMS, which may be liberated from said DMSP.

It is furthermore preferred that said malt compositions - even when kiln dried at a temperature in the range of 70 to 80°C - comprise at the most 2, preferably at the most 1, more preferably at the most 0.5 µg/g, even more preferably at the most 0.2 µg/g DMSP (preferably SMM).

It is furthermore preferred that said malt compositions - even when kiln dried at a temperature of range of 50 to 70°C - comprise at the most 2, preferably at the most 1, more preferably at the most 0.5 µg/g, even more preferably at the most 0.2 µg/g DMSP (preferably SMM).

It is furthermore preferred that said malt compositions - even when kiln dried at a temperature of range of 40 to 50°C - comprise at the most 2, preferably at the most 1, more preferably at the most 0.5 µg/g, even more preferably at the most 0.2 µg/g DMSP (preferably SMM).

In another aspect the disclosure relates to green malt compositions comprising at the most 5000, more preferably at the most 2500, yet more preferably at the most 1000, even more preferably at the most 500, yet more preferably at the most 250, for example at the most 150 ppb DMSP. It is also preferred that said green malt compositions comprises at the most 200, preferably at the most 150, more preferably at the most 100, even more preferably at the most 50, such as at the most 25 ppb free DMS.

Although the primary use of malt is for beverage production, it can also be utilized in other industrial processes, for example as an enzyme source in the baking industry, or in the food industry as a flavouring and colouring agent, e.g. in the form of malt or malt flour or indirectly as a malt syrup, etc. Thus, the plant product according to the invention may be any of the aforementioned products.

In another aspect, the plant products according to the invention comprise, or even consist of syrup, such as a barley syrup, or a barley malt syrup. The plant product may also be an extract of barley or malt.

Malt may be further processed, for example by milling. Thus, the plant product according to the invention may be any kind of malt, such as unprocessed malt or milled malt, such as flour. Milled malt and flour thereof comprise chemical components of the malt and dead cells that lack the capacity to re-germinate.

Preferably milling is performed in a dry state, i.e. the malt is milled while dry. Thus, it is preferred that malt is not milled under water.

### Mashing

The method according to the invention comprises a step of producing wort by mashing double-null-LOX-null-MMT barley and/or malt and optionally additional adjuncts. Said mashing step may also optionally comprise sparging, and accordingly said mashing step may be a mashing step including a sparging step or a mashing step excluding a sparging step.

In general, wort production is initiated by the milling of the double-null-LOX-null-MMT malt and/or double-null-LOX-null-MMT barley. If additional adjuncts are added, these may also be milled depending on their nature. If the adjunct is a cereal, it may for example be milled, whereas syrups, sugars and the like will generally not be milled. Milling will facilitate water access to grain particles in the mashing phase. During mashing enzymatic depolymerization of substrates initiated during malting may be continued.

In **FIG. 9****,** steps 4 to 6 illustrate a common method for preparation of wort from malt. In general, wort is prepared by combining and incubating milled malt and water, i.e. in a mashing process. During mashing, the malt/liquid composition may be supplemented with additional carbohydrate-rich adjunct compositions, for example milled barley, maize, or rice adjuncts. Unmalted cereal adjuncts usually contain little or no active enzymes, making it important to supplement with malt or exogenous enzymes to provide enzymes necessary for polysaccharide depolymerization etc.

During mashing, milled double-null-LOX-null-MMT malt and/or milled double-null-LOX-null-MMT barley -and optionally additional adjuncts are incubated with a liquid fraction, such as water. The incubation temperature is in general either kept constant (isothermal mashing), or gradually increased, for example increased in a sequential manner. In either case, soluble substances in the malt/barley/adjuncts are liberated into said liquid fraction. A subsequent filtration confers separation of wort and residual solid particles, the latter also denoted "spent grain". The wort thus obtained may also be denoted "first wort". Additional liquid, such as water may be added to the spent grains during a process also denoted sparging. After sparging and filtration, a "second wort" may be obtained. Further worts may be prepared by repeating the procedure. Non-limiting examples of suitable procedures for preparation of wort is described by Briggs et al. (supra) and Hough et al. (supra).

It has been described that LOX activity may be reduced by heat treatment of the enzymes and that DMS levels may be reduced by heat treatment. Also, it has been described that wort may be heat treated to reduce LOX activity and reduce levels of DMS and/or that mashing may be performed at high temperatures in order to reach the same objective. However, heat treatment may have adverse effects, such as reducing other enzymatic activities and heat treatment is furthermore energy consuming. In addition, heat treatment only reduces lipoxygenase activity and DMS levels from the point when the heat treatment is undertaken and thus it does not affect the prior accumulation of LOX activity derived products and DMS precursors.

Accordingly, in one embodiment wort is prepared using a method wherein the initial mashing temperature does not exceed 70°C, preferably does not exceed 69°C, thus for example the initial mashing temperature may be in the range of 30°C to 69°C, such as in the range of 35°C to 69°C, for example in the range of 35°C to 65°C, such as in the range of 35°C to 55°, for example in the range of 35°C to 45°C, such as approximately 40°C. It is also preferred that the wort according to the invention has not been subjected to temperatures of 70°C or higher for more than 25 min, preferably not for more than 20 min, and that the wort has not been subjected to temperatures of 78°C or more, for more than 20 min, preferably not for more than 15 min, more preferably not for more than 10 min during mashing. If the mashing temperatures are too high, this property will affect the enzymatic activities in the mash and may reduce, or even abolish, desirable enzymatic activities, which will result in an altered quality of the wort. It is furthermore preferred that the wort according to the invention has not been subjected to temperatures of 65°C or higher for more than 100 min, preferably not for more than 90 min, more preferably not for more than 80 min, yet more preferably not for more than 70 min during mashing.

In a preferred embodiment, the temperature during mashing does not exceed 80 °C, preferably does not exceed 78°C.

One non-limiting example of a suitable mashing is:
(1) mashing-in at a temperature in the range of 35-45°C, such as approximately 40°C, in the range of 10 to 30 min, such as approximately 20 min;
(2) heating to a temperature in the range of 60 to 70°C, preferably in the range of 60 to 65°C, such as approximately 65°C, in the range of 30 to 90 min, preferably in the range of 45 to 75 min, such as approximately 60 min;
(3) heating to a temperature in the range of 70 to 80°C, preferably in the range of 75 to 78°C, such as approximately 78°C, in the range of 5 to 15 min, such as approximately 10 min.

Another non-limiting example of a suitable mashing is:
(4) mashing-in at a temperature in the range of 55-65°C, such as approximately 60°C, in the range of 10 to 30 min, such as approximately 20 min;
(5) heating to a temperature in the range of 60 to 70°C, preferably in the range of 60 to 65°C, such as approximately 65°C, in the range of 30 to 90 min, preferably in the range of 45 to 75 min, such as approximately 60 min;
(6) heating to a temperature in the range of 70 to 80°C, preferably in the range of 75 to 78°C, such as approximately 78°C, in the range of 5 to 15 min, such as approximately 10 min.

As mentioned above, the wort composition may be prepared by mashing double-null-LOX-null-MMT barley plants, or parts thereof, such as unmalted double-null-LOX-null-MMT kernels, in particular milled, unmalted double-null-LOX-null-MMT kernels, or parts thereof. Unmalted barley kernels lack or contain only a limited amount of enzymes beneficial for wort production, such as enzymes capable of degrading cell walls or enzymes capable of depolymerising starch into sugars. Thus, in embodiments of the invention where unmalted double-null-LOX-null-MMT is used for mashing, it is preferred that one or more suitable, external brewing enzymes are added to the mash. Suitable enzymes may be lipases, starch degrading enzymes (e.g. amylases), glucanases [preferably (1-4)- and/or (1-3,1-4)-β-glucanase], and/or xylanases (such as arabinoxylanase), and/or proteases, or enzyme mixtures comprising one or more of the aforementioned enzymes, e.g. Cereflo, Ultraflo, or Ondea Pro (Novozymes). A method for producing a beverage from wort prepared from unmalted barley may also be referred to as "barley brewing", and a wort composition thereof as "barley wort", or "barley-brewed" wort.

The wort composition may also be prepared by using a mixture of malted and unmalted double null-LOX-null-MMT barley plants, or parts thereof, in which case one or more suitable enzymes may be added during preparation. More specifically, barley of the invention can be used together with malt in any combination for mashing - with or without external brewing enzymes - such as, but not limited to, the proportions of barley: malt = approximately 100 : 0, or approximately 75 : 25, or approximately 50 : 50, or approximately 25 : 75.

In other embodiments of the invention, it is preferred that no external enzymes, in particular that no external protease, and/or no external celluluase and/or no external α-amylase and/or no external β-amylase and/or no external maltogenic α-amylase is added before or during mashing.

The wort obtained after mashing may also be referred to as "sweet wort". In conventional methods, the sweet wort is boiled with or without hops where after it may be referred to as boiled wort.

The term "approximately" as used herein means ±10%, preferably ±5%, yet more preferably ±2%.

### Heating wort

The method for preparing a barley based beverage according to the present invention involves a step of heating wort obtained after the mashing step described herein above in the section "Mashing".

It is an advantage of the present invention that barley based beverages with low levels of off-flavours and precursors thereof - in particular T2N and DMS and precursors thereof - may be prepared without the requirement for extensive heating of wort.

In conventional brewing, wort is generally boiled for an extensive length of time. Wort boiling has several objectives, in particular: (i) inactivation of enzymes; (ii) coagulation of protein; (iii) sterilization of the wort; (iv) extraction of hop compounds; (v) isomerisation of α-acids; (vi) conversion of DMSP to DMS; and (vii) evaporation of DMS and T2N.

Several of these objectives may be reached without extensive boiling. Thus, sterilization only requires short boiling or heating. Extraction of hop compounds may also be done during a short boiling or heating. Pre-isomerized α-acids are commercially available and may be added to the wort.

By employing a barley plant with loss of functional LOX-1 and LOX-2 there is no requirement for denaturation of LOXs. Furthermore, the requirement for reduction of T2N levels is also abolished by employing a barley plant with loss of functional LOX-1 and LOX-2. By employing a barley plant with loss of functional MMT there is also no requirement for decreasing the levels of DMS and DMS potential, because said levels are minute in such barley plants. In addition, barley grains may be dried and germination may be stopped even at low temperatures.

It has been attempted to reduce the energy consumption during wort boiling by various means, for example by reducing evaporation to as little as 3% combined with a stripping process extracting unwanted volatile compounds into steam. However, generation of steam is also an energy consuming process.

It has also been attempted to prepare wort without classic wort boiling, However, these methods in general utilize high temperatures of up to 95°C during mashing as well as stripping of wort with steam, thus considered high-energy-consuming processes.

The present method provides the possibility for reduced evaporation, and preferably even in the absence of a stripping process. Thus, according to the methods of the present invention wort is heated in a manner so that at the most 4%, preferably at the most 3%, even more preferably at the most 2%, even more preferably at the most 1.5%, yet more preferably at the most 1%, even more preferably at the most 0.5%, even more preferably at the most 0.1%, such as at the most 0.01% of the wort volume is evaporated. Even more preferably the aforementioned reduced evaporation is performed in the absence of steam treatment of the wort. It is also preferred that the aforementioned reduced evaporation is performed in the absence of stripping of the wort, e.g. with steam.

The reduced evaporation may be accomplished by heating wort in an essentially closed container or preferably in a closed container.

It is furthermore preferred that no extensive evaporation of liquid is undertaken in any of the other steps of the method. A preferred embodiment of the present invention accordingly relates to methods for preparing barley based beverages with low levels of one or more off-flavours and precursors thereof (preferably T2N and DMS and precursors thereof), wherein the method involves reduced energy input, the method comprising the steps of:
(i) providing a barley plant or part thereof, wherein said barley plant comprises:
   (a) a first mutation that results in a total loss of functional LOX-1; and
   (b) a second mutation resulting in a total loss of functional LOX-2; and
   (c) a third mutation resulting in a total loss of functional MMT;
(ii) optionally malting at least part of said barley, thereby obtaining malted barley;
(iii) mashing said barley and/or malted barley and optionally additional adjuncts, thereby obtaining a wort;
(iv) heating said wort optionally in the presence of additional ingredient(s);
(v) processing said heated wort into a beverage;
wherein at the most 4%, for example at the most 3%, such as at the most 2%, for example at the most 1.5%, such as at the most 1% of the liquid volume is evaporated during the method after completion of step (ii), or even after completion of step (iv) above, thereby preparing a barley derived beverage with low levels of one or more off-flavours and precursors thereof.

It is preferred that step (iv) herein above is performed with the wort in an essentially closed container, such as in a closed container.

Preferably the liquid/wort/beverage is heated to a temperature above 80 °C for at the most 30 min, more preferably for at the most 20 min, thereby preparing a barley derived beverage with low levels of one or more off-flavours and precursors thereof.

Preferably, said liquid/wort/beverage is heated to a temperature in the range of 80 to 99.8°C, preferably to a temperature in the range of 80 to 99.5°C, such as to a temperature of 80 to 99°C, yet more preferably to a temperature of in the range of 90 to 99°C, yet more preferably to a temperature of in the range of 95 to 99°C for at the most 30 min, preferably at the most 20 min, such as for in the range of 10 to 30 min, for example for in the range of 10 to 20 min during said method.

First, second and further worts may be combined, and thereafter subjected to heating or each individual kind or wort may be heated. According to the methods of the invention, the wort must not necessarily be boiled. The non-boiled wort, either a pure first wort or a combined wort, is also referred to as "sweet wort"; after boiling it may be referred to as "boiled wort". If the wort is to be used in production of beer, hops are frequently added prior to boiling.

In traditional brewing methods, the wort is boiled for a long time, in general in the range of 60 min to 120 min, in order to evaporate at least 5% and some times even up to 25% of the wort volume. However, extended boiling is undesirable for a number of other reasons, for example because extended boiling requires pronounced energy supply.

According to the present invention, wort with low levels of T2N, T2N potential, DMS and DMSP can be produced from double-null-LOX-null-MMT barley even without extended boiling. Thus, the wort is preferably boiled for at the most 30 min, more preferably for at the most 15 min, even more preferably for at the most 10 min, yet more preferably for at the most 5 min, even more preferably for at the most 1 min, yet more preferably the wort is not boiled at all. It is furthermore, preferred that after completion of step ii) of the method according to the invention the liquid/wort/beverage is boiled for at the most at the most 30 min, more preferably for at the most 15 min, even more preferably for at the most 10 min, yet more preferably for at the most 5 min, even more preferably for at the most 1 min, yet more preferably the liquid/wort/beverage is not boiled at all. Preferably, heating of wort is undertaken in an essentially closed container, preferably in a closed container.

In this context the term, "boiling" means bringing the liquid or wort or beverage to a temperature where water evaporates. Accordingly, at normal pressure boiling would mean to bring an aqueous liquid, such as wort, to 100°C or slightly above.

It is thus also preferred that the wort is kept at a temperature of at least 100°C for at the most 30 min, more preferably for at the most 15 min, even more preferably for at the most 10 min, yet more preferably for at the most 5 min, even more preferably for at the most 1 min, yet more preferably the wort is not heated to a temperature of at least 100°C at all. Furthermore, it is preferred that after completion of step (ii) of the method according to the invention the liquid/wort/beverage is kept at a temperature of at least 100°C for at the most at the most 30 min, more preferably for at the most 15 min, even more preferably for at the most 10 min, yet more preferably for at the most 5 min, even more preferably for at the most 1 min, yet more preferably the liquid/wort/beverage is not at all heated to a temperature of at least 100°C.

It is also preferred that the entire method of preparing barley based beverages according to the invention at no time involves heating to a temperature of more than 99.8°C, preferably 99.5°C, yet more preferably 99°C.

Rather the method may involve a step of heating wort to a temperature of at the most 99.8°C, such as at the most 99.5°C, for example at the most 99°C, such as at the most 98°C for a limited amount of time. Said limited amount of time is preferably at the most 30 min, more preferably at the most 15 min, even more preferably at the most 10 min.

Thus, it is preferred that the wort is heated to a temperature above 80°C, preferably in the range of 80 to 99.8°C, such as in the range of 80 to 99.5°C, for example in the range of 80 to 99°C, yet more preferably to a temperature of in the range of 90 to 99°C, yet more preferably to a temperature of in the range of 95 to 99°C for at the most 30 min, preferably at the most 20 min, such as for in the range of 10 to 30 min, for example for in the range of 10 to 20 min.

Additionally, it is preferred that the wort is not subjected to (e.g. washing with CO₂) subsequent to boiling of wort and prior to fermentation.

### Wort

In another aspect, the disclosure relates to types of plant products, which are wort compositions. Said wort compositions are preferably prepared from malt compositions derived from double-null-LOX-null-MMT kernels. Said malts may be prepared from only double-null-LOX-null-MMT kernels, or mixtures comprising other kernels as well. The disclosure also relates to wort compositions prepared using double-null-LOX-null-MMT barley, or parts thereof, such as green malt, alone or mixed with other components.

The wort compositions are preferably prepared by mashing as described herein above in the section "Mashing". Furthermore, the wort compositions may have been heated as described herein above in the section "Heating wort".

It is preferred that said wort compositions preferably comprise less than 60%, more preferably less than 50%, even more preferably less than 40% T2N potential compared to a wort composition prepared in the same manner from a wild-type barley, preferably from cv. Power or cv. Rosalina.

Said wort may be the first, and/or the second, and/or further worts and/or mixtures thereof. The wort composition may be sweet wort, heated wort, or a mixture thereof. Heated wort is preferably heated as described in the section "Heating wort" herein above. The wort composition may also be barley wort. In general, a wort composition contains a high content of amino nitrogen and fermentable carbohydrates, the latter mainly being maltose.

The wort may in one embodiment be sweet wort, i.e. wort which has not been subjected to heat treatment. Said sweet wort preferably comprises less than 60%, more preferably less than 50% T2N potential compared to a wort composition prepared in the same manner from a wild-type barley, preferably from cv. Power or cv. Rosalina. If said wort has been prepared from malt that was kiln dried at low temperatures it may even comprise less T2N potential. Thus in embodiments wherein said sweet wort has been prepared from malt, which has been kiln dried at a temperature in the range of 50 to 70°C, then said sweet wort may preferably comprise less than 50%, even more preferably less than 45%, such as less than 40% T2N potential compared to a wort composition prepared in the same manner from a wild-type barley, preferably from cv. Power. In embodiments wherein said sweet wort has been prepared from malt, which has been kiln dried at a temperature in the range of 40 to 50°C, then said sweet wort may preferably comprise less than 50%, even more preferably less than 40%, yet more preferably less than 30% T2N potential compared to a wort composition prepared in the same manner from a wild-type barley, preferably from cv. Quench or cv. Rosalina.

Said sweet wort preferably also comprises low levels of DMSP, preferably less than 150 µg/L, more preferably less than 100 µg/L, even more preferably less than 50 µg/L, yet more preferably less than 30 µg/L, even more preferably less than 20 µg/L, yet more preferably less than 15 µg/L DMSP (preferably SMM). Even if said wort has been prepared from malt that was kiln dried at low temperatures it still comprises low levels of DMSP. Thus in embodiments wherein said sweet wort has been prepared from malt, which has been kiln dried at a temperature in the range of 50 to 70 °C, then said sweet wort may preferably comprise less than 150 µg/L, more preferably less than 100 µg/L, even more preferably less than 50 µg/L, yet more preferably less than 30 µg/L, even more preferably less than 20 µg/L, yet more preferably less than 15 µg/L DMSP. In embodiments wherein said sweet wort has been prepared from malt, which has been kiln dried at a temperature in the range of 40 to 50 °C, then said sweet wort may preferably comprise less than 150 µg/L, more preferably less than 100 µg/L, even more preferably less than 50 µg/L, yet more preferably less than 30 µg/L, even more preferably less than 20 µg/L, yet more preferably less than 15 µg/L DMSP.

Preferably, said sweet wort also comprises low levels of DMS, preferably less than 90 µg/L, even more preferably less than 50 µg/L, yet more preferably less than 30 µg/L, even more preferably less than 20 µg/L DMS.

The wort may also be wort, which has only been heat treated for a short time, such as at a temperature in the range of 95 to 99.8°C or in the range of 95 to 99°C for in the range of 10 to 30 min. In this case said wort preferably comprises at the most 60%, more preferably at the most 50%, even more preferably at the most 45%, more preferably at the most 40% T2N potential compared to a wort composition prepared in the same manner from a wild-type barley, preferably from cv. Power or cv Rosalina Said wort also preferably comprises at the most 150 µg/L, more preferably less than 100 µg/L, even more preferably less than 50 µg/L, yet more preferably less than 30 µg/L, even more preferably less than 20 µg/L, yet more preferably less than 15 µg/L DMSP. Said wort also preferably comprise at the most 150 µg/L, more preferably less than 100 µg/L, even more preferably less than 50 µg/L, yet more preferably less than 30 µg/L, even more preferably less than 20 µg/L DMS.

The wort may also be heated wort (preferably heated as described herein above in the section "Heating wort") in which case the wort preferably comprises at the most 60%, more preferably at the most 50%, yet more preferably at the most 40% T2N potential compared to a wort composition prepared in the same manner from a wild-type barley, preferably from cv. Power.

Said heated wort preferably also comprises low levels of DMSP, preferably less than 150 µg/L, more preferably less than 100 µg/L, even more preferably less than 50 µg/L, yet more preferably less than 30 µg/L, even more preferably less than 20 µg/L, yet more preferably less than 15 µg/L DMSP. In embodiments wherein said cooled wort has been prepared from malt, which has been kiln dried at a temperature in the range of 50 to 70°C, then said sweet wort may preferably comprise less than 150 µg/L, more preferably less than 100 µg/L, even more preferably less than 50 µg/L, yet more preferably less than 30 µg/L, even more preferably less than 20 µg/L, yet more preferably less than 15 µg/L DMSP. Even in embodiments wherein said cooled wort has been prepared from malt, which has been kiln dried at a temperature in the range of 40 to 50°C, then said sweet wort may preferably comprise less than 150 µg/L, more preferably less than 100 µg/L, even more preferably less than 50 µg/L, yet more preferably less than 30 µg/L, even more preferably less than 20 µg/L, yet more preferably less than 15 µg/L DMSP. Said heated wort preferably also comprises low levels of DMS, preferably less than 30 µg/L, more preferably less than 20 µg/L DMSP.

The wort may also be heated wort (preferably heated as described herein above in the section "Heating wort"), which subsequently has been cooled (herein also referred to as cooled wort) in which case the wort preferably comprises at the most 60%, more preferably at the most 50%, yet more preferably at the most 40% T2N potential compared to a wort composition prepared in the same manner from a wild-type barley, preferably from cv. Power or cv. Rosalina. In embodiments wherein said cooled wort has been prepared from malt, which has been kiln dried at a temperature in the range of 40 to 50°C, then said sweet wort may preferably comprise less than 50%, even more preferably less than 40%, yet more preferably less than 30% T2N potential compared to a wort composition prepared in the same manner from a wild-type barley, preferably from cv. Power or cv. Rosalina.

Said cooled wort preferably also comprises low levels of DMSP, preferably less than 90 µg/L, even more preferably less than 50 µg/L, yet more preferably less than 30 µg/L, even more preferably less than 20 µg/L, yet more preferably less than 15 µg/L DMSP. Said cooled wort preferably also comprises low levels of DMS, preferably less than 90 µg/L, even more preferably less than 50 µg/L, yet more preferably less than 30 µg/L, even more preferably less than 20 µg/L DMS.

In one specific embodiment the wort composition is a barley wort, such as heated barley wort, i.e. wort prepared by incubating unmalted (and preferably milled) double-null-LOX-null-MMT kernels with water, preferably by mashing and sparging. Such barley wort is characterized by extremely low levels of T2N and T2N potential. It is preferred that said barley wort comprises less than 50%, more preferably less than 40%, even more preferably less than 30% T2N potential compared to a barley wort composition prepared in the same manner from a wild-type barley, preferably from cv. Power. It is also preferred that said barley wort preferably comprises less than 50%, more preferably less than 40%, even more preferably less than 30% T2N precursor compared to a barley wort composition prepared in the same manner from a wild-type barley, preferably from cv. Power or cv. Quench or cv. Rosalina.

### Beverages

The present invention relates to barley based beverages prepared from double-null-LOX-null-MMT barley. Said beverages may in one preferred embodiment be malt beverages, even more preferred fermented beverages, such as fermented malt beverages, preferably alcoholic beverages, such as beer, wherein said beverage is prepared using double-null-LOX-null-MMT barley, or parts thereof. Hence, in one preferred embodiment of the invention, the beverage is preferably prepared by fermentation of double-null-LOX-null-MMT barley, or parts thereof, or extracts thereof, for example by fermentation of wort from double-null-LOX-null-MMT malt, alone or in combination with other ingredients.

In a preferred embodiment of the invention, the above-mentioned beverage is prepared by a method comprising the steps of:
(i) providing a barley plant or part thereof, wherein said barley plant comprises:
   (a) a first mutation that results in a total loss of functional LOX-1; and
   (b) a second mutation resulting in a total loss of functional LOX-2; and
   (c) a third mutation resulting in a total loss of functional MMT;
(ii) optionally malting at least part of said barley, thereby obtaining malted barley;
(iii) mashing said barley and/or malted barley and optionally additional adjuncts, thereby obtaining a wort;
(iv) heating said wort optionally in the presence of additional ingredient(s), wherein at the most 4% of the wort volume is evaporated, thereby obtained heated wort;
(v) processing said heated wort into a beverage.

However, in other embodiments the invention relates to any barley based beverage prepared from double-null-LOX-null-MMT barley. Thus, the invention also relates to barley based beverages prepared from double-null-LOX-nufl-MMT barley using conventional methods, such as conventional brewing methods.

In some embodiments, the beverage is a non-fermented beverage, for example wort, preferably wort prepared from double-null-LOX-null-MMT malt.

It is also comprised within the present invention that said beverage may be prepared from unmalted barley plants, preferably unmalted double-null-LOX-null-MMT barley plants, or parts thereof, e.g. by barley brewing.

The beverage may be a non-alcoholic beverage, such as non-alcoholic beer or other kinds of non-alcoholic beverages, such as non-alcoholic malt beverages, such as maltina.

Preferably, however, said beverage is prepared from a malt composition prepared from double-null-LOX-null-MMT barley kernels. More preferably, said beverage is beer. This may be any kind of beer known to the person skilled in the art. In one embodiment, the beer is, for example, a lager beer. The beer is preferably brewed using a malt composition comprising germinated double-null-LOX-null-MMT barley, more preferably said beer is brewed using a malt composition prepared exclusively from germinated double-null-LOX-null-MMT barley. The malt composition may, however, also comprise other components, for example other germinated or non-germinated cereals, such as wild-type barley, double-null-LOX-null-MMT barley, wheat and/or rye, or non-germinated raw materials that comprise sugars, or compositions derived from malted or unmalted raw materials, including syrup compositions. Preferably, however, all barley, such as all malted and/or unmalted barley and/or germinated and/or non-germinated barley used for preparation of said beer is preferably double-null-LOX-null-MMT barley.

Thus, the invention also relates to methods of producing a beverage comprising the steps of:
(i) providing a malt composition comprising germinated double-null-LOX-null-MMT kernels;
(ii) processing said malt composition into a beverage.

In a preferred embodiment, the beverage according to the invention is beer that has been produced from wort prepared from kilned malt (preferably prepared as described herein above in the section "Malting"), more preferably by mashing said kilned malt (preferably as described herein above in the section "Mashing"), wherein said mashing may also optionally contain a sparging step. Furthermore, said wort has preferably been heated, preferably as described herein above in the section "Heating wort", although in certain embodiments the wort may be heated in a conventional manner by boiling. Beer thus produced may also be referred to as "malted" herein. However, the beverage according to the invention may also be beer prepared from barley wort. Such beer is also referred to as "barley beer".

In general terms, alcoholic beverages - such as beer - may be manufactured from malted and/or unmalted barley grains. Malt, in addition to hops and yeast, contributes to flavour and colour of the beer. Furthermore, malt functions as a source of fermentable sugar and enzymes. A schematic representation of a general process of beer production is shown in FIG. 9, while detailed descriptions of examples of suitable methods for malting and brewing can be found, for example, in publications by Briggs et al. (1981) and Hough et al. (1982). Numerous, regularly updated methods for analyses of barley, malt and beer products are available, for example, but not limited to, American Association of Cereal Chemists (1995), American Society of Brewing Chemists (1992), European Brewery Convention (1998), and Institute of Brewing (1997). It is recognized that many specific procedures are employed for a given brewery, with the most significant variations relating to local consumer preferences. Any such method of producing beer may be used with the present invention.

The first step of producing beer from wort preferably involves heating said wort as described herein above, followed by a subsequent phase of wort cooling and optionally whirlpool rest. After being cooled, the wort is transferred to fermentation tanks containing yeast. Preferably, said yeast is brewer's yeast, Saccharomyces carlsbergensis. The wort will be fermented for any suitable time period, in general in the range of 1 to 100 days. During the several-day-long fermentation process, sugar is converted to alcohol and CO₂ concomitantly with the development of some flavour substances.

Subsequently, the beer may be further processed, for example chilled. It may also be filtered and/or lagered - a process that develops a pleasant aroma and a less yeasty flavour. Also additives may be added. Furthermore, CO₂ may be added. Finally, the beer may be pasteurized and/or filtered, before it is packaged (e.g. bottled or canned). In a preferred embodiment, the beverage comprises less than 70%, preferably less than 60%, more preferably less than 50% T2N potential compared to the T2N potential of a beverage prepared in the same manner from wild-type barley, preferably from cv. Power or cv. Quench or cv. Rosalina. It is also preferred that the beverages according to the invention comprise at the most 2 ppb, more preferably at the most 1.5 ppb T2N potential if the °P in the original extract upon which the beverage is based is adjusted to in the range of 10 to 12°P, more preferably to 11 °P.

In a preferred embodiment, the beverage comprises less than 70%, preferably less than 60%, more preferably less than 50% T2N precursors compared to the T2N precursors of a beverage prepared in the same manner from wild-type barley, preferably from cv. Power or cv. Quench or cv. Rosalina. It is also preferred that the beverages according to the invention comprise at the most 2 ppb, more preferably at the most 1.5 ppb T2N precursor if the °P in the original extract upon which the beverage is based is adjusted to in the range of 10 to 12°P, more preferably to 11 °P.

Said beverage - preferably beer - also comprises less than 10 ppb DMS.

In one specific embodiment, the beverage is barley beer, which comprises less than 60%, preferably less than 50% T2N potential compared to the T2N potential of a barley beer prepared in the same manner from wild-type barley, preferably from cv. Power or Quench.

In another specific embodiment, the beverage - preferably beer - is prepared from heated wort, which has only been heat treated for a short time, such as at a temperature in the range of 95 to 99.8°C or in the range of 95 to 99°C for in the range of 10 to 30 min. Preferably, said heated wort has been heated as described herein above in the section "Heating wort". In embodiments wherein the beverage - preferably beer - is prepared from such heated wort, then said beverage - preferably beer - comprises less than 60%, preferably less than 50%, more preferably less than 45% T2N potential compared to the T2N potential of a beer prepared in the same manner from wild-type barley, preferably from cv. Power or cv. Quench or cv. Rosalina. In this embodiment, it is also preferred that said beverage-preferably beer - comprises less than 60%, preferably less than 50%, more preferably less than 45% T2N precursor compared to the T2N precursors of a beverage-preferably beer prepared in the same manner from wild-type barley, preferably from cv. Power or cv. Quench or cv. Rosalina. In this embodiment, it is also preferred that the beer according to the invention comprises at the most 2 ppb, more preferably at the most 1.5 ppb T2N potential. In this embodiment, it is furthermore preferred that the beer according to the invention comprises at the most 2 ppb, more preferably at the most 1.5 ppb T2N precursor if the °P in the original extract upon which the beverage is based is adjusted to in the range of 10 to 12°P, more preferably to 11 °P. Said beverage - preferably beer - also comprises less than 10 ppb DMS.

"Organoleptic qualities" means qualities appealing to the human olfactory and taste senses. Said qualities are analyzed, for example, by a specialist beer taste panel. Preferably, said panel is trained in tasting and describing beer flavours, with special focus on aldehydes, papery taste, old taste, esters, higher alcohols, fatty acids and sulphury components.

In general, the taste panel will consist of in the range of 3 to 30 members, for example in the range of 5 to 15 members, preferably in the range of 8 to 12 members. The taste panel may evaluate the presence of various flavours, such as papery, oxidized, aged, and bready off-flavours as well as flavours of esters, higher alcohols, sulfur components and body of beer. In relation to the present invention, it is preferred that papery and/or aged off-flavours are in particular reduced, whereas flavours such as aromatic, estery, alcoholic/solvent, floral, and/or hoppy may preferably be increased as compared to a beverage prepared from wild type barley using an identical method. A method of determining the "organoleptic qualities" of a beverage is described in Example 6 in international patent application WO 2005/087934. Another method of determining "organoleptic qualities" of a beverage is described in Examples 8 and 9 in international patent application PCT/DK2009/050355 (published as WO 2010/075860). Yet another example is described herein below in Example 9. In preferred embodiments, the stable organoleptic qualities are, at least partly, a result of low levels of T2N or T2N potential. Aromatic, estery, alcoholic/solvent, floral, and/or hoppy taste may preferably be determined as described in Example 7 of international patent application PCT/DK2009/050315 (published as WO2010/063288). In one preferred embodiment the beverages of the invention have scores for aromatic, estery, alcoholic/solvent, floral, and/or hoppy taste as described in international patent application PCT/DK2009/050315 (published as WO2010/063288) on p. 41, I. 15 to p. 44, I. 9.

It is preferred that the beverages according to the present invention are characterized by having a less papery taste compared to a similar beverage prepared in the same manner from a wild-type barley plant (preferably cv. Quench or cv. Rosalina) after storage for at least 1 week at in the range of 30 to 40°C, such as around 37°C. Preferably, said papery taste is less than 90%, more preferably less than 80%, such as less than 70% as evaluated by a trained taste panel.

It is also preferred that the beverages according to the present invention have reduced papery taste as compared to similar beverages prepared from wild-type barley after storage at elevated temperatures. When the property "papery taste" is determined by a trained, specialized taste panel (as described above), and scored on a scale from 0 to 5 - in which 0 is absent and 5 is extreme - it is preferred that the beverages of the invention have one, or preferably both, of the following scores for papery taste:
(i) a score for papery taste of at least 0.5, preferably at least 0.7 lower than the score for papery taste of a beverage prepared in the same manner from wild-type barley, preferably from cv. Quench or cv. Rosalina after incubation at 37°C for one week;
(ii) a score for papery taste at least 0.5, preferably at least 0.7, more preferably at least 0.8, at least 1 lower than the score for papery taste of a beverage prepared in the same manner from wild-type barley, preferably from cv. Power or cv. Rosalina after incubation at 37°C for two weeks.

Interestingly, the present invention discloses that the overall flavour score of a beverage manufactured according to the present invention is improved compared to beverages prepared from wild-type barley. This may partly be attributed to the finding that DMS may mask certain desirable tastes.

Accordingly, it is preferred that the beverages of the present invention have an overall flavour score which is at least 1, preferably at least 1.5, more preferably at least 2 higher that the flavour score of a beverage prepared in the same manner from wild-type barley, preferably from cv. Quench or cv. Rosalina, when said beverage has been prepared without boiling the corresponding wort, preferably using a pressurized heating. Said flavour score should be scored on a scale from 1 to 9, where 9 represents the best score given by a specialist beer taste panel.

A beverage is said to have "stable organoleptic qualities", when said beverage comprises very low levels of free T2N, even after storage. Accordingly, it is an objective of the present invention to provide beverages (such as beer with stable organoleptic qualities), manufactured using a double-null-LOX-null-MMT barley plant.

Accordingly, it is preferred that the beverages of the present invention comprises less than 80%, preferably less than 70%, more preferably less than 65%, even more preferably less than 60% even more preferably less than 55% free T2N compared to a beverage prepared in the same manner from wild-type barley, preferably from cv. Quench or cv. Rosalina - after storage for at least 1 week, preferably at least 2 weeks, more preferably at least 3 weeks, even more preferably for at least 4 weeks at a temperature in the range of 30 to 40°C, preferably at 37°C. It is also preferred that the beverages of the invention comprises less than 0.025 ppb free T2N after storage for 2 weeks at 37°C.

In another specific embodiment, the beverage - preferably beer - is prepared from heated wort, which has only been heat treated for a short time, such as at a temperature in the range of 95 to 99°C for in the range of 10 to 30 min. Preferably said heated wort has been heated as described herein above in the section "Heating wort". In embodiments wherein the beverage - preferably beer - is prepared from such heated wort, then said beverage - preferably beer - comprises less than 80%, preferably less than 70%, more preferably less than 65% free T2N compared to a beverage prepared in the same manner from wild-type barley, preferably from cv. Quench or cv. Rosalina - after storage for at least 1 week, preferably at least 2 weeks, more preferably at least 3 weeks, even more preferably for at least 4 weeks at a temperature in the range of 30 to 40°C, preferably at 37°C.

In particular, it is preferred that such beverages - preferably beer - prepared from said heated wort comprise very low levels of T2N, preferably less than 80%, preferably less than 70%, more preferably less than 65% free T2N compared to a beverage prepared in the same manner from wild-type barley, preferably from cv. Quench or cv. Rosalina - after storage for 2 weeks at a temperature in the range of 30 to 40°C, preferably at 37°C in the presence of a level of sulphite not exceeding 10 ppm, preferably a level of sulphite in the range of 1 to 10 ppm, more preferably in the range of 1 to 8 ppm, more preferably in the range of 2 to 6 ppm, yet more preferably in the range of 3 to 6 ppm sulphite.

It is also particularly preferred that the beverages according to the invention - such as beer, for example barley beer - comprise very low levels of T2N, preferably less than 80%, preferably less than 70%, more preferably less than 60%, even more preferably less than 5% free T2N compared to a beverage prepared in the same manner from wild-type barley, preferably from cv. Quench or cv. Rosalina - after storage for 8 weeks at 37°C.

In one embodiment, the disclosure relates to beverages, such as beer, with low levels of certain trihydroxy octadecenoic acids (also denoted THAs), in particular to beverages with low levels of 9,12,13-THA and 9,10,13-THA. THAs are characterized by a bitter taste (Baur and Grosch, 1977; Baur et al., 1977), making said compounds undesirable in beverages.

It is thus desirable that the level of 9,12,13-THA and 9,10,13-THA is as low as possible, for example lower than 1.3 ppm, such as lower than 1 ppm. Accordingly, it is preferred that the level of 9,12,13-THA is as low as possible, for example lower than 1.3 ppm, such as lower than 1 ppm. It is also preferred that the level 9,10,13-THA is as low as possible, for example lower than 1.3 ppm, such as lower than 1 ppm. However, the overall concentration of 9,12,13-THA and 9,10,13-THA in a malt-derived beverage-such as beer - is also dependent on the amount of malt used for preparation of said specific beverage. Thus, in general, a strong beer will comprise more 9,12,13-THA and 9,10,13-THA than a lighter beer, making a higher over-all level of 9,12,13-THA and 9,10,13-THA acceptable in a stronger beer. Accordingly, it is preferred that the beverage according to the invention comprises a lower level of 9,12,13-THA and 9,10,13-THA than a beverage prepared in the same manner from wild-type barley, preferably from cv. Power or from cv. Quench or cv. Rosalina. In particular, a beverage according to the invention preferably has a level of 9,12,13 THA, which is at the most 50%, preferably at the most 40%, more preferably at the most 30% compared to the level in a beverage prepared in the same manner from a wild-type barley, preferably from cv. Power or cv. Rosalina. It is furthermore preferred that a beverage according to the invention has a level of 9,10,13-THA, which is at the most 70%, preferably at the most 60%, such as at the most 50%, for example at the most 40% compared to the level in a beverage prepared in the same manner from a wild-type barley, preferably from cv. Power or cv. Quench or cv. Rosalina. Such beverages may be prepared by using double-null-LOX-null-MMT barley.

In one embodiment, the beverages prepared from double-null-LOX-null-MMT barley have improved foam quality. This is in particular relevant when the beverage is a beer. Accordingly, it is an objective of the invention to provide beverages, such as beer, with superior foam quality. Preferably, the beverages of the invention produce at least 1.5 times more, preferably at least 2 times more, yet more preferably at least 3 times more foam in 60 to 80 min, preferably in 80 min as compared to a beverage prepared in the same manner from wild-type barley, preferably from cv. Quench. Said foam production is determined as described in Example 8 herein below.

### Plant products

It is an objective of the present invention to provide barley plant products characterized by low levels of one or more off-flavours. In particular, it is an objective of the invention to provide plant products, preferably barley plant products, with low levels of T2N, DMS and the corresponding precursors thereof. As described by the invention, such plant products may advantageously be prepared from double-null-LOX-null-MMT barley. Said plant product may be malt, preferably any of the malts described herein above in the section "Malting". The product may be wort, preferably any of the worts described herein above in the section "Wort"; it may also be a beverage, preferably any of the beverages described herein above in the section "Beverage". However, the plant product may also be other plant products that are characterized by low levels of T2N, T2N potential, DMS and DMSP prepared from double-null-LOX-null-MMT barley plants, or parts thereof.

The present invention thus relates to plant products, which are compositions comprising the barley plants described herein below, or parts thereof, or compositions prepared from said barley plants, or parts thereof, such as plant products prepared from said barley plants, or parts thereof. Because said barley plants lack LOX-1, LOX-2 and MMT activities, the compositions in general comprise very low levels of off-flavours and their precursor molecules and in particular of T2N, T2N potential, DMS and DMSP. Examples of useful plant products comprising, or prepared from, barley plants having a first mutation resulting in a total loss of functional LOX-1, a second mutation resulting in a total loss of functional LOX-2 and a third mutation resulting in a total loss of functional MMT are described herein.

It is preferred that said plant products comprise one or more of the following, preferably at least two of the following, even more preferably all of the following:
(i) less than 60%, even more preferably less than 50%, yet more preferably less than 40%, such as less than 30%, preferably less than 20%, more preferably less than 10%, free T2N;
(ii) less than 60%, even more preferably less than 50%, yet more preferably less than 40%, such as less than 30%, preferably less than 25% T2N potential;
(iii) less than 30%, preferably less than 20%, more preferably less than 15%, even more preferably less than 10% DMS;
(iv) less than 30%, preferably less than 20%, more preferably less than 15%, even more preferably less than 10%, such as less than 5%, for example less than 2% SMM;
(v) less than 30%, preferably less than 20%, more preferably less than 15%, even more preferably less than 10% of the DMSP;
as compared to a similar plant product prepared from wild-type barley plants, preferably from cv. Power or cv. Quench or cv. Rosalina, in the same manner.

The present invention relates, in one aspect, to barley kernels having a first mutation that results in a total loss of functional LOX-1, a second mutation resulting in a total loss of functional LOX-2 and a third mutation resulting in a total loss of functional MMT. The present invention also relates to compositions comprising said kernels, and compositions prepared from said kernels, as well as to plant products prepared from said kernels.

In a further aspect, the present invention relates to plant products, which may be food compositions, feed compositions, and fragrance raw material compositions that comprise double-null-LOX-null-MMT barley plants, or parts thereof. Food compositions, for example, may be, but are not limited to, malted and unmalted barley kernels, milled barley, barley meals, bread, porridge, cereal mixes comprising barley, health products, such as beverages comprising barley, barley syrups, and flaked, milled, micronized or extruded barley compositions. Feed compositions, for example, include compositions comprising barley kernels, and/or meals. Feed compositions may for example be mash. Fragrance raw material compositions are described herein below.

The invention also relates to mixtures of various plant products of the invention. For example, the invention relates to a composition prepared by a mixture of:
(i) a composition comprising a barley plant, or a part thereof, comprising a first mutation that results in a total loss of functional LOX-1, a second mutation resulting in a total loss of functional LOX-2 and a third mutation resulting in a total loss of functional MMT; and
(ii) a malt composition prepared from double-null-LOX-null-MMT kernels.

Various methods are available to determine whether a barley plant, or a plant product, is prepared from a barley plant carrying mutations in the genes for LOX-1, LOX-2 and MMT, causing a total loss of functional LOX-1, a total loss of functional LOX-2 and a total loss of functional MMT, respectively. Plant products will, in general, comprise at least some genomic DNA from the plant utilized for its production. Thus, malt will contain large amounts of genomic DNA, but even barley or malt extracts, such as wort, may comprise genomic DNA or fragments thereof from said barley or malt. Also barley-based beverages, such as beer, may comprise genomic DNA or fragments thereof from said plant. By analysis of DNA in a plant product, it may be established whether the plant, from which the plant product is prepared, carries mutations in the LOX-1, LOX-2 and MMT genes, causing a total loss of functional LOX-1, a total loss of functional LOX-2 and a total loss of functional MMT. Said mutations could, for example, be any of the mutations in the LOX-1 and LOX-2 genes described hereinbelow in the section "Loss of functional LOX". Said mutation in the MMT gene could, for example, be any of the mutations in the MMT gene described hereinabove in the section "Loss of functional MMT". The genomic DNA may be analyzed by any useful method, such as sequencing or by amplification-based methods, including PCR-based methods. If particular mutations in the LOX-1 gene and/or the LOX-2 gene and/or the MMT gene are assumed, then polymorphism analysis may be employed, for example SNP analysis. In relation to determination of a mutation in the LOX-1 gene and/or the LOX-2 gene a non-limiting example of a useful SNP analysis is described in international patent application PCT/DK2009/050355 (published as WO 2010/075860) in Example 10. In relation to determination of a mutation in the MMT gene a non-limiting example of a useful SNP analysis is described in international patent application PCT/DK2009/050315 (published as WO2010/063288) in Examples 13 and 17. The skilled person will be able to adapt the specific SNP analysis described in these examples for use with other mutations or other starting material.

If the above-mentioned plant products are prepared only from barley plants, which are double-null-LOX-null-MMT, then the presence vs. the absence of barley LOX-1 mRNA, LOX-2 mRNA and MMT mRNA and/or barley LOX-1 protein, LOX-2 protein and MMT protein may also be indicative of whether said plant product is prepared from a double-null-LOX-null-MMT barley plant. Examination of the plant product may also be accomplished by western blot analysis, or other protein analyses, or by RT-PCR, or by Northern blot analysis, or by other mRNA analyses. Such analyses are particularly useful when the plant product is malt.

### Barley plant

Cereal based beverages are described. Cereals may for example be selected from the group consisting of barley, wheat, rye, oat, maize, rice, sorghum, millet, triticale, buckwheat, fonio and quinona. More preferably, the cereal is selected from the groups consisting of barley, wheat, rye, oat, maize and rice, more preferably the cereal is barley.

Thus, the invention relates to barley based beverages and barley plants useful for preparing the beverages of the invention.

Barley is a family of plants. "Wild barley", Hordeum vulgare ssp. spontaneum, is considered the progenitor of today's cultivated forms of barley. The transition of barley from a wild to a cultivated state is thought to have coincided with a radical change of allele frequencies at numerous loci. Rare alleles and new mutational events were positively selected for by the farmers who quickly established the new traits in the domesticated plant populations, denoted "barley landraces". These are genetically more closely related to modern cultivars than wild barley. Until the late 19th century, barley landraces existed as highly heterogeneous mixtures of inbred lines and hybrid segregates, including few plants derived from random crossings in earlier generations. Most of the landraces have been displaced in advanced agricultures by pure line cultivars. Intermediate or high levels of genetic diversity characterize the remaining landraces. Initially, "modern barley" cultivars represented selections from landraces. These were later derived from successive cycles of crosses between established pure lines, such as those of diverse geographical origins. Eventually, the result was a marked narrowing of the genetic base in many, probably all, advanced agricultures. Compared with landraces, modern barley cultivars have numerous improved properties (Nevo, 1992; von Bothmer et al., 1992), for example one or more, but not limited to the following: (i) covered and naked kernels; (ii) seed dormancy; (iii) disease resistance; (iv) environmental tolerance (for example to drought or soil pH); (v) quantities of lysine and other amino acids; (vi) protein content; (vii) nitrogen content; (viii) carbohydrate composition; (ix) hordein content and composition; (x) (1-3,1-4)-β-glucan and arabinoxylan content; (xi) yield; (xii) straw stiffness; and (xiii) plant height.

Within the present invention, the term "barley plant" comprises any barley plant, such as barley landraces or modern barley cultivars. Thus, the invention relates to any barley plant comprising a first mutation resulting in a total loss of functional LOX-1, and a second mutation resulting in a total loss of functional LOX-2 and a third mutation resulting in a total loss of functional MMT. An example of such a barley plant is described in the Examples herein below and denoted "Triple-Null" or "Triple-Null barley".

However, preferred barley plants for use with the present invention are modern barley cultivars or pure lines. The barley cultivar to be used with the present invention may, for example, be selected from the group consisting of Sebastian, Quench, Celeste, Lux, Prestige, Saloon, Neruda, Harrington, Klages, Manley, Schooner, Stirling, Clipper, Franklin, Alexis, Blenheim, Ariel, Lenka, Maresi, Steffi, Gimpel, Cheri, Krona, Camargue, Chariot, Derkado, Prisma, Union, Beka, Kym, Asahi 5, KOU A, Swan Hals, Kanto Nakate Gold, Hakata No. 2, Kirin - choku No. 1, Kanto late Variety Gold, Fuji Nijo, New Golden, Satukio Nijo, Seijo No. 17, Akagi Nijo, Azuma Golden, Amagi Nijpo, Nishino Gold, Misato golden, Haruna Nijo, Scarlett, Rosalina and Jersey preferably from the group consisting of Haruna Nijo, Sebastian, Quench, Celeste, Lux, Prestige, Saloon, Neruda and Power, preferably from the group consisting of Harrington, Klages, Manley, Schooner, Stirling, Clipper, Franklin, Alexis, Blenheim, Ariel, Lenka, Maresi, Steffi, Gimpel, Cheri, Krona, Camargue, Chariot, Derkado, Prisma, Union, Beka, Kym, Asahi 5, KOU A, Swan Hals, Kanto Nakate Gold, Hakata No. 2, Kirin - choku No. 1, Kanto late Variety Gold, Fuji Nijo, New Golden, Satukio Nijo, Seijo No. 17, Akagi Nijo, Azuma Golden, Amagi Nijpo, Nishino Gold, Misato golden, Haruna Nijo, Scarlett and Jersey preferably from the group consisting of Haruna Nijo, Sebastian, Tangent, Lux, Prestige, Saloon, Neruda, Power, Quench, NFC Tipple, Barke, Class and Vintage.

In one embodiment of the invention, the barley plant is accordingly a modern barley cultivar (preferably a cultivar selected from the group of barley cultivars listed herein above) comprising a first mutation resulting in a total loss of functional LOX-1 and a second mutation resulting in a total loss of functional LOX-2 activity, and a third mutation resulting in a total loss of functional MMT. In this embodiment, it is thus preferred that the barley plant is not a barley landrace.

The barley plant may be in any suitable form. For example, the barley plant according to the invention may be a viable barley plant, a dried plant, a homogenized plant, or a milled barley kernel. The plant may be a mature plant, an embryo, a germinated kernel, a malted kernel, a milled malted kernel, a milled kernel or the like.

Parts of barley plants may be any suitable part of the plant, such as kernels, embryos, leaves, stems, roots, flowers, or fractions thereof. A fraction may, for example, be a section of a kernel, embryo, leaf, stem, root, or flower. Parts of barley plants may also be a fraction of a homogenate or a fraction of a milled barley plant or kernel.

In one embodiment, parts of barley plants may be cells of said barley plant, such as viable cells that may be propagated in vitro in tissue cultures. In other embodiments, however, the parts of barley plants may be viable cells that are not capable of maturing into an entire barley plant, i.e. cells that are not a reproductive material.

### Loss of functional LOX

The present invention relates to barley plants - or part thereof, or plant products thereof - having a first, a second mutation and a third mutation, wherein the first mutation leads to a total loss of functional LOX-1, and the second mutation leads to a total loss of functional of LOX-2. The third mutation leads to a total loss of functional MMT as is described in more detail in the section "Loss of function MMT" herein below.

The total loss of functional LOX-1 and the total loss of functional LOX-2 may independently be based on different mechanisms. For example, the total loss of function of one or both of LOX-1 and LOX-2 activities may be caused by malfunctioning proteins in the barley plant, i.e. a malfunctioning LOX-1 and/or LOX-2 protein, such as a mutated LOX-1 protein with no detectable 9-HPODE-forming activity (wherein 9-HPODE preferably may be determined as described in Example 4 of international patent application PCT/DK2009/050355 (published as WO 2010/075860), see below), and/or a mutated LOX-2 protein with no detectable 13-HPODE forming activity (wherein 13-HPODE preferably may be determined as described in Example 4 of international patent application PCT/DK2009/050355 (published as WO 2010/075860), see below).

The total loss of functional LOX-1 and/or LOX-2 may be caused by the lack of LOX-1 and/or LOX-2 protein. It is apparent that lack of LOX-1 protein will lead to loss of functional LOX-1, and that lack of LOX-2 protein will lead to total loss of functional LOX-2. Thus, the barley plant may preferably comprise no - or only very little, more preferably no detectable - LOX-1 and/or LOX-2 protein. The LOX-1 and/or LOX-2 protein(s) may be detected by any suitable means known to the person skilled in the art. Preferably, however, the protein(s) is detected by techniques wherein LOX-1 protein is detected by specific LOX-1 and LOX-2 antibodies, such as polyclonal antibodies to LOX-1 and LOX-2. Said techniques may, for example, be Western blotting or ELISA. Said antibodies may be monoclonal or polyclonal. Preferably, however, said antibodies are of a polyclonal nature, recognizing several different epitopes within the LOX-1 and LOX-2 protein, respectively. LOX-1 and/or LOX-2 protein may also be detected indirectly, for example, by methods determining LOX-1 activity, or by methods determining LOX-2 activity. In one preferred embodiment, LOX-1 protein is detected using the methods outlined in Example 4 of the international patent application WO 2005/087934. LOX-2 protein may be detected in a similar manner, using antibodies binding to barley LOX-2.

The total loss of function of one or both of LOX-1 and LOX-2 activities may also be a result of no, or very little, preferably no expression of a LOX-1 transcript and/or a LOX-2 transcript. The skilled person will acknowledge that the absence of a LOX-1 and/or a LOX-2 transcript also will result in the absence of translated LOX-1 and/or LOX-2 protein, respectively. Alternatively, the total loss of functional LOX-1 and functional LOX-2 may also be a result expression of an aberrant LOX-1 transcript and/or an aberrant LOX-2 transcript. An aberrant LOX-1 and/or LOX-2 transcript may be caused by aberrant splicing of the transcript, for example, due to a mutation in a splice site. Thus, the barley plants of the invention may carry a mutation in a splice site, such as a 5' splice site or a 3' splice site, for example in one or the two most 5' nucleotides of an intron, or in one of the most 3' nucleotides of an intron. An example of a mutant with aberrant splicing of the LOX-1 transcript is described as mutant A618 in WO 2005/087934. Expression of transcripts encoding LOX-1 or LOX-2 may, for example, be detected by Northern blotting or RT-PCR experiments.

Mutations have caused the total loss of functional LOX-1 and LOX-2 enzymes of the barley plants of the present invention. Thus, the barley plants of the present invention in general carry a mutation in the LOX-1 gene. Said mutation may be in the regulatory regions, for example within the promoter or introns, or said mutation may be in the coding region. The mutation may also be deletion of the LOX-1 gene or part thereof, such as deletion of the entire coding region. Similarly, the barley plants of the present invention in general carry a mutation in the LOX-2 gene. Said mutation may be in the regulatory regions, for example within the promoter or introns, or said mutation may be in the coding region. The mutation may also be deletion of the LOX-2 gene or part thereof, such as deletion of the entire coding region. Thus, the cause of the total loss of functional LOX-1 and/or LOX-2 enzymes may also be detected by the identification of mutations in the gene encoding LOX-1, or in the gene encoding LOX-2. Mutations in the genes encoding LOX-1 and LOX-2 may, for example, be detected by sequencing said genes. Preferably, after identifying a mutation, the total loss of function is confirmed by testing for LOX-1 and/or LOX-2 activities.

The term "LOX-1 protein" is meant to cover the full-length LOX-1 protein of barley as set forth in SEQ ID NO:3 (corresponding to SEQ ID NO:3 of WO 2005/087934), or in SEQ ID NO:7 of WO 2005/087934, or a functional homolog thereof. The active site of LOX-1 is situated in the C-terminal part of the enzyme. In particular, it is anticipated that the region spanning amino acid residues 520-862, or parts thereof, (preferably the entire region of amino acids nos. 520-862) are relevant for LOX-1 activity. Accordingly, in one embodiment, null-LOX-1 barley preferably comprises a gene that encodes a mutated form of LOX-1 that lacks some or all of amino acids 520-862 of LOX-1. Said mutated LOX-1 may also lack other amino acid residues, which are present in wild-type LOX-1.

Accordingly, double-null-LOX barley of the invention may comprise a truncated form of LOX-1, which is not functional - such as an N- or a C-terminal truncated form. Preferably, said truncated form comprises no more than 800, more preferably no more than 750, even more preferably no more than 700, yet more preferably no more than 690, even more preferably no more than 680, yet more preferably no more than 670 consecutive amino acids of LOX-1, such as no more than 665, for example no more than 650, such as no more than 600, for example no more than 550, such as no more than 500, for example no more than 450, such as no more than 425, for example no more than 399 consecutive amino acids of LOX-1 of SEQ ID NO:3 (corresponding to SEQ ID NO:3 of WO 2005/087934). Preferably, said truncated form comprises only an N-terminal fragment of LOX-1, preferably at the most the 800, more preferably at the most the 750, even more preferably at the most the 700, yet more preferably at the most the 690, even more preferably at the most the 680, yet more preferably at the most the 670, even more preferably at the most the 665 N-terminal amino acids of SEQ ID NO:3 (corresponding to SEQ ID NO:3 of WO 2005/087934), such as no more than 665, for example no more than 650, such as no more than 600, for example at the most the 550, such as at the most the 500, for example at the most the 450, such as at the most the 425, for example at the most the 399 N-terminal amino acids of SEQ ID NO:3 (corresponding to SEQ ID NO:3 of WO 2005/087934). In addition to the fragment of LOX-1, said truncated form may optionally comprise additional C-terminal sequences not present in wild-type LOX-1. This may in particular be the case if the truncated form has arisen from aberrant splicing. Preferably, said additional C-terminal sequences consist of at the most 50, more preferably at the most 30, even more preferably at the most 10, yet more preferably of at the most 4, or at the most 1 amino acid.

In one very preferred embodiment, the truncated form may consist of amino acids 1-665 of SEQ ID NO:3 (corresponding to SEQ ID NO:3 of WO 2005/087934).

In a preferred embodiment, the barley plant comprises a LOX-1-encoding gene that is transcribed into mRNA, which comprises a nonsense codon or a stop codon upstream of the stop codon of wild-type LOX-1 mRNA. Such a nonsense codon is herein denoted a premature nonsense codon. Preferably, all LOX-1-encoding genes transcribed into mRNA of said plant comprise a premature nonsense codon or a stop codon. The nonsense codon or stop codon is preferably situated at the most 800, more preferably at the most the 750, even more preferably at the most the 700, yet more preferably at the most the 690, even more preferably at the most the 680, yet more preferably at the most the 670, even more preferably at the most the 665 codons downstream of the start codon. The sequence of wild-type genomic DNA encoding LOX-1 is given in SEQ ID NO:1 (corresponding to SEQ ID NO:1 of WO 2005/087934) or SEQ ID NO:5 of WO 2005/087934.

In one preferred embodiment, the barley plant comprises a gene encoding LOX-1, wherein the corresponding pre-mRNA transcribed from said gene comprises the sequence corresponding to SEQ ID NO: 2 (corresponding to SEQ ID NO:2 of WO 2005/087934).

In a very preferred embodiment, the gene encoding mutant LOX-1 of the double null-LOX barley plant according to the invention comprises a nonsense mutation, said mutation corresponding to a G→A substitution at position 3574 of SEQ ID NO: 1 (corresponding to SEQ ID NO:1 of WO 2005/087934).

The term "LOX-2 protein" is meant to cover the full-length LOX-2 protein of barley as set forth in SEQ ID NO:7 (corresponding to SEQ ID NO:5 of international patent application PCT/DK2009/050355 (published as WO 2010/075860)), or a functional homolog thereof. The active site of LOX-2 is situated in the C-terminal part of LOX-2. In particular, it is anticipated that the region spanning amino acid residues 515-717, or parts thereof, are relevant for LOX-2 activity. Based on an examination of the soybean LOX-1 crystal structure, anticipated sequence stretches of the active site cleft of the LOX-2 enzyme of barley are represented by amino acid residues 515-525 and 707-717. A translated, mutated LOX-2 protein, i.e. a C-terminally truncated form of LOX-2 of barley double null-LOX mutant A689 contains max. 684 residues, and will therefore lack the second sequence stretch of the active site cleft - making it inactive. According to one embodimen, double-null-LOX barley of the invention preferably comprises a gene encoding a mutant form of LOX-2 that lacks some, or all, of amino acids 515-717 of LOX-2, preferably lacking some or all of amino acids 707 to 717, even more preferably lacking all of amino acids 707-717. Said mutant LOX-2 may also lack other amino acid residues, which are present in wild-type LOX-2.

Accordingly, double-null-LOX barley may comprise a truncated form of LOX-2, which is not functional, such as an N-terminal or a C-terminal truncated form. Preferably, said truncated form comprises no more than 800, more preferably no more than 750, even more preferably no more than 725, yet more preferably no more than 700, even more preferably no more than 690, yet more preferably no more than 684 consecutive amino acids of LOX-2 of SEQ ID NO:7 (corresponding to SEQ ID NO:5 of international patent application PCT/DK2009/050355 (published as WO 2010/075860)). Preferably, said truncated form comprises only an N-terminal fragment of LOX-2. Hence, preferably said truncated form comprises at the most the 800, more preferably at the most the 750, even more preferably at the most the 725, yet more preferably at the most the 700, even more preferably at the most the 690, yet more preferably at the most the 684 N-terminal amino acids of SEQ ID NO:7 (corresponding to SEQ ID NO:5 of international patent application PCT/DK2009/050355 (published as WO 2010/075860). In addition to the fragment of LOX-12, said truncated form may optionally comprise additional C-terminal sequences not present in wild-type LOX-2. This may in particular be the case if the truncated form has arisen from aberrant splicing. Preferably, said additional C-terminal sequences consist of at the most 50, more preferably at the most 30, even more preferably at the most 10, yet more preferably of at the most 4, or at the most 1 amino acid.

In one very preferred embodiment, the truncated form may consist of amino acids 1-684 of SEQ ID NO: 7 (corresponding to SEQ ID NO:5 of international patent application PCT/DK2009/050355 (published as WO 2010/075860)).

In a preferred embodiment, the barley plant comprises a gene transcribed into mRNA for LOX-2, wherein said mRNA comprises a nonsense codon or a stop codon upstream of the stop codon of wild-type LOX-2 mRNA. Such a nonsense codon is herein designated a premature nonsense codon. Preferably all genes transcribed into mRNA encoding LOX-2 of said plant comprise a premature nonsense codon or a stop codon. The nonsense codon or stop codon is preferably situated at the most 800, more preferably at the most the 750, even more preferably at the most the 725, yet more preferably at the most the 700, even more preferably at the most the 690, yet more preferably at the most the 684 codons downstream of the start codon. The sequence of wild-type genomic DNA encoding LOX-2 is given in SEQ ID NO: 5 (corresponding to SEQ ID NO:1 of international patent application PCT/DK2009/050355 (published as WO 2010/075860)).

In a very preferred embodiment, the gene encoding mutated LOX-2 of the double null-LOX barley plant comprises a nonsense mutation, said mutation corresponding to a G→A substitution at position 2689 of SEQ ID NO: 5 (corresponding to SEQ ID NO:1 of international patent application PCT/DK2009/050355 (published as WO 2010/075860)).

The barley plant according to the invention may be prepared by any suitable method known to the person skilled in the art, preferably by one of the methods outlined herein below in the section "Preparing double-null-LOX-null-MMT barley".

### Loss of functional MMT

The present invention relates to barley plants - or part thereof, or plant products thereof - having a first, a second mutation and a third mutation, wherein the first mutation leads to a total loss of functional LOX-1, and the second mutation leads to a total loss of functional of LOX-2 - both described herein above in more detail in the section "Loss of functional LOX". The third mutation leads to a total loss of functional MMT.

The total loss of a functional MMT may be based on different mechanisms. For example, the total loss of functional MMT may result from a malfunctioning protein in said plant, i.e. a malfunctioning MMT enzyme, such as a mutant MMT protein with no detectable activity. For instance, the MMT protein of the mutant may be a truncated protein. The loss of MMT activity may similarly be based on different mechanisms, for example caused by a malfunctioning MMT protein.

Preferably, the activity of a mutated MMT protein is determined by its capacity to catalyze transfer of a methyl group from SAM to Met, thereby forming SMM. This may, for example, be undertaken as described in Example 4 in international patent application PCT/DK2009/050315 (published as WO2010/063288), see below. Preferably, the amino acid sequence of a mutated MMT is obtained by determining the translated sequence of the corresponding, isolated barley cDNA. This may be done essentially as described in Example 8 of international patent application PCT/DK2009/050315 (published as WO2010/063288). Alternatively, the mutated MMT of a barley plant of the invention is obtained by heterologous expression in a bacterial cell culture as described in Example 11 and Example 12 in international patent application PCT/DK2009/050315 (published as WO2010/063288), followed by verifying that the recombinant protein is inactive as an MMT enzyme.

The total loss of functional MMT may be realized by the lack of MMT protein. Lack of MMT protein will lead to loss of MMT function. Thus, the barley plant may comprise no, or only very little, preferably no detectable MMT protein. The presence or absence of MMT protein may be detected by any suitable means known to the person skilled in the art. However, the protein(s) is preferably analyzed by techniques wherein MMT protein is detected by specific antibodies that recognize MMT. Said techniques may, for example, be western blotting or enzyme-linked immunosorbent assay, and said specific antibodies may be monoclonal or polyclonal. Preferably, however, said antibodies are polyclonals that recognize several different epitopes within the MMT protein. This may also be detected indirectly, for example, by methods for MMT activity determination. Thus, in the invention, a barley plant is said to carry a mutation in the gene encoding MMT, thus causing a total loss of MMT activity, when no MMT protein is detectable in said plant. In particular, this is the case when no MMT protein with an approximate mass of 120 kDa, ±10%, is detectable in said barley plant - preferably in kernels of said barley plant, as analyzed by western blotting.

The total loss of functional MMT may also be a result of no, or very little, preferably no, transcription of an MMT mRNA. The skilled person will acknowledge that the absence of an MMT transcript also will result in the absence of MMT protein.

Preferably, however, the total loss of functional MMT is a result of expression of an aberrant MMT transcript. Said transcript may preferably be caused by an aberrant splicing event of the primary transcript, for example, due to a mutation in a splice site. Expression of transcripts encoding MMT may, for instance, be detected by Northern blotting, or by RT-PCR methods.

The total loss of functional MMT in the barley plants of the present invention is caused by one or more mutations. Thus, the barley plants of the present invention, in general, carry at least one mutation in the MMT gene. Said mutation(s) may be in regulatory regions, for instance within the promoter, or introns, or said mutation(s) may be in the protein coding region. The mutation may also be deletion of the MMT gene or part thereof, for example deletion of the coding region of the MMT gene. Thus, the loss of functional MMT may also be detected by analyzing for mutations in the gene encoding MMT. Mutations in the MMT-encoding gene may, for example, be detected by sequencing said gene, followed by comparing it to the wild-type sequence, preferably the wild-type sequence of cv. Prestige given in SEQ ID NO:9 (corresponding to the sequence given in international patent application PCT/DK2009/050315 (published as WO2010/063288) as SEQ ID NO:3), or that of cv. Sebastian (SEQ ID NO:11 corresponding to SEQ ID NO:16 of international patent application PCT/DK2009/050315 (published as WO2010/063288)). Preferably, after identifying a mutation, the loss of function is confirmed by testing for MMT activity, for instance as described in Example 2 or Example 4 (see below) of international patent application PCT/DK2009/050315 (published as WO2010/063288).

The term MMT protein is meant to cover the full length MMT protein of barley as set forth in SEQ ID NO:13 (corresponding to SEQ ID NO:6 of international patent application PCT/DK2009/050315 (published as WO2010/063288)), or a functional homolog thereof. In this context, a functional homolog is an MMT protein with the same level of MMT activity, ±25%, as that of the MMT protein of barley as set forth in SEQ ID NO:13, wherein the MMT activity is determined as described in Example 2 or Example 4 (see below) of international patent application PCT/DK2009/050315 (published as WO2010/063288).

The barley plant carrying a third mutation causing a total loss of MMT activity may comprise a non-functional, truncated form of MMT, such as an N-terminal or a C-terminal truncated form. A barley plant may comprise more than one truncated form of MMT, such as 2, or for example 3, or such as more than 3 different truncated forms of MMT, which may result from aberrantly spliced transcripts. Said truncated forms comprise only an N-terminal fragment of MMT. In addition to the N-terminal fragment of wild-type MMT, said truncated forms of MMT may comprise additional C-terminal sequences not found in wild-type MMT. Said additional C-terminal sequences may, for instance, be translated intron sequences, such as those comprised in the mutant mRNA due to aberrant splicing. Preferably, said truncated MMT forms comprise at the most the 500, more preferably at the most the 450, even more preferably at the most the 400, yet more preferably at the most the 350, even more preferably at the most the 320, yet more preferably at the most 311, or at the most 288 N-terminal amino acid residues of SEQ ID NO:13 (corresponding to SEQ ID NO:6 of international patent application PCT/DK2009/050315 (published as WO2010/063288)). This is in particular the case when said barley plant has a total loss of MMT activity. However, the MMT may also comprise less, such as no more than 300, for example no more than 250, such as no more than 200, for example at the most the 150, for example no more than 147, or no more than 133 N-terminal amino acids of SEQ ID NO:13 (corresponding to SEQ ID NO:6 of international patent application PCT/DK2009/050315 (published as WO2010/063288)).

In one very preferred embodiment, the truncated MMT form may consist of amino acids 1-311 or amino acids 1-288 of SEQ ID NO:13 (corresponding to SEQ ID NO:6 of international patent application PCT/DK2009/050315 (published as WO2010/063288)) and optionally additional C-terminal sequences not present in wild-type MMT. Preferably, said additional C-terminal sequences consist of at the most 50, more preferably at the most 30, even more preferably at the most 10, yet more preferably of at the most 4, or at the most 1 amino acid. In a very preferred embodiment, the truncated form of MMT may be the protein according to SEQ ID NO: 18 (corresponding to SEQ ID NO:11 of international patent application PCT/DK2009/050315 (published as WO2010/063288)), or SEQ ID NO: 19 (corresponding to SEQ ID NO:13 of international patent application PCT/DK2009/050315 (published as WO2010/063288)), or SEQ ID NO: 20 (corresponding to SEQ ID NO:15 of international patent application PCT/DK2009/050315 (published as WO2010/063288)). None of the proteins of SEQ ID NO: 18, SEQ ID NO: 19 or SEQ ID NO: 20 (corresponding, respectively to SEQ ID NO:11, SEQ ID NO:13, and SEQ ID NO:15 of international patent application PCT/DK2009/050315 (published as WO2010/063288)) represent functional MMT enzymes.

In another very preferred embodiment, the truncated MMT form may consist of amino acids 1-147, or of amino acids 1-133, of SEQ ID NO: 14 (corresponding to SEQ ID NO:18 of international patent application PCT/DK2009/050315 (published as WO2010/063288)), and optionally additional C-terminal sequences not present in wild-type MMT. Preferably, said additional C-terminal sequences consist of at the most 50, more preferably at the most 40, even more preferably at the most 39, or at the most 33, or at the most 30 amino acids. In a very preferred embodiment, the truncated form of MMT may be the protein according to SEQ ID NO:15, SEQ ID NO:16 or SEQ ID NO:17 (corresponding to SEQ ID NO:22, or SEQ ID NO:24, or SEQ ID NO:26 of international patent application PCT/DK2009/050315 (publishes as WO2010/063288), respectively). None of the proteins of SEQ ID NO:15, SEQ ID NO:16 or SEQ ID NO:17 (corresponding to SEQ ID NO:22, or SEQ ID NO:24, or SEQ ID NO:26 of international patent application PCT/DK2009/050315 (published as WO2010/063288), respectively) are functional MMT enzymes.

The above-mentioned truncated forms of MMT may, for example be present in barley plants carrying a mutation in the MMT gene, wherein said mutation introduces a premature stop codon resulting in a gene encoding above-mentioned truncated forms of MMT.

In a preferred embodiment, the barley plant comprises an MMT gene that is transcribed into mRNA, which comprises some, but not all, of the wild-type MMT gene spliced together without intervention (the intron-exon structure of the wild-type MMT gene of barley is shown in FIG. 9 of international patent application PCT/DK2009/050315 (published as WO2010/063288)). In one embodiment it is accordingly preferred that the MMT mRNA of the barley plant according to the invention comprises at the most exons 1, 2, 3, 4, and 5 spliced together without intervention, or for example at the most exons 1 and 2 spliced together without intervention. In addition to said spliced-together exons, the MMT mRNAs of the barley plant according to the invention may comprise additional 3' terminal sequences derived from wild-type introns and/or exons, wherein introns separate exons sequences. Preferred examples of aberrant MMT mRNAs of barley plants according to the invention - as determined by RT-PCR and accordingly with fragment lengths in bp - are illustrated in FIG. 12 and FIG. 16 of international patent application PCT/DK2009/050315 (published as WO2010/063288). More preferably, the aberrant mRNAs of barley plants according to the invention are those illustrated in FIG. 12 of international patent application PCT/DK2009/050315 published as WO2010/063288), further comprising exons 1 and 2 at the 5' end, or the mRNAs illustrated in FIG. 16 of international patent application PCT/DK2009/050315 (published as WO2010/063288), further comprising exon 1 at the 5' end.

In a very preferred embodiment, the barley plant carrying a third mutation in the gene for MMT causing a total loss of functional MMT comprises a mutation in a splice site within the MMT gene, which results in aberrantly spliced mRNA. More preferably, said mutation is positioned in an intron of the MMT gene, even more preferably in the 5' splice site of an intron, such as in the 5' splice site on intron 1 (the intron separating exons 1 and 2), such as in the 5' splice site on intron 2 (the intron separating exons 2 and 3), such as in the 5' splice site on intron 3 (the intron separating exons 3 and 4), such as in the 5' splice site on intron 4 (the intron separating exons 4 and 5), such as in the 5' splice site on intron 5 (the intron separating exons 5 and 6), such as in the 5' splice site on intron 6 (the intron separating exons 6 and 7), most preferably in the 5' splice site on intron 2 or intron 5.

It is preferred that said mutation is a G→A mutation of the terminal 5' base of the aforementioned introns. Thus, a very preferred mutation is a G→A mutation of the terminal 5' base of intron 2, or a G→A mutation of the most 5' base of intron 5.

The barley plant according to the invention may be prepared by any suitable method known to the person skilled in the art, preferably by the method outlined herein below in the section "Preparing double-null-LOX-null-MMT barley".

### Preparing double-null-LOX-null-MMT barley

The barley plant according to the invention may be prepared by any suitable method known to the person skilled in the art. Preferably, the barley plant of the invention is prepared by a method comprising the steps of mutagenizing barley plants or parts thereof, for example barley kernels, followed by screening and selecting barley plants characterized by a total loss of functional LOX-1, total loss of functional LOX-2 and/or total loss of functional MMT.

The barley plants according to the invention comprise at least 3 mutations. Accordingly, the plants may be prepared by preparing separate barley plants that comprise only one of the mutations and thereafter crossing said barley plants to obtain a barley plant with all of the 3 mutations - or by successively introducing the mutations into a barley plant or by a combination of these methods.

Thus, the barley plant according to the invention may be prepared by mutagenizing a barley plant or parts thereof, for example barley kernels, followed by screening and selecting barley plants characterized by a total loss of functional LOX-1, and mutagenizing another barley plant or parts thereof, for example barley kernels, followed by screening and selecting barley plants characterized by a total loss of functional LOX-2 and mutagenizing yet another barley plant or parts thereof, for example barley kernels, followed by screening and selecting barley plants characterized by a total loss of functional MMT. The selected barley plants may eventually be crossed in several rounds to obtain barley plants carrying all of the three mutations.

Alternatively, the barley plant of the present invention may be prepared by mutagenizing barley plants or parts thereof, for example barley kernels, followed by screening and selecting barley plants characterized by a total loss of functional XX. Said selected barley plants may optionally be propagated and then these barley plants - or parts thereof, for example barley kernels - may be mutagenised, followed by screening and selecting barley plants characterized by a total loss of functional YY. Said selected barley plants, or parts thereof, may optionally be propagated and then either:
(i) these barley plants - or parts thereof, for example barley kernels - may be mutagenised, followed by screening and selecting barley plants characterized by a total loss of functional ZZ; or
(ii) these barley plants may be crossed with a barley plant characterized by total loss of functional ZZ.

In the above-mentioned crossings, XX, YY and ZZ each denotes either LOX-1, LOX-2 or MMT, wherein XX is different to YY, which is different to ZZ.

In one preferred embodiment, the barley plant may be prepared by a method involving mutagenizing barley plants, or parts thereof, for example barley kernels, wherein said barley plants already carry a mutation causing a total loss of functional LOX-1 enzyme followed by screening and selecting barley plants further carrying a mutation that causes a total loss of functional LOX-2 (i.e. null-LOX-1-null-LOX-2, or double-null-LOX plants). This method furthermore involves mutagenizing other barley plants, or parts thereof and screening and selecting barley plants with total loss of functional MMT and eventually crossing these barley plants with the null-LOX-1-null-LOX-2 barley plants.

Suitable null-LOX-1 barley plants are, for example, described in international patent application WO 2005/087934.

It is preferred that the screening method utilises germinated embryos as starting material for the identification of barley plants characterized by a total loss of functional LOX-2. Interestingly, the present inventors have found that the use of mature embryos as starting material for a screening for LOX-2 activity is less preferable, based on the screening of as many as 21,000 mature embryos, which did not reveal a single null-LOX-2 barley mutant.

It is an objective of the present disclosure to provide methods of preparing a double-null-LOX-null-MMT barley plant comprising the steps of: (i) preparing a double-null-LOX barley plant; and (ii) preparing a null-MMT barley plant; (iii) crossing said double-null-LOX barley plant and said null-MMT barley plant; (iv) selecting double-null-LOX-null-MMT barley plants.

Preparing said double-null-LOX barley plants may preferably be done by a method comprising the steps of:
(i) providing a barley plant, or parts thereof, with a total loss of function of LOX-1 activity, such as total loss of functional LOX-1 enzyme; and
(ii) mutagenizing said barley plant, and/or barley cells, and/or barley tissue, and/or barley kernels, and/or barley embryos from said barley plant, thereby obtaining generation M0 barley; and
(iii) breeding said mutagenized barley plants, kernels, and/or embryos for at least 2 generations, thereby obtaining generation Mx barley plants, wherein x is an integer ≥2; and
(iv) obtaining embryos from said Mx barley plants; and
(v) germinating said embryos; and
(vi) determining the LOX-1 and LOX-2 activities in said germinated embryos, or parts thereof; and
(vii) selecting plants with a total loss of LOX-1 activity and LOX-2 activity in the germinated embryos; and
(viii) analyzing for a mutation in the LOX-1 gene and in the LOX-2 gene; and
(ix) Selecting plants carrying a mutation in the LOX-1 gene and the LOX-2 gene, i.e. double-null-LOX plants;
thereby obtaining a barley plant carrying mutations in the genes for LOX-1 and LOX-2, causing a total loss of functional LOX-1 and functional LOX-2.

Preparing said null-MMT barley plants may preferably be done using a method comprising the steps of:
(i) mutagenizing barley plants, and/or barley cells, and/or barley tissue, and/or barley kernels, and/or barley embryos, thereby obtaining generation M0 barley; and
(ii) propagating, e.g. by breeding, said mutagenized barley plants, kernels, and/or embryos for ≥2 generations, thereby obtaining barley plants of generation Mx, wherein x is an integer ≥2; and
(iii) obtaining a sample of said Mx barley plants; and
(iv) determining the level of SMM in said sample; and
(v) selecting plants wherein the sample comprises less than 10 ppb SMM, preferably less than 5 ppb SMM, more preferably no detectable SMM; and
(vi) sequencing at least part of the MMT gene; and
(vii) selecting plants carrying a mutation in the MMT gene.

The aforementioned barley plant with a total loss of LOX-1 activity may, for example, be any of the barley plants with a total loss of LOX-1 activity described in WO 2005/087934, preferably mutant D112, or progeny plants thereof.

The mutagenizing steps in the aforementioned methods may involve mutagenizing living material selected from the group consisting of barley plants, barley cells, barley tissue, barley kernels, and barley embryos - preferably selected from the group consisting of barley plants, barley kernels, and barley embryos, more preferably barley kernels.

Mutagenesis may be performed by any suitable method. In one embodiment, mutagenesis is performed by incubating a barley plant, or a part thereof - for example barley kernels or individual cells from barley - with a mutagenizing agent. Said agent is known to the person skilled in the art, including, for example, but not limited to, sodium azide (NaN₃), ethyl methanesulfonate (EMS), azidoglycerol (AG, 3-azido-1,2-propanediol), methyl nitrosourea (MNU), and maleic hydrazide (MH).

In another embodiment, mutagenesis is performed by irradiation, for example by UV, a barley plant or a part thereof, such as the kernel. In preferred embodiments, the mutagenesis is performed according to any of the methods outlined herein below in the section "Chemical mutagenesis". A non-limiting example of a suitable mutagenesis protocol is given in Example 2 of international patent application PCT/DK2009/050355 (published as WO 2010/075860).

It is preferred that the mutagenesis is performed in a manner such that the expected frequency of desired mutants is at least 0.5, such as in the range of 0.5 to 5, for example in the range of 0.9 to 2.3 per 10,000 grains, when screening barley of generation M3. In a preferred embodiment, mutagenesis is performed on barley kernels. The kernels applied to the mutagen are designated as generation M0 (see also FIG. 8).

The LOX activity may be determined in a sample consisting of germinating barley embryo, preferably in a liquid extract of a germinating barley embryo. Said sample, such as said extract may be prepared from any suitable part of said germinating embryo. In general, the barley sample must be homogenized using any suitable method prior to preparation of an extract of said sample and determination of LOX-2 activity. In particular, it is preferred that a protein extract is prepared from the germinating embryo, or part thereof, and that the LOX activity is determined using said extract. Homogenization may, for example, be performed using mechanical forces, for example by shaking or stirring, such as by shaking in the presence of a bead, such as a glass or a sand bead.

In a preferred embodiment, the germinating embryo is of generation Mx, wherein x is an integer ≥2; preferably x is an integer in the range of 2 to 10, more preferably in the range of 3 to 8. In a very preferred embodiment, LOX activity is determined in germinating embryos of generation M3, or a sample derived from such embryos. In that embodiment, it is preferred that mutagenized barley kernels of generation M0 are grown to obtain barley plants, which are crossed to obtain kernels of generation M1. The procedure is repeated until kernels of generation M3 are available (see also FIG. 8).

Determination of LOX activity may be carried out using any suitable assay, preferably by one of the methods outlined hereinafter. In particular, it is preferred that the assay provides data on the dioxygenation of linoleic acid to 9-HPODE and 13-HPODE by LOX-1 and LOX-2. In general, assaying will therefore involve the steps of:
(i) providing a protein extract prepared from a germinated barley embryo or part thereof; and
(ii) providing linoleic acid; and
(iii) incubating said protein extract with said linoleic acid; and
(iv) detecting dioxygenation of linoleic acid to 9-HPODE and 13-HPODE.

Step (iv) of the method preferably comprises determining the level of 9-HPODE and 13-HPODE in said germinating embryos, preferably in a protein extract prepared from said germinating embryos. The step may comprise a direct or an indirect determination of the levels of 9-HPODE and 13-HPODE. The total level of all HPODEs may be determined, in which case it is preferred that specific measurements of 9-HPODE and 13-HPODE are performed for confirmation. One method could, for example, be a method wherein protein extracts from germinating embryos are incubated with linoleic acid as substrate for formation of 9-HPODE and 13-HPODE. Said HPODEs can then be detected by various methods. One method may involve generation of a detectable compound, such as a dye. For example the method may be the oxidative coupling of 3-dimethylaminobenzoic acid and 3-methyl-2-benzothiazolinone hydrazone in the presence of hemoglobin, catalyzed by the formed HPODEs to form the indamine dye, which can be measured at A₅₉₅ using a spectrophotometer. An example of such a method is described in Examples 1 and 2 in international patent application PCT/DK2009/050355 (published as WO 2010/075860). Using this assay, an absorption reading of less than 0.2 A₅₉₅ unit is considered as indicative of the absence of LOX-1 and the absence of LOX-2 activities. However, a more precise method for determining LOX-1 and LOX-2 activities is to incubate a protein extract from germinating embryos with linoleic acid, followed by determination of 9-HPODE and 13-HPODE contents. 9-HPODE and 13-HPODE contents may, for example, be determined using HPLC-based analysis.

Dioxygenation of linoleic acid to 9-HPODE and 13-HPODEs may be measured directly or indirectly. Any suitable detection method may be used with the present invention. In one embodiment, linoleic acid hydroperoxides are detected. 9-HPODE and 13-HPODE may be detected directly, for example, by chromatographic methods, such as HPLC as described in Example 4 in international patent application PCT/DK2009/050355 (published as WO 2010/075860), see below.

Certain aspects of the procedure for extraction of protein from the germinating embryo for determination of LOX activity are of great importance. Thus, it is preferred that the protein is extracted using an acidic buffer, preferably a buffer with a pH in the range of 2 to 6, more preferably in the range of 3 to 5, even more preferably in the range of 3.5 to 5, yet more preferably in the range of 4 to 5, even more preferably a pH of 4.5. The buffer used for extraction is preferably based on an organic acid, more preferably a lactic acid buffer. Most preferably, the protein extract is prepared using a 100-mM lactic acid buffer, pH 4.5.

Certain methods for detection of null-LOX-1 and null-LOX-2 plants that involve reaction of 9-HPODE and 13-HPODE with a dye, e.g. 3-methyl-2-benzothiazolinone hydrazone. Preferably, said dye, e.g. 3-methyl-2-benzothiazolinone hydrazone, is added to the protein extract after addition of linoleic acid. Preferably, the dye is added at least 1 min, more preferably at least 5 min, even more preferably at least 10 min, such as in the range of 1 to 60 min, for example in the range of 5 to 30 min, such as in the range of 10 to 20 min after contacting the protein extract with the linoleic acid.

Preferred methods for selecting barley plants according to the invention are detailed in Example 2 of international patent application PCT/DK2009/050355 (published as WO 2010/075860).

The selection procedure may be adjusted for microtitre plate-based assay procedures, or other known repetitive, high-throughput assay formats to allow rapid screening of many samples. It is preferred that at least 5000, such as at least 7500, for example at least 10,000, such as at least 15,000, for example at least 20,000, such as at least 25,000 mutagenized barley plants are analyzed for LOX-1 and LOX-2 activities.

Determination of a mutation in the gene encoding LOX-1 may be performed by several different methods. For example, the LOX-1 gene may be sequenced completely or partly, and the sequence compared to SEQ ID NO:1 (corresponding to SEQ ID NO:1 of WO 2005/087934) or SEQ ID NO:5 of WO 2005/087934. If searching for a specific mutation, SNP analysis may be applied. The skilled person will be able to design useful primers for detection of a given specific mutation, such as one leading to a premature stop codon in the coding sequence for LOX-1 (e.g. any of the premature stop codons described hereinabove). One example of how to perform a SNP analysis is described in Example 10 of international patent application PCT/DK2009/050355 (published as WO 2010/0/75860), with primers that are useful for detecting a G→A mutation at nucleotide position 3474 of the LOX-1 gene.

Determination of a mutation in the gene encoding LOX-2 may be performed by several different methods. For example, the LOX-2 gene may be sequenced completely or partly, and the sequence compared to SEQ ID NO:5 (corresponding to SEQ ID NO:1 of international patent application PCT/DK2009/050355 published as WO 2010/075860).

If searching for a specific mutation, SNP analysis may be used. The skilled person will be able to design useful primers for detection of a given specific mutation, such as one leading to a premature stop codon in the LOX-2 coding sequence (e.g. any of the premature stop codons described hereinabove). An example of how to perform a SNP analysis is described in Example 10 in international patent application PCT/DK2009/050355 (published as WO 2010/075860), as are primers useful for detecting a G→A mutation at nucleotide position 2689 of the gene for LOX-2.

Steps (viii) and (ix) of the method of preparing a double null-LOX barley plant, as detailed in this section hereinabove, may be performed prior to steps (vi) and (vii), in which case the method will comprise the steps (i), (ii), (iii), (iv), (v), (viii), (ix), (vi), and (vii) in that order. In particular, this could be the case when searching for a specific mutation, for example in progeny plants of already identified double null-LOX barley plants.

Preferably, selection of barley plants with total loss of functional MMT comprises obtaining a sample from a mutagenized barley plant, preferably from a germinating mutagenized barley plant, even more preferably from a mutagenized barley plant, which has germinated for 4 d. It is preferred that the sample is from a coleoptile and/or a primary leaf, preferably from a leaf. Thus, the sample may, for example, be in the range of 1 cm to 3 cm leaf tissue.

The sample may be extracted and analyzed following a newly developed multistep protocol, as described herein, involving the successive use of different solvents and binding materials. In general, the sample may be extracted, for example with a solvent or a mixture of solvents, preferably water and/or organic solvents. The organic solvent may, for example, be an alcohol, preferably methanol - or the organic solvent may for example be an alkyl-halide, preferably chloroform. In one preferred embodiment, the solvent is a mixture of water, methanol, and chloroform. Said extraction may advantageously be performed while mixing, for example, using a shaker or a mixer. A solid support may be added to the solvent/sample mixture - for instance a bead, such as a glass bead.

In a preferred embodiment, the aforementioned leaf sample for determination of MMT activity is taken from generation Mx kernels, wherein x is an integer ≥2, preferably in the range of 2 to 10, more preferably in the range of 3 to 8. In a very preferred embodiment, the level of SMM is determined in M3-germinated plants, or in samples thereof (such as leaves). In said embodiment, it is preferred that mutagenized barley kernels of generation M0 are grown to obtain barley plants, which subsequently are crossed to obtain kernels of generation M1. The procedure is repeated until kernels of generation M3 are available (cf. FIG. 8).

Determination of the SMM level is preferably based on the novel procedure described below. Interestingly, this method allows for high-throughput screenings, rendering it feasible to identify barley plants characterized by a total loss of functional MMT.

In general terms, the method preferably involves reacting the sample, or preferably an extract of said sample, prepared as described above, with a compound capable of binding SMM. It was found that the OPA reagent (Sigma, cat.no. P7914; cf. FIG. 2 of international patent application PCT/DK2009/050315 (published as WO2010/063288)), hereinafter just referred to as OPA, is particularly useful for determining SMM levels. OPA reacts, amongst others, with SMM to form the molecule referred to as SMM-OPA (cf. FIG. 2 of international patent application PCT/DK2009/050315 (published as WO2010/063288)). The reaction preferably involves incubating OPA with an extract of the sample prepared as described above. In addition, it is preferred that 3-mercaptopropionic acid is added to the reaction mixture. The mixture is preferably kept at alkaline pH, preferably in the range pH 8 to pH 11, more preferably in the range pH 9 to pH 11, even more preferably in the range pH 9.5 to pH 10.5, such as at pH 10. Incubation is preferably performed at a temperature in the range of 0°C to 10°C, preferably in the range of 1 °C to 8°C, even more preferably in the range of 2°C to 6°C, yet more preferably in the range of 3°C to 5°C, such as at 4°C. Incubation time is preferably ≥10 min.

Based on the observation that SMM-OPA absorbs and emits light of 340 nm and 450 nm, respectively, its detection was possible by using fluorescence spectroscopy. The initial process of detection preferably involves extract separation over a column, preferably on a 30x2 mm Gemini 3µ C18 column (Phenomenex, cat.no. 00A-4439-80; Phenomenex, 2006), followed by fluorescence detection using a high-throughput liquid chromatography system, preferably an Ultra Performance Liquid Chromatography (UPLC system, Waters), designed to identify and measure the fluorescent level of molecules having excitation at 340 nm and emission at 450 nm. When using this method, "no detectable SMM" means the absence of detectable compounds that co-elute with SMM. In this context, a small "shoulder" on a chromatogram peak is considered an artifact peak. A small shoulder on the right hand side of the Asn/Ser peak, cf. FIG. 2, is accordingly not considered to represent a SMM peak. Thus, by way of example, the upper two chromatograms as shown in FIG. 2B are considered to depict "no detectable SMM", whereas the lower chromatogram in said figure represents the separation of a sample comprising SMM.

Detection of SMM may preferably be done as described in Example 2 or Example 4. A preferred method for selecting barley plants according to the invention is described hereinafter in Example 2. Tissue for analysis is preferably sampled from a germinating barley plant, even more preferably from a barley plant, which has germinated for 4 days. It is notable that the above-mentioned screening method is particularly useful. First of all the analytical method is novel. Furthermore, it is a significant advantage of the above method that it is established for determination of SMM levels in germinating barley plants, such as leaves of germinating barley plants. The timing of sampling from the germinating barley makes an unexpectedly clean preparation for UPLC-based detection of SMM. Other samples, for example wort samples of similar grains as described above are too complex in composition, and can generally not be utilized in the mentioned chromatography method for determination of SMM levels.

Subsequent to the identification of a barley plant having less than 10 ppb SMM, preferably no detectable SMM, the corresponding MMT gene, or part thereof, is typically sequenced to determine whether the barley plant in question can be classified as having a mutation in the MMT gene. Barley plants characterized by having no detectable SMM, and wherein one or more bases of the MMT-encoding gene are different as compared with the wild-type sequence, are then selected. In this context, the wild-type sequence is preferably the sequence found in the corresponding wild-type barley cultivar, preferably the sequence given as SEQ ID NO:9 (corresponding to SEQ ID NO:3 in international patent application PCT/DK2009/050315 (published as WO2010/063288)). Preferred mutations are described hereinabove.

Selected barley mutants may be further propagated, and plants of subsequent generations re-screened for SMM content. After selection of useful barley plants, these may be included in breeding programs utilizing those conventional methods that are described herein below in the section "Plant breeding".

Once a double-null-LOX-null-MMT barley plant has been identified, which contains a particular mutation in the LOX-1 gene and a particular mutation in the LOX-2 gene and a particular mutation in the MMT gene (such as any of the above-mentioned mutations), additional barley plants with the identical mutations may be generated by conventional breeding methods, such as those well known to the skilled person. For example, said double-null-LOX barley plant may be backcrossed with another barley cultivar.

Subsequent to the selection of useful barley plants with total loss of functional LOX-1, LOX-2 and MMT, one or more additional screenings may optionally be performed. For example, selected mutants may be further propagated, and plants of new generations may be tested for the total loss of functional LOX-1, LOX-2 and MMT.

In one embodiment, it is preferred that the double-null-LOX-null-MMT barley plant according to the present invention has plant growth physiology and grain development similar to that of wild-type barley. It is hence preferred that the double-null-LOX-1-null-MMT barley plant is similar to wild-type barley (preferably to cv. Power or cv. Quench or cv. Rosalina) with respect to plant height, number of tillers per plant, onset of flowering, and/or number of grains per spike.

Also, it is preferred that the double-null-LOX-null-MMT barley plant according to the present invention is similar to wild-type barley, in particular similar to cv. Power or cv Quench with respect to plant height, heading date, disease resistance, lodging, ear-breakage, maturation time, and yield. In the present context, "similar" is to be understood as the same ±10% in case of numbers. These parameters may be determined as described hereinafter in Example 5.

In a very preferred embodiment, the barley plant is prepared by crossing the barley Line A689 (ATCC Patent Deposit Designation: PTA-9640), with the barley Line 8063 (ATCC Patent Deposit Designation: PTA-9543) and optionally followed by further breeding.

Seeds of barley line A689 have been deposited 4 December 2008 under the name "Barley, Hordeum vulgare L.; Line A689" with American Type Culture Collection (ATCC), Patent Depository, 10801 University Blvd., Manassas, VA 20110, United States (deposit number PTA-9640).

Seeds of barley line 8063 have been deposited on 13 October 2008 with American Type Culture Collection (ATCC), Patent Depository, 10801 University Blvd., Manassas, VA 20110, United States and referred to as "Barley, Hordeum vulgare; Line 8063" (ATCC Patent Deposit Designation: PTA-9543).

### Chemical mutagenesis

In order to generate double-null-LOX-null-MMT barley plants according to the present invention, a very large number of barley mutants are prepared - typically in multiple rounds - by any suitable mutagenesis method, for example by the use of chemical mutagenesis of barley kernels. This method is known to introduce mutations at random. Mutagenesis of barley may be performed using any mutagenizing chemical. However, it is preferably performed by treating kernels with NaN₃, letting the surviving kernels germinate, followed by analysis of off-spring plants. The plant generation growing from the mutagenized kernels, referred to as M0, contains heterozygote chimeras for any given mutation. Progeny plants collected after self-pollination are referred to as the M1 generation, in which a given mutation segregates into the corresponding heterozygotes and homozygotes (cf. **FIG. 8****).**

Treating kernels with NaN₃ is not equivalent to treating a single cell, because the kernels after the treatment will contain some non-mutant cells and a variety of cells having DNA mutations. Since mutations in cell lineages that do not lead to the germ line will be lost, the goal is to target the mutagen to the few cells that develop into reproductive tissues which contribute to development of the M1 generation.

To assess the overall mutation efficiency, albino chimeras and albino plants may be counted in the generations M0 and M1. Scoring mutant number as a function of surviving plants gives an estimate for the mutation efficiency, while scoring mutant number as a function of treated seeds measures the combination of both mutation efficiency and kernel kill.

It is notable that cells have quality assurance mechanisms at virtually every step of gene expression, possibly to moderate the effects of damaging mutations. One well-studied example in eukaryotes is nonsense-mediated mRNA decay, denoted NMD, which prevents the synthesis of potentially deleterious, prematurely truncated proteins (Maquat and Carmichael, 2001; Wu et al., 2007). In NMD, a termination codon is identified as premature by its position relative to downstream destabilizing elements. Mutations that generate premature termination (nonsense) codons (PTCs) sometimes increase the levels of alternatively spliced transcripts that skip the offending mutations, thereby potentially saving protein function (Mendell and Dietz, 2001).

### Plant breeding

In one embodiment, the objective is to provide agronomical useful barley plants comprising the double-null-LOX-null-MMT trait. Crop development is often a lengthy and difficult process that begins with the introduction of the new trait. From the perspective of a plant breeder, however, this step almost always results in a plant that has a less desirable overall profile of agronomic traits than do current commercial varieties.

In addition to the double-null-LOX-null-MMT trait, there are additional factors which also may be considered in the art of generating a commercial barley variety useful for malting and/or brewing and/or as base for beverages, for example kernel yield and size, and other parameters that relate to malting performance or brewing performance. Since many - if not all - relevant traits have been shown to be under genetic control, the present disclosure also provides modern, homozygous, high-yielding malting cultivars, which may be prepared from crosses with the double-null-LOX-null-MMT barley plants that are disclosed in the present publication. The skilled barley breeder will be able to select and develop barley plants, which - following crossings with double-null-LOX barley-null-MMT barley - will result in superior cultivars. Alternatively, the barley breeder may utilize plants of the present invention for further mutagenesis to generate new cultivars derived from double-null-LOX-null-MMT barley.

One method to ensure that the double-null-LOX-null-MMT trait is maintained in progeny lines concerns SNP analysis of the LOX-1 gene, the LOX-2 gene and the MMT gene. Preferably, LOX-1, LOX-2 and MMT activities are also determined.

The barley plants according to the present invention may be introduced into any suitable breeding scheme.

Another objective is to provide agronomical elite barley plants comprising the double-null-LOX-null-MMT trait. Accordingly, this disclosure also is directed to methods for producing a new double-null-LOX-null-MMT barley plant by crossing a first parental barley plant with a second parental barley plant, wherein the first or second plant is a double-null-LOX-null-MMT barley. Additionally, both first and second parental barley plants can come from a double-null-LOX-null-MMT barley variety. Thus, any such methods using the double-null-LOX-null-MMT barley variety are part of this disclosure: selfing, backcrossing, crossing to populations, and the like. All plants produced using a double-null-LOX-null-MMT barley variety as a parent are within the scope of this invention, including those plants developed from varieties derived from a double-null-LOX-null-MMT barley variety. The double-null-LOX-null-MMT barley can also be used for genetic transformation in such cases where exogenous DNA is introduced and expressed in the double-null-LOX-null-MMT plant or plant tissue.

Backcrossing methods can be used with the present invention to introduce into another cultivar the double-null-LOX-null-MMT trait of a mutated barley plant, for example cv. Scarlett or cv. Jersey or cv. Quench or cv. Rosalina, which are contemporary, high-yielding malting barley cultivars. In a standard backcross protocol, the original variety of interest, i.e. the recurrent parental plant, is crossed to a second variety (non-recurrent parental plant), carrying the mutant LOX genes of interest to be transferred. The resulting double null-LOX progeny plants from this cross are subsequently crossed to the recurrent parental plant, with the process being repeated until a barley plant is obtained wherein essentially all of the characteristics specified by the recurrent parent are recovered in the generated plant - in addition to the double-null-LOX-null-MMT trait of the nonrecurrent parental plant. Eventually, the last-generated, backcrossed plant is selfed to yield a pure double-null-LOX-null-MMT breeding progeny plant.

A way to accelerate the process of plant breeding comprises the initial multiplication of generated mutants by application of tissue culture and regeneration techniques. Thus, another aspect of the present disclosure is to provide cells, which upon growth and differentiation produce barley plants having the double-null-LOX-null-MMT trait. For example, breeding may involve traditional crossings, preparing fertile anther-derived plants or using microspore culture.

### LOX pathway products

In various embodiments, the present disclosure relates to barley plants, and products thereof, comprising low levels of T2N and T2N potential. LOX enzymes catalyze dioxygenation of polyunsaturated fatty acids with a cis-1,cis-4 pentadiene system. In barley, the C₁₈ polyunsaturated fatty acids linoleic acid (18:2^{Δ9,12}) and α-linolenic acid (18:3^{Δ9,12,15}) are major LOX substrates. The lipoxygenase pathway of fatty acid metabolism is initiated by the addition of molecular oxygen at the C-9 position (mostly catalyzed by LOX-1) or C-13 position (mostly catalyzed by LOX-2) of the acyl chain, yielding the corresponding 9- and 13-HPODEs [9- and 13-hydroperoxy octadecatrienoic acids (HPOTEs) are products when the substrate is α-linolenic acid, but HPOTEs do not function as precursors for T2N]. In the hydroperoxide lyase branch of the LOX pathway, both 9- and 13-HPODEs may be cleaved to short-chain oxoacids and aldehydes (cf. **FIG. 1A****).** In particular, 9-HPODE may be cleaved to form cis-nonenal that is converted to T2N, whereas 13-HPODE is the precursor of 2-E-hexenal. Thus, 13-HPODE, the major product of LOX-2-catalyzed dioxygenation of linoleic acid was not anticipated to be an upstream component in the pathway leading to formation of the stale flavour T2N.

It is recognized that the present disclosure encompasses influencing production of downstream metabolites of LOX-1 and LOX-2 catalysis, which are not produced as a direct product of a LOX-1 or LOX-2-catalyzed reaction, but as a result of a subsequent series of reactions. These include spontaneous, factor-induced, or enzyme-catalyzed isomerizations and conversions. Thus, the production of these downstream metabolites could be influenced by modulating the expression of other components of the pathway, for example hydroperoxide lyase (HPL).

### T2N and DMS and precursors thereof

The present invention relates to methods for preparing beverages with low levels of one or more off-flavours and precursors thereof. Preferably said off-flavours are T2N and DMS and said precursors thereof are T2N potential and DMSP, respectively.

One objective of the present invention is thus to reduce or eliminate the T2N potential. Thus, it is an objective of the present invention to reduce the formation of T2N precursors and aldehyde adducts. Although several chemical reactions related to beer staling remain elusive, generation of free T2N from T2N potential is recognized as a major cause of the development of stale flavour in beer products (Kuroda et al., supra). Therefore, it is an objective of the present invention to provide beverages with low level of T2N potential as well as beverages with low level of T2N precursors.

Most of the T2N potential is transferred from wort to the finished beer, in which free T2N may be liberated (Liégeois et al., 2002), with the conditions of acidity and temperature being important factors in this process. With reference to the present invention, T2N potential is defined as described hereinabove in the definitions. Other methods for determining the level of T2N potential are also available. In order to avoid confusion, the meaning of "T2N potential" in the present context is as described herein above in the definitions. The chemical substances which have the capacity to release T2N or be converted into T2N are denoted "T2N precursors" herein, and T2N precursors determined or measured by alternative methods other than the method for determining T2N potential are referred to as "T2N precursors". T2N precursors may in particular be determined by first treating a sample such that essentially all (preferably all) of its chemical substances, which have the capacity to release T2N or be converted into T2N actually do release T2N and/or convert to T2N, respectively. Thereafter, the level of T2N is determined.

Barley kernels of the instant invention comprise no LOX-1 and LOX-2 activities in addition to no MMT activity. Interestingly, such barley kernels contain very little T2N potential.

Beers produced using double-null-LOX-null-MMT barley kernels will therefore not only possess a very low level of T2N, but also a very low level of T2N potential. Within the scope of the present disclosure are double-null-LOX-null-MMT barley kernels, which yield beer products that contain very low levels of T2N potential, preferably less than 60%, more preferably less than 50% of the level of T2N potential of a similar beer product produced in the same manner from wild-type barley (preferably cv. Power).

Also, it is preferred that plant products derived from double-null-LOX-null-MMT barley kernels possess a very low level of T2N precursors. Within the scope of the present invention are plant products prepared from double-null-LOX-null-MMT barley kernels, said plant products containing less than 60%, more preferably less than 50% T2N precursors of a similar plant product produced in the same manner from wild-type barley (preferably cv. Power).

It is notable that measured T2N values often are higher in samples of, and in products from, a micro-malted raw material than that from a raw material produced in larger scale, for example from a 30-kg-large pilot-malted sample. However, the relative, experimental values of T2N between large- and small-scale experiments are in general similar.

Similarly, it is notable that measured T2N potentials and T2N precursors often are higher in samples of, and in products from, a micro-malted raw material than that from a raw material produced in larger scale, for example from a 30-kg-large pilot-malted sample. However, the relative, experimental values of T2N potentials between large- and small-scale experiments are in general similar.

It is also an objective of the present invention to reduce or eliminate DMS and DMSP, wherein DMSP preferably is SMM.

The amount of SMM and DMS in a plant product may be determined by any suitable method. SMM may be determined essentially as described hereinabove in the section "Preparing double-null-LOX-null-MMT barley plants", wherein is described determination of SMM levels in a barley sample. Thus, SMM may be determined by coupling it to a compound, such as OPA, and determining fluorescence, for example, by using a UPLC system. For a quantitative measurement, the chromatogram area corresponding to a SMM peak may be determined.

For a more precise measure, the amounts of both DMS and DMSP (such as SMM), the latter compound measured as DMS after activation, are preferably determined using high resolution capillary gas chromatography. Total DMS in samples of wort or beer are defined herein as the quantitative sum of free DMS and its precursor forms, denoted DMSP. Using this definition, the quantity of DMSP in a wort or beer sample can be determined as the difference between total DMS (measured in the boiled sample, preferably in a sample boiled at alkaline conditions for 1 h), and free DMS (measured in the non-boiled sample). Example 4 details preferable ways to measure levels of total and free DMS.

The amount of DMSP and also of SMM herein is given as the concentration of DMS which may be liberated from said DMSP or said SMM by boiling in alkaline conditions for 1 h.

### EXAMPLES

The examples herein illustrate the invention and should not be considered as limiting for the invention.

Unless otherwise indicated, basic molecular biological techniques were performed for manipulating nucleic acids and bacteria as described in Sambrook and Russel (2001).

### Example 1

### Screening for low LOX-2 activity in germinating barley embryos

Improved screening material. Kernels collected from barley plants of null-LOX-1 line Ca211901 - generated by the crosses (null-LOX-1 mutant D112 x Jersey) x Sebastian - were incubated with the mutagen NaN₃ according to the details provided by Kleinhofs et al. (1978). Barley null-LOX-1 mutant D112 is described in WO 2005/087934 and deposited with American Type Culture Collection (ATCC), 10801 University Boulevard, Manassas, Va. 20110, USA on September 11, 2003, under the number PTA-5487.

This procedure was chosen since it is known to induce point mutations in the genomic DNA of barley, eventually conferring amino acid residue substitutions or truncations in proteins encoded by the mutagenized DNA. In the mutagenesis experiments of the instant publication, it was chosen to propagate mutated grains of generation M1 in field plots through two subsequent generations, eventually yielding a high proportion of homozygous plants for screening purposes (cf. FIG. 8). While grains of generation M2 were not screened, primarily because these were expected to contain a relatively high proportion of heterozygous point mutations, mutant grains of generation M3 were used as screening material, expecting 0.9-2.3 mutations per 10,000 grains (Kleinhofs et al., supra).

Surprisingly, the instant inventors found that analysis of germinating embryos provided much-improved assay results as compared to analysis of extracts of mature embryos (as described in Example 1 of international application PCT/DK2009/050355 (published as WO 2010/075860)). A high-throughput screening procedure was therefore established to measure LOX-2 activity in the germinating embryo, including its scutellum tissue.

Two embryos were isolated from mature grains of 35,125 barley ears (20,977 lines of generation M4 of null-LOX-1 mutant D112, and 14,148 lines of generation M3 of null-LOX-1 line Ca211901lines), and transferred to 96-well storage plates (ABgene). Embryo germination was initiated following addition of 20 µL water to each well, which was covered with a wet Kimnett tissue and a plastic lid. The plates were incubated in plastic bags at 20°C for 48 h. After incubation, LOX-2 enzyme was extracted; to each well was first added a 5-mm glass bead and 200 µL of extraction buffer (100 mM lactic acid solution, pH 4.5), followed by milling for 35 sec at a frequency of 27 sec⁻¹ in an MM 300 laboratory mill (Retsch). Subsequently, the plate was centrifuged at 4,000 rpm for 10 min at 4°C in an Allegra 6R centrifuge (Beckman-Coulter), to precipitate insoluble material. LOX-2 activity was determined basically as described for analysis of LOX-2 activity of mature embryo extracts (cf. Example 1 in international application PCT/DK2009/050355 (published as WO 2010/075860)), only differing in the usage of only 30 µL extract per assay instead of 40 µL.

Identification of potential mutants. As described above, two grains each of the above-mentioned 35,125 barley lines were analyzed for LOX-2 activity, with the aim to identify grains highly reduced in said activity when compared with null-LOX-1 and wild-type grains. A total of 7 potential raw mutants were identified in the M3 generation of line Ca211901. These were further propagated in the greenhouse, harvested, and then re-screened for the trait related to very low LOX activity. Eventually, only one mutant of line Ca211901, denoted mutant A689, was shown to exhibit essentially no LOX-2 activity. Detailed measurements of total LOX activity were performed with extracts of germinated embryos in which the LOX activity was conferred almost exclusively by LOX-2 (Schmitt and van Mechelen, 1997). For germinated embryos of M3 grains of mutant A689, the total LOX activity - as determined by the colourimetric LOX assay - was 0.163±5.5% A₅₉₅ U/germinated embryo, while that for the null-LOX-1 mother variety Ca211901 was 1.224±3.8% A₅₉₅ U/germinated embryo (the corresponding value for null-LOX-1 raw mutant D112 was 1.215±6.0% A₅₉₅ U/germinated embryo). Seeds of barley line A689 have been deposited 4 December 2008 under the name "Barley, Hordeum vulgare L.; Line A689" with American Type Culture Collection (ATCC), Patent Depository, 10801 University Blvd., Manassas, VA 20110, United States (deposit number PTA-9640).

An analysis for HPODE in mutant A689 is described in Example 4 of international patent application PCT/DK2009/050355 (published as WO 2010/075860), see below.

Properties of mutant A689 are described in Example 5 of international patent application PCT/DK2009/050355 (published as WO 2010/075860).

Sequencing of the gene for LOX-2 in barley mutant A689 is described in Example 10 of international patent application PCT/DK2009/050355 (published as WO 2010/075860) and Table 7 therein summarizes the mutations in the LOX-1 and LOX-2 genes of mutant A689.

A method for detecting the double-null-LOX mutant A689 is described in Example 11 of international patent application PCT/DK2009/050355 (publishes as WO 2010/075860). The method is a SNP based method for detecting the mutation in LOX-1 and the mutation in LOX-2.

### Example 2

### Screening for null-MMT barley mutants

Kernels collected from barley plants of cv. Prestige and cv. Sebastian were incubated separately with the mutagen NaN₃, following the experimental details provided by Kleinhofs et al. (1978). This procedure was chosen because of its known potential for inducing point mutations in the barley genomic DNA.

In the experiments, mutated grains of generation M1 were propagated in field plots through two subsequent generations, eventually yielding a high proportion of homozygous plants of generation M3 for screening purposes. Mutated grains of generation M3 were expected to contain gene mutations at a frequency of 0.9-2.3 per 10,000 grains (Kleinhofs et al., supra). It is notable that M2 grains were not screened.

Interestingly, the present disclosure describes a rapid high-throughput screening procedure for detection of M3 mutant barley grains lacking MMT activity, providing lack of detectable SMM synthesis during malting. Thus, the inventors found that SMM mainly accumulated in the coleoptile and primary leaf of germinating barley, and that detection of SMM can be performed by extracting amino acids from crushed leaf tissue of 4-d-old germinated grains, followed by reacting the extracted amino acids with OPA to form highly fluorescent products (cf. **FIG. 2****).**

In practical terms, each assay was performed by germinating - in a closed plastic box with one piece of Whatman #1 filter paper (296x20.9 mm) -two grains from each of 94 potential mutants and two wild-type plants. The assay was repeated for multiple, potential mutant grains (see below). At the beginning of germination, 25 mL of tap water was added to said plastic box, followed by additional 15 mL of tap water at 2 d of germination. After 4 d of germination, 1-3 cm of leaf tissues were transferred to storage plates (ABgene), in which each of the 96 1.2-mL wells contained a 5-mm-diameter glass bead and 500 µL of a 12:5:6 (v/v/v) mixture of water:methanol:chloroform. The plate was then shaken for 45 sec at a frequency of 30 Hz in an MM 300 laboratory mill (Retsch). Subsequently, the plate was transferred to a centrifuge (Rotanta 460R, Hettich), and spun at 4,000 rpm for 15 min at room temperature to precipitate insoluble material. 10 µL of the supernatant was transferred to a 96-well storage plate (Waters, cat no. 186002481), and mixed with 200 µL H₂O and 60 µL of a reaction solution containing a 15,000:45 (v/v) mixture of OPA reagent (Sigma, cat.no. P7914):3-mercaptopropionic acid (Aldrich, cat.no. M5801). The mixture was incubated at 4°C for at least 10 min to obtain a quantitative derivatization of sample amino acids with OPA. Using a Waters-based UPLC system equipped with a fluorescence detector, 2 µL of the derivatized mixture was separated on a 2.1 x30-mm C18 Gemini column of 3-µm particles (Phenomenex, cat.no. 00A-4439-80), using gradient elution by mixing mobile phase A (a 40-mM NaH₂PO₄ buffer, adjusted to pH 7.8) and mobile phase B [a 45:45:10 (v:v:v) solution of acetonitrile:methanol:water as described (Phenomenex, 2006)]. Excitation of eluted OPA derivatives was at 340 nm, while light emission was measured at 450 nm. An example of a chromatogram is shown in **FIG. 2** to illustrate the elution profile of aspartic acid (Asp), glutamic acid (Glu), asparagine (Asn), serine (Ser) and SMM. The latter compound was included, as the overall project aim was to identify a barley plant that lacked the capacity to synthesize SMM, i.e. a plant for which the corresponding chromatogram peak was very small or preferably absent.

A total of 10,248 and 3,858 NaN₃-mutated kernels of barley cv. Prestige and cv. Sebastian, respectively, were screened for SMM content, with the aim to identify those highly reduced in said content when compared with wild-type grains. Only 2 potential mutants of the M3 generation were identified, namely grains of sample no. 8,063 (derived from cv. Prestige, and hereinafter denoted Mutant 8063, a designation also used for grains of subsequent generations), and grains of sample no. 14,018 (derived from cv. Sebastian, and hereinafter denoted Mutant 14018, a designation also used for grains of subsequent generations). Grains of each mutant were propagated to the M4 generation, then harvested, and eventually re-analyzed. The result verified that grains of Mutant 8063 and Mutant 14018 had extremely low SMM contents, possibly totally lacking SMM.

Western blot analysis has verified that Mutant 8063 and Mutant 14018 lacked MMT enzyme (see Example 3 of international patent application PCT/DK2009/050315 (published as WO2010/063288)).

Also MMT activity measurements has verified that Mutant 8063 lacks MMT activity (see Example 4 of international patent application PCT/DK2009/050315 (published as WO2010/063288)).

Sequencing of the gene for MMT in barley mutant 8063 as described in Example 9 of international patent application PCT/DK2009/050315 (published as WO2010/063288) revealed a G→A base transition at the first base of intron 5 (nucleotide no. 3076 of SEQ ID NO:10 - corresponding to SEQ ID NO:8 of international patent application PCT/DK2009/050315 (published as WO2010/063288)). Sequencing of the gene for MMT in barley mutant 14018 as described in Example 14 of international patent application PCT/DK2009/050315 (published as WO2010/063288) revealed a G→A base transition in a splice donor site immediately downstream of exon 2 at the first base of intron 2, more specifically at nucleotide no. 1462.

It has furthermore been confirmed that MMT mRNA is truncated in mutant 8063 (see Example 11 of international patent application PCT/DK2009/050315 (published as WO2010/063288)) and that the mutant MMT protein encoded by said truncated mRNA has no MMT activity (see Example 12 of international patent application PCT/DK2009/050315 (published as WO2010/063288)). It has also been confirmed that MMT mRNA is truncated in mutant 14018 (see Example 15 of international patent application PCT/DK2009/050315 (published as WO2010/063288)) and that the mutant MMT protein encoded by said truncated mRNA has no MMT activity (see Example 16 of international patent application PCT/DK2009/050315 (published as WO2010/063288)).

A method for detecting the presence of the mutation in the MMT gene of mutant 8063 is described in Example 11 of international patent application PCT/DK2009/050315 (published as WO2010/063288), and a method for detecting the presence of the mutation in the MMT gene of mutant 14018 is described in Example 17 of international patent application PCT/DK2009/050315 (published as WO2010/063288).

### Example 3

### Barley crossings

**FIG. 3** summarizes how the double-null-LOX-null-MMT barley line of the instant invention was developed by first crossing barley line A689 [double null-LOX cf. PCT patent application no PCT/DK2009/050355 (published as WO 2010/075860)] with line 8063 [null-MMT cf. PCT Patent Application No. PCT/DK2009/050315 (published as WO2010/063288)]. Using standard breeding techniques, doubled haploid lines were developed, and propagated in the greenhouse. Of these, the best-performing lines with regard to agronomic performance - as well as an absence of LOX-1 activity (cf. Example 2 in U.S. Patent No. 7,420,105 to Breddam, K. et al.), an absence of LOX-2 activity (cf. Example 2 in PCT Application No. PCT/DK2009/050355 (published as WO 2010/075860), and Example 1 herein), as well as an absence of SMM and MMT activity (Examples 2 and 4 (see below) in PCT Application No. PCT/DK2009/050315 (published as WO2010/063288), and Example 2 herein) - were selected for further propagation and analysis. These lines are denoted "Triple-Null" herein. In general for LOX activity determinations, seeds of double haploid lines were harvested and followed by analysis of 12 grains of each line and control varieties, giving a <5% standard deviation for the measurements **(****FIG. 4****).**

### Example 4

### Determination of SMM levels

Measurement of SMM was performed essentially as described in PCT Application PCT/DK2009/050315 (published as WO2010/063288). First, SMM was extracted from 1-3-cm-long sections of barley leaves that were placed in 1.2-mL wells of microtitre plates, in which each well contained a 5-mm-diameter glass bead and 500 µL of a 12:5:6 (v/v/v) mixture of water:methanol:chloroform. The plate was then incubated for 45 sec in an MM 300 laboratory mill (Retsch), electronically adjusted to shake at a frequency of 30 Hz. After centrifugation, 10 µL of the supernatant was transferred to a 96-well storage plate (Waters, cat no. 186002481), and mixed with 200 µL of water and 60 µL of a reaction solution containing a 15,000:45 (v/v) mixture of OPA reagent (Sigma, cat. No. P7914) : 3-mercaptopropionic acid (Aldrich, cat. No. M5801). The mixture was incubated at 4°C for at least 10 min to quantitatively derivatize amino acids of the sample with OPA. Using an UPLC system (Waters) equipped with a fluorimeter, 2 µL of the derivatized mixture was separated on a 2.1 x30-mm C18 Gemini column of 3-µm particles (Phenomenex, cat. No. 00A-4439-80), using a 40-mM Na-phosphate buffer, adjusted to pH 7.8, and containing a 45:45:10 (v:v:v) solution of acetonitrile:methanol:water as the mobile phase (Phenomenex 2006). Excitation of OPA-derivatives were at 340 nm, while light emission was measured at 450 nm. An example of a chromatogram is shown in **FIG. 5** to illustrate the elution profile of aspartic acid (Asp), glutamic acid (Glu), asparagine (Asn), serine (Ser) and SMM from wild-type and the Triple-Null mutant. A notable lack of capacity to synthesize SMM was observed for the Triple-Null mutant.

### Example 5

### Agronomic performance

The commercial barley cvs. Quench and Power, as well as null-LOX-1, null-MMT, null-LOX-1-null-LOX-2 (double-null-LOX), null-LOX-1-null-MMT and Triple-Null plants were tested in field trials to compare their agronomic performances. Data were regularly obtained for plant height, heading date, disease resistance, lodging, maturation time and yield (see **Table 1).**

The trials were performed according to standard procedures for field trials. Accordingly, equal amounts of kernels of the commercial varieties and the mutant lines were sown in 7.88-m² plots in 2 locations, each comprising 3 replications. No major differences with respect to agronomic traits were observed between mutants and the commercial varieties. The barley quality analysis of the different mutant lines and varieties demonstrated that all of the harvested grains possessed good and acceptable properties with regard to malting and brewing.

### Example 6

### Micro-maltings and micro-mashings

### Experimental set-up

Micro-malting and -mashing experiments were performed with the following six different barley lines and cultivars **(****FIG. 6A****),** which also illustrates the experimental workflow described herein below): (1) Triple-Null; (2) null-LOX-1-null-MMT; (3) null-LOX-1-null-LOX-2 (barley line A689); (4) null-MMT (barley line 8063); (5) null-LOX-1 (barley line D112); (6) cv. Power.

A micro-malting experiment was done with three 225-g barley samples from each of the aforementioned lines or cultivar. Steeping and germination were performed as follows:
(i) steeping at 16°C: 3 h wet; 21 h dry; 3 h wet; 21 h dry; 3 h wet; 21 h dry;
   final water content 45%;
(ii) germination: 48-72 h at 16°C until modification >95%.

After germination, the three samples were subjected to different drying regimes (denoted kiln drying in **FIG. 6A****):**
(i) 85°C - Drying: 12.5 h starting at 30°C and ramping to 55°C followed by 7.5 h ramping to at 85°C; 1.5 h at 85°C;
(ii) 75°C - Drying: 12.5 h starting at 30°C and ramping to 55°C followed by 7.5 h ramping to at 75°C; 1.5 h at 75°C;
(iii) 40°C - Drying: 48 h at 40 °C.

The samples dried at 85ºC and at 40ºC were processed immediately after germination while the remaining samples were frozen for 2 d, thawed and subsequently dried at 75ºC as outlined above.

Micro-mashing was performed by mixing 90-g samples of a milled malt with 270 mL of tap water, followed by incubation in 500-mL bottles at 40ºC for 20 min. The temperature was ramped to 65ºC in 25 min, followed by a 60-min-long saccharification pause at 65°C. Thereafter, the temperature was ramped to 78ºC in 13 min in advance of a 10-min-long mashing-off phase at 78ºC. The resulting wort was then cooled on ice, diluted with 700 mL of ice-cold tap water and filtered through a folded MN-616¼ filter (Macherey-Nagel). 400 mL of wort was transferred to a 500-mL bottle, tightly capped and heated for 60 min in a boiling water bath. The bottles were left in the water bath without further heating for an additional time period of 60 min.

### Data on DMSP levels

To examine how various kiln drying temperatures affect the content of DMSP in wild-type and mutant malts of the instant application, grains were first micro-malted and subsequently separated in aliquots before kilning at three different temperatures, 40°C, 75°C and 85°C.

Grains of barley lines containing the wild-type MMT gene were characterized by high levels of DMSP in the final malt. However, reduced levels were measured following kilning at higher temperatures **(Table 2**). In contrast, notably low levels of DMSP were measured in all of the malts of the null-MMT genotypes.

DMSP levels were also measured in the worts produced from the above-mentioned malts, giving DMSP levels in correspondence with those measured in the malts. This finding is in line with previous results of Dickenson and Anderson (1981), describing a tight correlation between the DMSP content of the malt and wort. For all of the null-MMT genotypes, notably low DMSP levels were measured in the corresponding worts, irrespective of the kiln drying temperature with which the malt was made.

### Data on levels of T2N precursors and free T2N

To examine how various kiln drying temperatures affect the levels of free T2N and T2N precursors, concentrations of said compounds were determined in sweet wort and cooled wort produced by micro-maltings and -mashings as described herein above. The results are shown in **Table 3.**

For wort of wild-type malt, the kiln drying temperature had a notable effect on the concentration of T2N and T2N precursors. A high kilning temperature was absolutely necessary to avoid high production of T2N precursors when using wild-type malt.

The kilning temperature had less effect on generation of T2N and its adduct of null-LOX-1-null-MMT malt.

It is also notable that for the worts of Triple-Null malt, the concentrations of free T2N and its precursors were low in all of the samples, irrespective of the kiln drying temperature.

### Example 7

### Micro-mashings with unmalted barley

Micro-mashings of unmalted barley of cv. Power and Triple-Null were achieved by mixing 90-g samples of milled barley with 270 mL of tap water as well as 0.12 g the barley brewing enzyme mixture Ondea Pro (Novozymes), followed by incubation in 500-mL bottles at 54ºC for 30 min. The temperature was ramped to 64ºC in 10 min, followed by a 45-min-long saccharification pause at 64°C. This was followed by a 14-min ramp to 78ºC before a 10-min-long mashing-off phase at said temperature. Subsequent cooling, dilution, filtration and heating procedures were performed as described for the micro-malt mashings (see Example 4).

DMSP levels were measured in worts produced form barley flours of cv. Power and Triple-Null **(Table 4).** It was evident that the DMSP contents in wort produced of Triple-Null barley were remarkably lower than those of wild-type, cv. Power. This was a surprising finding since barley is not considered to contain DMSP (Yang, B. et al.: Factors involved in the formation of two precursors of dimethylsulphide during malting, J. Am. Soc. Brew. Chem. 56:85-92, 1998).

As shown in PCT Patent Application No. PCT/DK2009/050355 (published as WO 2010/075860) (cf. FIG. 12 and Example 9 in said application), the levels of T2N precursors of boiled, barley-brewed and normal worts were markedly low in the samples from double-null-LOX. Accordingly, it is expected that a similar property characterizes worts of Triple-Null barley, making Triple-Null barley a premium raw material for barley-brewed beverages low in, or completely lacking, the T2N and DMS off-flavours.

### Example 8

### Malting and brewing in pilot scale

### Experimental set-up

Malting and brewing analyses with malt of Triple-Null and cv. Quench (reference malt) involved the following steps: (i) malting; (ii) wort preparation; (iii) wort separation; (iv) wort boiling; (v) fermentation of wort with the yeast Saccharomyces carlsbergensis; (vi) lagering of beer; (vii) bright beer filtration; and (viii) bottling of beer (cf. **FIG. 6B,C).**

Malting experiments were carried out with kernels of Triple-Null and cv. Quench in 20-kg-large scales, performed in a malt house as follows:
(i) steeping at 16°C: 1 h wet; 1 h dry; 1 h wet; 1 h dry; 1 h wet; final water content 45%;
(ii) germination for 120 h, starting at 16°C and ramping to 14°C;
(iii) drying for 14 h, starting at 65°C and ramping to 85°C; 3 h at 85°C.

For mashings of both Triple-Null and cv. Quench (the latter used as reference), samples of 25 kg malt were used. Following milling of the individual malt samples, tap water was added to give 146-L volumes. Mashing-in was performed at 40°C for 20 min, followed by a 25 min ramp from 40°C to 65°C. The saccharification pause at 65°C was for 60 min, followed by a 13-min heat-up phase to 78°C, and 10 min of mashing-off at 78°C.

One wort sample of wild-type cv. Quench and one of Triple-Null were separately boiled for 60 min at 101ºC (resulting in 6.7% evaporation), while the remaining two wort samples were heated at 98ºC for 60 min (resulting in 3.9% evaporation). The remaining brewing steps as referred to hereinabove - i.e. filtration, whirlpool separation, fermentation, lagering, and packaging in green glass bottles - were according to specifications for standard brewing practice.

DMSP and DMS levels were measured essentially as described by Hysert et al. (1980), with sulphur-specific detection using static headspace gas chromatography on a 350B sulfur chemiluminescence detector (Sievers). Headspace sampling was performed using a HS-40 automated equipment (Perkin Elmer).

Total levels of DMS, i.e. the sum of free DMS and DMSP, in wort and extracts of green and kilned malt, were obtained by boiling the respective samples under alkaline conditions for 1 h. Boiled and un-boiled samples were then subjected to headspace analysis for determination of DMS levels. The difference between total DMS (measured in the boiled samples) and free DMS (measured in the un-boiled samples) was defined to equal the amount of DMSP present in the samples. The quantity of free DMS in beer was determined essentially as that in wort (Hysert et al., supra).

### Data on levels of DMSP/DMS and T2N precursor/free T2N in wort samples

Using modern brewing equipment, evaporation of 6-10% wort is normally required in order to achieve satisfactory DMS levels in the corresponding, finished beer - i.e. [DMS]<50 ppb, which is the human taste threshold level of the off-flavour. Based on these facts, pilot brewing trials were designed as described above. The aim was to test the effect of reduced energy input, such as no boiling through a pressurized incubation, on DMS levels in the worts and the final beer. Accordingly, the experimental set-up included a comparison with wort boiling according to standard conditions.

High levels of DMSP and free DMS were measured during heating of worts of cv. Quench, with an increase in free DMS levels observed over time in the pressurized wort. In contrast, the boiled, evaporated wort accumulated less DMS until the end of boiling, after which free DMS accumulated again. In parallel with the results following micro-mashings as described hereinabove, worts of pilot-scale Triple-Null malt were characterized by extremely low levels of DMSP and free DMS as compared to similar samples derived from cv. Quench (cf. **Table 5).** It is noteworthy that even in the wort, said DMS levels of Triple-Null malt were well below the 50-ppb taste-threshold level.

The concentrations of T2N precursors and free T2N in wort and beer from wild-type malt cv. Quench and Triple-Null malt were determined by GC-MS following derivatization of carbonyls with O-(2,3,4,5,6-pentafluorobenzyl)-hydroxylamine, essentially as described by Gronqvist et al. (1993).

High concentrations of T2N precursors were measured during the heating of wort of cv. Quench, with a maximum found at the beginning of the boiling/heat treatment **(Table 6).** For wort made from the Triple-Null malt notably lower levels of T2N precursors (approximately 40% of those when using a wild-type raw material) were found - also with a maximum at the start of boiling/heat treatment. A low energy heating regime (i.e. applying a pressurized heat treatment) had only minor effect on the levels of T2N precursors for both malt types. In all the wort samples, low levels of free T2N were determined.

It is notable that the beneficial effect of the null- LOX1 and null-LOX-2 mutations is clearly visible, even when a reduced input of energy is applied during wort heating.

### Data on levels of DMS and T2N precursors/free T2N in beer samples

The concentration of DMS were measured in the fresh beer made from Triple-Null malt as well as from wild-type malt cv. Quench using the two different boiling/heating regimes as described herein above. The results are summarized in **Table 7.**

Beer produced from wild-type malt of cv. Quench using a standard boiling regime contains 65 ppb DMS, i.e. slightly above the 50-ppb taste threshold. Using an energy-saving procedure such as "no boiling through a pressurized incubation" as described herein above with the wild-type malt resulted in a very high concentration of DMS (151 ppb) in the final beer.

In contrast, beer produced from the Triple-Null malt contained very low levels of DMS, regardless of the heating procedure.

Regardless of the wort preparation method applied, beer produced from Triple-Null malt contained much fewer T2N precursors, (amounting to a 56 to 58% reduction) than beer produced from wild-type malt cv. Quench regardless of boiling method **(Table 7).**

The levels of free T2N in fresh beer were low in all four fresh beers tested (i.e. using malt of cv. Quench or Triple-Null combined with evaporation-allowed or pressurized wort preparation), but after forced ageing for 2 weeks at 37°C, a marked difference was seen. While both beers made from wild-type malt produced with standard boiling or pressurized heating contained 0.041 ppb and 0.061 ppb T2N, respectively , the corresponding values for beers produced from Triple-Null malt were lower, i.e. reduced to 95% and 64%, respectively. (Table 7).

### Data on beer foam

Pilot-brewed beer, produced with either evaporation or pressurization at wort heating, of cv. Quench (cf. **FIG. 6B****)** and Triple-Null (cf. **FIG. 6C****)** malts were compared. Beers were taken at sampling point 9 (cf. **FIG. 6B,C),** degassed for 20 min in an ultrasonic bath before 50 mL H₂O was added to 150 mL beer. The mixture was slowly poured into a foam tower, consisting of a 16-cm-long, 7-cm-wide glass tube (with a glass filter and connector at the bottom and top, respectively). N₂ gas, at a flow rate of 400 mL/min, was bubbled through the mixture from the bottom to generate beer foam. This was led through a tube, and collected in a graded sedimentation cone positioned on a weight.

The total foam weight of each of the four beers was recorded at 5-min intervals until foam development ceased **(****FIG. 7****).** Foam levels were similar, irrespective of evaporation or pressurization procedures. However, foam development was notably improved in those beers where Triple-Null malt was used as raw material.

### Beer tastings

An expert taste panel evaluated the four beers produced (i.e. using malt of cv. Quench or Triple-Null combined with evaporation-allowed or pressurized wort preparation), both the freshly produced beer and after forced ageing for 1 and 2 weeks at 37°C **(Table 8).**

The fresh beer produced from Triple-Null malt obtained the highest Total Flavour Score, regardless of boiling method. In contrast, a slightly lower Total Flavour Score was obtained for the beer produced from wild-type malt and using the standard boiling regime; it was deemed "slightly DMS". Application of the pressurized heating technique resulted in a beer with a very low Total Flavour Score, and deemed "Strongly DMS"

After forced ageing for 1 or 2 weeks at 37°C the beers produced from Triple-Null malt got markedly lower total ageing scores than beers produced from wild-type malt, especially due to a reduced score for the "Papery" attribute, originating from lower concentrations of the ageing component T2N.

### Example 9

### Malting and brewing in pilot scale

### Experimental set-up

Malting and brewing analyses with malt of Triple-Null (double-null-LOX-null-MMT), and cv. Rosalina (wild-type reference), involved the following steps: (i) malting; (ii) wort preparation; (iii) wort separation; (iv) wort boiling; (v) fermentation of wort with the yeast Saccharomyces carlsbergensis; (vi) lagering of beer; (vii) bright beer filtration; and (viii) bottling of beer.

Malting experiments were carried out with kernels of Triple-Null and cv. Rosalina in 21-kg-large scales, performed in a malt house as follows:
(i) steeping at 16°C: 1 h wet; 1 h dry; 1 h wet; 1 h dry; 1 h wet; dripping of water over 36 h to a final water content 45%;
(ii) germination for 120 h, starting at 16°C and ramping to 14°C;
(iii) drying/kilning of the germinated kernels were with either a normal temperature programme or a low temperature programme in a pilot kiln;
(iv) normal drying/kilning programme, starting at 45°C with ramp-up to 85°C over 14 h, followed by incubation for 2 h at 85°C;
(v) low temperature drying/kilning programme, beginning at 45°C and with ramp-up to 75°C over 12 h, followed by incubation for 2 h at 75°C.

For mashings of both Triple-Null and cv. Rosalina, samples of 34 kg malt were used. Following milling of the individual malt samples, tap water was added to give 180-L volumes. Mashing-in was performed at 60°C for 20 min, followed by a 5-min ramp from 60°C to 65°C. The saccharification pause at 65°C was for 60 min, followed by a 13-min heat-up phase to 78°C, and 10 min of mashing-off at 78°C.

Wort sample of wild-type cv. Rosalina and of Triple-Null were separately boiled either for 60 min at 100ºC in an open vessel (resulting in 4.5% evaporation), or heated to 99.5°C and kept at 99.5°C for 60 min in a closed vessel (resulting in 0% evaporation).

Six different worts were made with different combinationons of cultivar, kilning conditions and boiling conditions (see **Table 9).**

The remaining brewing steps as referred to hereinabove - i.e. whirlpool separation, fermentation, lagering, filtration and packaging in green glass bottles - were according to specifications for standard brewing practice.

DMSP and DMS levels were measured as described herein above in Example 8.

### Data on levels of DMSP/DMS in malt samples

In a modern malting plant, drying of kernels with a curing temperature at 85°C for at least 2 h is normally required in order to reduce DMSP in malt to a low level - i.e. less than 4.5 mg/kg malt - in order to obtain a malt from which a wort can be obtained with satisfactory DMS level (see below).

Based on these facts, pilot malting trials were designed as described above. The aim was to test the effect of reduced energy input, such as low temperature drying, on DMS and DMSP levels in the malts, worts and the final beer. Accordingly, the experimental set-up included a comparison with kernel drying according to standard conditions.

In the malt made with cv. Rosalina using standard drying at 85°C, DMSP was measured to 4.7 mg/kg malt. In the malt of cv. Rosalina made with drying at 75°C DMSP was measured to 16.2 mg/kg **(Table 10).** In malt made with .grains of Triple-Null, extremely low DMSP levels, actually below the detection limit, were obtained regardless of the drying temperature.

### Data on levels of DMSP/DMS in wort samples

Using modern brewing technology, at least one hour boiling with an evaporation of 4.5-10% wort is normally required in order to achieve satisfactory DMS levels in the corresponding, finished beer - preferably [DMS]<50 ppb. Based on these facts brewing trials were designed as described above. The aim was to test the effect of reduced energy input, such as low temperature kernel drying or heat treatment without evaporation in a closed vessel, on DMS levels in the worts and the final beer. Accordingly, the experimental set-up included a comparison with kernel drying and wort boiling according to standard conditions.

High levels of DMSP and free DMS were measured during heating of worts of cv. Rosalina made with normal drying. An increase in free DMS levels was observed over time in the closed vessel. In contrast, the boiled, evaporated wort accumulated less DMS until the end of boiling, after which free DMS accumulated again. **(Table 11)** Using a low temperature drying with cv. Rosalina the DMSP and DMS levels were much higher than with normal drying. In all three worts made with cv. Rosalina the final wort had a DMS level higher than 50-ppb.

In parallel with other results described herein above, wort of Triple-Null malt were characterized by extremely low levels of DMSP and free DMS as compared to similar samples derived from cv. Rosalina. It is noteworthy that even in the wort, said DMS levels of Triple-Null malt were well below 50-ppb regardless of kilning and boiling regimes.

### Data on levels of T2N precursor/free T2N in wort samples

The concentrations of T2N precursors and free T2N in wort and beer from wild-type malt cv. Rosalina and Triple-Null malt were determined by GC-MS following derivatization of carbonyls with O-(2,3,4,5,6-pentafluorobenzyl)-hydroxylamine, essentially as described by Groenqvist et al. (1993).

High concentrations of T2N precursors were measured in wort of cv. Rosalina (Table 12). For wort made from the Triple-Null malt, particularly lower levels of T2N precursors, ∼40% of those when using a wild-type raw material, were found. Low energy heating regime, i.e. low kilning temperature or heat treatment of wort without evaporation in a closed vessel, had only little effect on the levels of T2N precursors for both malt types. In all of the wort samples, low levels of free T2N were determined.

It is notable that the beneficial effect of the null-LOX-1 and null-LOX-2 mutations is clearly visible, even when a reduced input of energy is applied.

### Data on levels of DMS and T2N precursors/free T2N in beer samples

DMS concentrations were measured in the fresh beer made from Triple-Null malt, as well as from wild-type malt of cv. Rosalina using the two different kernel drying and boiling/heating regimes as described herein above. The results are summarized in **Table 13.**

Beer produced using a standard boiling regime from wild-type malt of cv. Rosalina, produced using standard drying condition, contained 117 ppb DMS, i.e. above 50-ppb. Using an energy saving procedure, such as "no evaporation in a closed vessel" as described hereinabove, with the wild-type malt resulted in 174 ppb DMS, i.e. a very high concentration of the off-flavour in the final beer,

In contrast, beer produced from the Triple-Null malt contained very low levels of DMS, regardless of the boiling and kilning procedures.

The levels of free T2N in fresh beer were low in all beers tested (i.e. using malt of cv. Rosalina or Triple-Null combined with evaporation-allowed boiling or heat treatment without evaporation in a closed vessel), but a marked difference was seen after forced ageing for 2 weeks at 37°C. While beer made from wild-type malt produced with standard boiling and heat treatment without evaporation in a closed vessel contained 0.055 ppb and 0.035 ppb T2N, respectively, the corresponding values for beers produced from Triple-Null malt were markedly lower, i.e. reduced by 67% and 51%, respectively (cf. **Table 13).**

### Beer tastings

An expert taste panel evaluated the 6 beers produced (i.e. using malt of cv. Rosalina or Triple-Null combined with evaporation-allowed boiling or heat treatment without evaporation in a closed vessel or standard or low temperature drying), both the freshly produced beer and after forced-ageing for 2 weeks at 37°C **(Table 14).**

The fresh beer produced from Triple-Null malt obtained the highest total flavour score, regardless of kilning and boiling methods. In contrast, much lower total flavour scores were obtained for the beer produced from wild-type malt kilned at 85°C and using the standard boiling regime; it was deemed "markedly DMS". Application of heat treatment without evaporation in a closed vessel in wort preparation resulted in a beer with an even lower total flavour score, which was deemed "strongly DMS".

After forced-ageing for 2 weeks at 37°C, the beer produced with normal boiling from Triple-Null malt kilned at 85°C got markedly lower total ageing scores than the corresponding beer produced from wild-type malt, especially due to a reduced score for the "papery" attribute, originating from lower concentrations of the ageing component T2N.

For beer produced from wild-type malt, evaluation of the staling characteristic was not really possible, primarily due to the markedly-to-strong DMS off-flavour.

### Example 10

### Comparisons of barley-brewed and normal beer - THAs.

Beer-specific THAs derived from linoleic acid were already described several decades back in time (Drost et al., 1974). Since then, various reports have verified that the total content of THAs in beer ranges from ∼5-12 ppm (Hamberg, 1991; and references therein). While 9,12,13-THA normally constitutes 75-85% of the THAs in beer, that of 9,10,13-THA amounts to 15- 25%; other isomers are found in trace amounts.

In beer produced from wort prepared from malt of barley Triple-Null (cf. Example 9), the concentration of 9,12,13-THA was reduced by 80-87% compared to the control beer made from malt of cv. Rosalina **(Table 15).** For the 9,10,13-THA isomer 65-72% reduction was observed. These measurements were carried out using standard HPLC-mass spectrometry analyses (Hamberg, supra).

### Example 11

### Amplification of raw material-derived DNA in beer

Where the PCR methodology can be used to establish the presence or absence of species-specific DNA sequences in malt flour mixes, authentication of beer samples have been more challenging. In beer production, the combined effects of raw material-derived nucleases, elevated temperatures and filtrations provide beer products that contain only low levels of intact DNA for amplification of plant gene fragments. Here, an effective novel method for combined DNA extraction and amplification is described that generates DNA in sufficient quantity for visualization.

A novel, four-step protocol was developed for raw material authentication of beer samples. It utilized in a consecutive manner the reagents and protocols of three biomolecular kits, eventually causing amplification of DNA fragments for visualization:
(i) extraction of DNA from beer through binding to magnetic beads [following the recommendations in the instructions for liquid samples of the DNA Extraction Kit, Speciation (Tepnel Biosystems Ltd., Cat. No. 901040N)];
(ii) amplification of DNA by isothermal strand displacement (Illustra GenomiPhi V2 DNA Amplification Kit, GE Healthcare, Cat. No. 25-6600-30);
(iii) PCR with primers for detection of specific DNA mutations, using the REDExtract-N-Amp PCR ReadyMix (Sigma, Cat. No. XNAP-1KT);
(iv) agarose gel electrophoresis to separate amplification products, followed by staining with ethidium bromide to visualize these.

Step (i) above concerned purification of DNA from 400-µL aliquots of the following samples:
(a) Tuborg Grøn Pilsner, produced using flour of a 75%:25% mixture of wild-type malt:barley (Carlsberg Breweries A/S; label inscription 26.11.11 704);
(b) beer produced in pilot-scale using a mixture comprising 50% null-LOX-1 malt (Carlsberg trial identifier 2C10084);
(c) non-boiled wort of a pilot-scale brew using malt produced from Triple-Null barley (Carlsberg trial identifier 2C11001);
(d) boiled wort of a pilot-scale brew using Triple-Null malt (Carlsberg trial identifier 2C11001), i.e. wort processed from that described in (c) above.

DNA purified from each of the above-mentioned samples (a), (b), (c) and (d) was finally resuspended in 50 µL of a 10-mM Tris-EDTA buffer, pH 7.4.

In step (ii) above, 1-µL sample aliquots of wort or beer-derived DNA - purified as detailed in (i) - was subjected to amplification. DNA was briefly heat-denatured and then cooled in sample buffer containing random hexamers that non-specifically bind to the DNA. A master-mix containing DNA polymerase, random hexamers, nucleotides, salts and buffers was added and isothermal amplification carried out at 30°C for 2.0 h, employing a final reaction volume of 20 µL. Subsequently, the polymerase was heat inactivated during a 10-min incubation at 65°C.

Regarding step (iii) above, parameters of 14-µL PCR amplifications - including either 1.0 or 4.2-µL template aliquots of samples prepared as described in (ii) above, and 7 pmol each of primer FL820 (SEQ ID NO: 10 of WO 2010/075860) and FL823 (SEQ ID NO:11 of WO 2010/075860) to detect the null-LOX-1 mutation; cf. FIG. 18B in WO 2010/075860 to Skadhauge, B. et al. - were as follows:
(i) 1 cyclus with denaturation at 96°C for 2 min;
(ii) 30 cycles with:
   (a) denaturation at 95°C for 1 min;
   (b) annealing at 68°C for 1 min;
   (c) extension at 72°C for 1 min
(iii) final extension at 72°C for 10 min
(iv) hold

Step (iv) above consisted of an electrophoretic separation of the entire PCR mixture in a 2% (w/v) agarose gel wherein ethidium bromide was incorporated. Following electrophoresis, the gel was irradiated with UV light and a photo taken (FIG. 10).

Analysis of the DNA banding pattern shown in FIG. 10 revealed no amplification product from an aliquot of Tuborg beer, an expected result as the raw material was of wild-type origin, i.e. not derived from null-LOX-1 barley. However, amplification products of the expected length could be detected samples from beer brewed on a mixture comprising 50% null-LOX-1 malt. Similarly, but more pronounced, staining was obtained in the non-boiled wort prepared from Triple-Null malt. Possibly due to heat damage and following precipitation, less PCR product was amplified in the sample of boiled wort of Triple-Null raw material.

Utilizing the amplified products described above in (ii) with primer pair FL1034-FL1039 (SEQ ID NO:9 and SEQ ID NO:8 of WO 2010/075860, respectively; see also FIG. 18 in WO 2010/075860 to Skadhauge et al.), primer set 20 (SEQ ID NO:77 and SEQ ID NO:81 of WO 2010/063288), and primer set 21 (SEQ ID NO:78 and SEQ ID NO:82 of WO 2010/063288; see also FIG. 15 in WO 2010/063288 A2 to Knudsen et al.) in separate PCRs, but at conditions similar to those detailed above in the instant Example, it is verified whether a wort or beer sample is produced by the use of a Triple-Null raw material.

### Example 4 of PCT/DK2009/050355 (published as WO2010/075860)

### HPLC-based analysis of HPODEs in germinated and micro-malted barley

Analysis for barley HPODEs was carried out essentially as described in International Patent Application WO 2005/087934. except utilizing germinating instead of mature embryos. Grains of generation M4 were germinated for 48 h as described in Example 2 of PCT/DK2009/050355 (published as WO2010/075860). Grains of generation M5 were subjected to a micro-malting procedure performed essentially as described in Example 6 of PCT/DK2009/050355 (published as WO20101075860), but the specific HPODE levels were determined after 72 h of germination, and the kilning procedure was omitted. Levels of 9- and 13-HPODEs were determined by letting crude protein extracts from germinating embryos of generations M4 and M5 of mutant A689 and control samples incubate with the substrate linoleic acid. Reaction products were analyzed by HPLC.

The germinated embryos were dissected from the barley grains using a scalpel to cut between the scutellum and the endosperm. Each 4-embryo-large sample was then placed between two pieces of weighing paper, and hammered gently to produce a homogenous flour. This was transferred to a 1.5-ml microcentrifuge tube; 600 µl of a 200-mM lactic acid buffer, pH 4.5, was added, and the tube was placed on ice for 10 min before further homogenization using a plastic pestle (Kontes). Subsequently, 600 µl H₂O was added to each tube, and the samples were centrifuged for 2 min at 20.000×g. A 100-µl aliquot of the resulting supernatant was transferred to a 15-ml centrifuge tube (Cellstar: cat. no.188271), to prepare for analysis of the reaction products following LOX action. 2 ml of a 100-mM Na-phosphate buffer, pH 6.5, containing 260 µM linoleic acid [this substrate was prepared by mixing 10 ml of a 100-mM Na-phosahate buffer, pH 6.5, with 100 µl of a 24-mM linoleic acid stock solution. The latter was made by first mixing 155 µl of linoleic acid (corresponding to 134 mg free acid; L-1376, Sigma) and 257 µl Tween-20, then adding H₂O to a volume of 5 ml, followed by addition of 600 µl 1 M NaOH. and when the solution turned clear. adjusting the volume to 20 ml with H₂O. After a 15-min incubation at ∼30 rpm on a blood tube rotator, 2 ml ethyl acetate was added, and the sample content mixed by rigorous shaking for 5 sec in order to extract 9- and 13-HPODEs. The sample was then centrifuged for 10 min at 800×g, and 1 ml ethyl acetate was transferred to a 1.5-ml microcentrifuge tube, in which ethyl acetate was evaporated under a stream of nitrogen gas. Subsequently, the HPODEs were resuspended in 300 µl methanol, and filtered through a 0.45-µm membrane (Millex-HN filter. Millipore). Analysis of the HPODE content was performed by HPLC. A total of 15 µl from each sample was injected into a HPLC apparatus (HP 1100 Series, Hewlett Packard), equipped with a 4.6×250 mm Symmetry C18 column (Waters). The mobile phase used was a 16:12:12:10:0.5 (v:v:v:v:v) mixture of water:methanol:acetonitrile:tetrahydrofuran:trifluoroacetic acid. The flow of the mobile phase was 1 ml min⁻¹, and the pressure measured in front of the column was 140 bars. The separation was performed at 30°C. Detection of linoleic acid hydroperoxides with conjugated double bonds was performed at 234 nm.

### Example 4 of PCT/DK2009/050315 (published as WO2010/063288)

### MMT activity measurements of Mutant 8063

The MMT enzyme catalyzes the transfer of a methyl group from SAM to Met, forming SMM (cf. FIG. 1B of PCT/DK2009/050315 (published as WO2010/063288)). Using [³H]SAM as a substrate, in which the methyl group is labeled with tritium, transfer of said methyl group can be monitored by scintillation counting after removal of the remaining [³H]SAM by activated charcoal. The latter compound binds the substrate, but not the newly synthesized, labeled product SMM (Pimenta et al., 1995). This allowed for determination of MMT activities and SMM contents, the latter determined as described in Example 2 of PCT/DK2009/050315 (published as WO2010/063288), in extracts of 15 shoots of each of Mutant 8063 and cv. Prestige (FIG. **4** of PCT/DK2009/050315 (published as WO2010/063288)). Data examination verified that MMT catalyzed the formation of SMM in wild-type kernels, a property that was absent in grains of Mutant 8063.

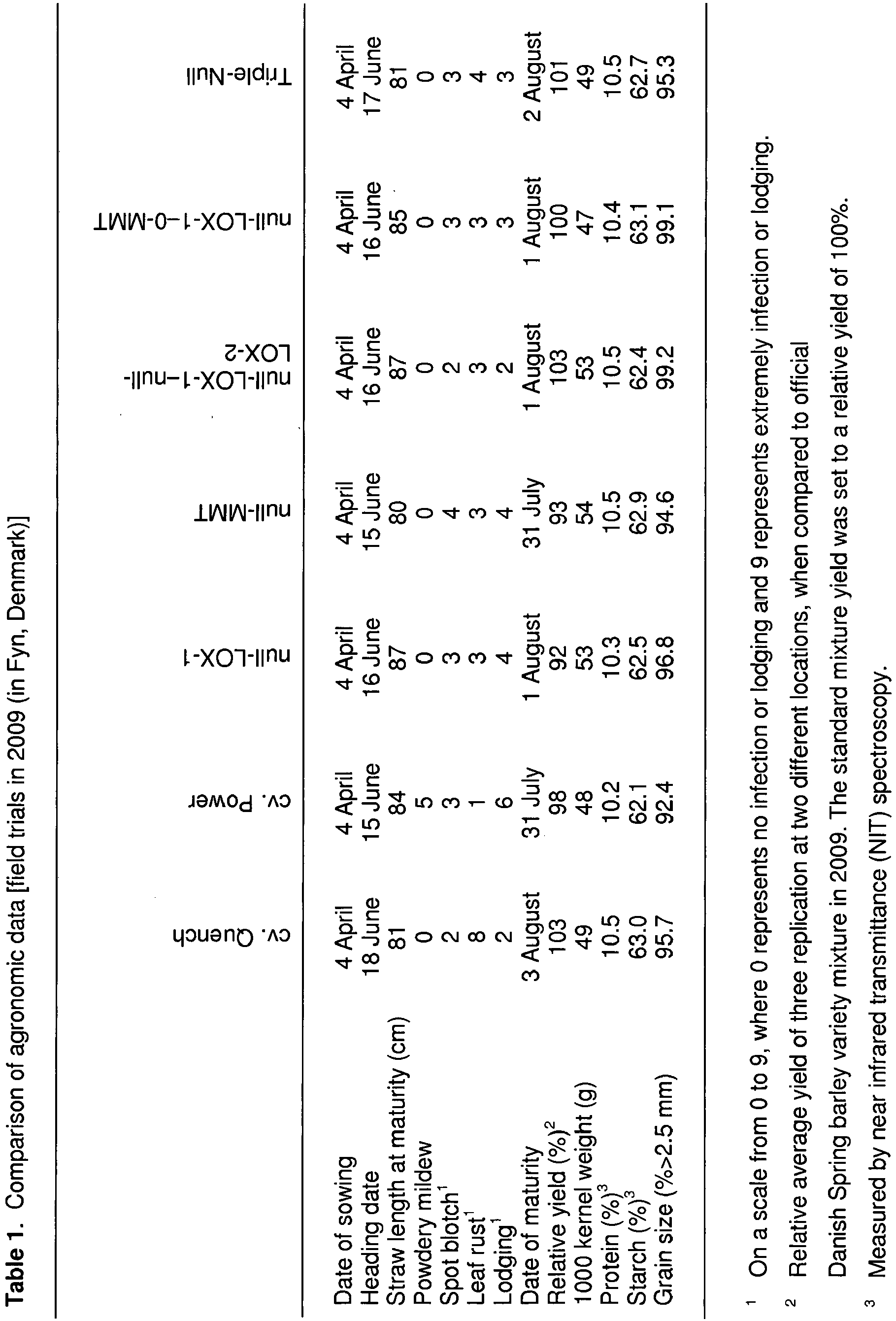

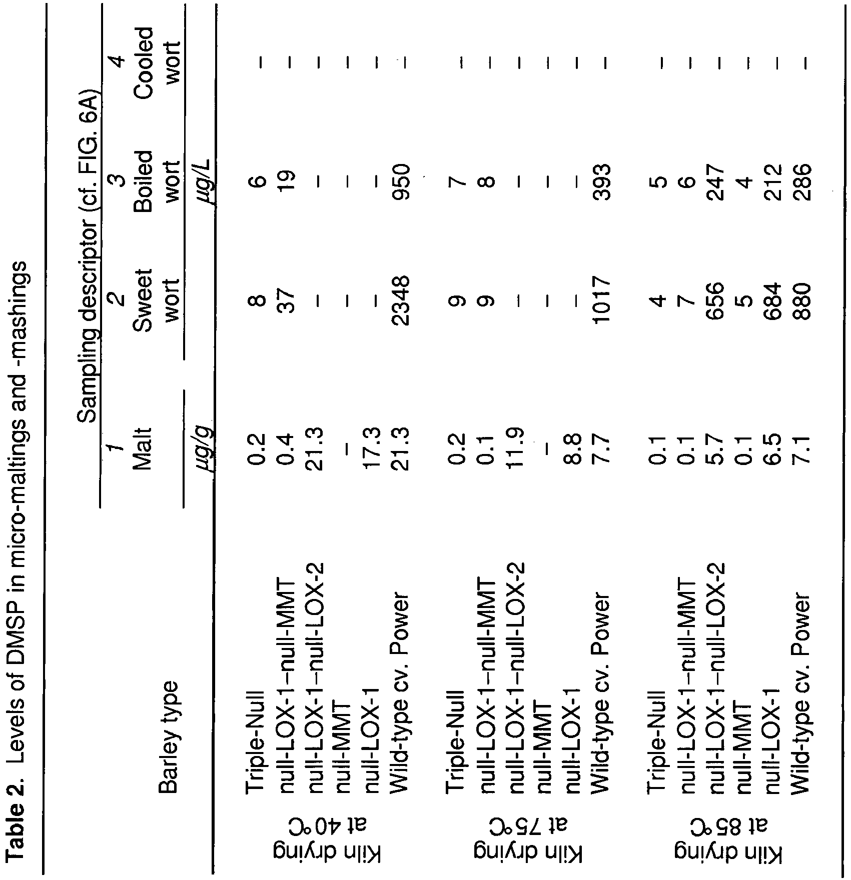

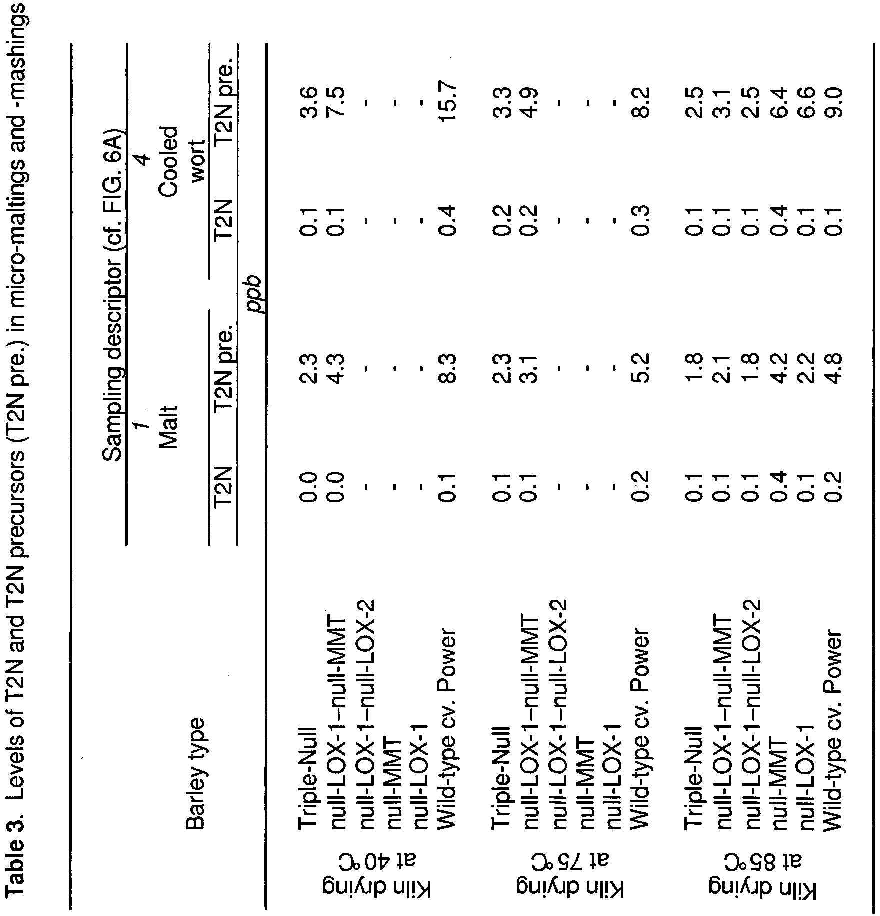

**Table 4. Levels of DMSP in micro-mashings**

| | Sampling descriptor | | | |
|---|---|---|---|---|
| Barley type | *2* Sweet wort | *3* 30-min boiled wort | *3* 60-min boiled Wort | *4* Cooled wort |
| | *µg*/*L* | | | |
| Wild-type cv. Power | 10.9 | 12.0 | 6.3 | 3.8 |
| Triple-Null | 0.1 | 0.6 | 0.6 | 0.3 |

**Table 5. Levels of DMSP and DMS in two types of pilot-scale mashings**

| Barley type | | Sampling descriptor (cf. FIG. 6B,C) | | | | | | | |
|---|---|---|---|---|---|---|---|---|---|
| | | *1* | *2* | *3* | *4* | *5* | *6* | *7* | *8* |
| | | Malt | Evaporation-allowed heating of sweet wort (min; °C) | | | | | | |
| | | | 0; 67 | 22; 99 | 37; 101 | 52; 101 | 67; 101 | 82; 101 | 142; 90 |
| | | *µg*/*g* | | | | *µg*/*L* | | | |
| Wild-type cv. Quench | DMSP | 4.7 | 725 | 682 | 516 | 369 | 288 | 219 | 101 |
| | DMS | 4.3 | 93 | 85 | 50 | 47 | 33 | 31 | 127 |
| Triple-Null | DMSP | 0.1 | 3 | 2 | 3 | 2 | 1 | 1 | 1 |
| | DMS | 0.1 | 14 | 13 | 11 | 12 | 11 | 12 | 12 |
| | | | | | | | | | |
| | | | Pressurized heating of sweet wort (min; °C) | | | | | | |
| | | | 0; 69 | 22; 99 | 37; 98 | 52; 98 | 67; 98 | 82; 98 | 142;80 |
| Wild-type cv. Quench | DMSP | 4.7 | 564 | 508 | 408 | 340 | 273 | 219 | 161 |
| | DMS | 4.8 | 51 | 51 | 60 | 64 | 76 | 80 | 164 |
| Triple-Null | DMSP | 0.0 | 15 | 5 | 3 | 3 | 3 | 2 | 2 |
| | DMS | 0.1 | 20 | 20 | 19 | 18 | 19 | 20 | 20 |

**Table 6. Levels of T2N precursor (T2N pre.) and free T2N in two types of pilot-scale mashings**

| Barley type | | Sampling descriptor (cf. FIG. 6B,C) | | | | | | | |
|---|---|---|---|---|---|---|---|---|---|
| | | *1* | *2* | *3* | *4* | *5* | *6* | *7* | *8* |
| | | Malt | Evaporation-allowed heating of sweet wort (min; °C) | | | | | | |
| | | | 0; 67 | 22; 99 | 37; 101 | 52; 101 | 67; 101 | 82; 101 | 142; 90 |
| | | | | | | *ppb* | | | |
| Wild-type cv. Quench | T2N pre. | - | 3.6 | 4.8 | 4.3 | 4.2 | 3.9 | 3.9 | 3.7 |
| | T2N | - | 0.3 | 0.3 | 0.2 | 0.2 | 0.1 | 0.1 | 0.1 |
| Triple-Null | T2N pre. | - | 1.7 | 1.9 | 1.6 | 1.4 | 1.4 | 1.2 | 1.4 |
| | T2N | - | 0.1 | 0.2 | 0.1 | 0.1 | 0.1 | 0.1 | 0.1 |
| | | | | | | | | | |
| | | | Pressurized heating of sweet wort (min; °C) | | | | | | |
| | | | 0; 69 | 22; 99 | 37; 98 | 52; 98 | 67; 98 | 82; 98 | 142; 80 |
| Wild-type cv. Quench | T2N pre. | - | 3.8 | 4.0 | 3.9 | 3.8 | 3.8 | 3.8 | 3.3 |
| | T2N | - | 0.2 | 0.2 | 0.2 | 0.2 | 0.2 | 0.2 | 0.2 |
| Triple-Null | T2N pre. | - | 1.6 | 1.8 | 1.8 | 1.6 | 1.5 | 1.4 | 1.5 |
| | T2N | - | 0.2 | 0.2 | 0.2 | 0.1 | 0.1 | 0.1 | 0.1 |

**Table 7. DMS, T2N precursors and free T2N in fresh and aged beer**

| | | Beer incubation conditions | | | | |
|---|---|---|---|---|---|---|
| | | | | Fresh | | 2 weeks at 37°C |
| Barley type | Boiling type | DMS | T2N precursors | Free T2N | SO₂ | Free T2N |
| | | (ppb) | (ppb) | (ppb) | (ppm) | (ppb) |
| Wild-type cv. Quench | Evaporation-allowed heating of sweet wort | 65 | 3.0 | 0.025 | 5 | 0.041 |
| Triple-Null | | 2 | 1.3 | 0.018 | 3 | 0.039 |
| Wild-type cv. Quench | Pressurized heating of sweet wort | 151 | 3.3 | 0.024 | 6 | 0.061 |
| Triple-Null | | 3 | 1.4 | 0.020 | 4 | 0.039 |

**Table 8. Sensory evaluation of fresh and aged beer produced from wild-type cv Quench and Triple-Null malt**

| | | Beer incubation conditons | | | | | |
|---|---|---|---|---|---|---|---|
| | | Fresh | | 1 week at 37 °C | | 2 weeks at 37°C | |
| Barley type | Boiling type | Flavour score¹ | Comments | Ageing score | Papery score | Ageing score | Papery score |
| Wild-type cv. Quench | Evaporation-allowed | 4.4 | Noticeably sour | 3.1 | 2.4 | 2.4 | 2.1 |
| | heating of sweet wort | | Slightly oxidised | | | | |
| | | | **Slightly DMS** | | | | |
| | | | Slightly sulphidic | | | | |
| | | | Slightly estery | | | | |
| Triple-Null | | 4.8 | Slightly sulphidic | 2.1 | 1.6 | 1.9 | 1.3 |
| | | | Slightly fatty acid | | | | |
| | | | Slightly grainy | | | | |
| Wild-type cv. Quench | Pressurized heating | 2.8 | Slightly estery | 3.5 | 2.3 | 3.3 | 2.4 |
| | of sweet wort | | Slightly aged | | | | |
| | | | **Strongly DMS** | | | | |
| | | | Slightly catty | | | | |
| | | | Slightly grainy | | | | |
| Triple-Null | | 4.8 | Slightly grainy | 2.6 | 1.9 | 1.9 | 1.1 |
| | | | Slightly fruity | | | | |
| | | | Slightly sour | | | | |
| | | | Slightly stale | | | | |

| | | | | | | | |
|---|---|---|---|---|---|---|---|
| ¹ On a scale from 1-9, where 9 is best ² On a scale from 0-5, where 0 designates a fresh beer, and 5 a very aged beer ³ On a scale from 0-5, where 5 designates an extremely papery beer | | | | | | | |

**Table 9. Experimental overviev**

| | | Drying/kilning | | Boiling | | |
|---|---|---|---|---|---|---|
| Brew no. | Cultivar | Progamme | Curring temperature (°C) | | Vessel | Evaporation (%) |
| 1 | Rosalina | Normal | 85 | | Open | 4.5 |
| 2 | Triple -Null | Normal | 85 | | Open | 4.5 |
| 3 | Rosalina | Normal | 85 | | Closed | 0 |
| 4 | Triple -Null | Normal | 85 | | Closed | 0 |
| 5 | Rosalina | Low temperature | 75 | | Open | 4.5 |
| 6 | Triple -Null | Low temperature | 75 | | Open | 4.5 |

**Table 10. DMS and DMSP in malt samples**

| | Drying/kilning | | DMS | DMSP |
|---|---|---|---|---|
| Cultivar | Progamme | Curring temperature | | |
| | | (°C) | (mg/kg) | (mg/kg) |
| Rosalina | Normal | 85 | 4.1 | 4.7 |
| Rosalina | Low temperature | 75 | 2.8 | 16.2 |
| Triple -Null | Normal | 85 | <DL* | <DL* |
| Triple -Null | Low temperature | 75 | <DL* | <DL* |

| | | | | |
|---|---|---|---|---|
| * below detection limit | | | | |

**Table 11. Levels of DMSP and DMS in two types of pilot-scale boiling**

| Normal kernel drying at 85°C and evaporation-allowed heating of wort in open vessel. | | | | | | | |
|---|---|---|---|---|---|---|---|
| Barley type | | Sample descriptor (cf. FIG. 6) | | | | | |
| | | *1* | *2* | *3* | *4* | *5* | *7* |
| | | Heating of sweet wort (min; °C) | | | | | |
| | | 0; 67 | 22; 99 | 37; 100 | 52; 100 | 82; 100 | 142;90 |
| | | *µg*/*L* | | | | | |
| Wild-type | DMSP | 1245 | 1257 | 882 | 759 | 415 | 208 |
| cv. Rosalina | DMS | 212 | 190 | 102 | 56 | 31 | 178 |
| Triple-Null | DMSP | trace | - | - | - | - | trace |
| | DMS | trace | - | - | - | - | trace |

| Normal kernel drying at 85°C and heating of wort in closed vessel without evaporation. | | | | | | | |
|---|---|---|---|---|---|---|---|
| Barley type | | Sample descriptor (cf. FIG. 6) | | | | | |
| | | 1 | *2* | *3* | *4* | *5* | *7* |
| | | Heating of sweet wort (min; °C) | | | | | |
| | | 0; 67 | 22; 99 | 37; 100 | 52; 100 | 82; 100 | 142; 90 |
| | | | | | *µg*/*L* | | |
| Wild-type | | | | | | | |
| cv. Rosalina | DMSP | 1180 | 1061 | 732 | 576 | 398 | 274 |
| | DMS | 183 | 227 | 410 | 412 | 471 | 709 |
| Triple-Null | DMSP | trace | - | - | - | - | trace |
| | DMS | trace | - | - | - | - | trace |

| Low-temperature drying of kernels at 75°C and evaporation-allowed heating of wort in open vessel. | | | | | | | |
|---|---|---|---|---|---|---|---|
| Barley type | | Sample descriptor (cf. FIG. 6) | | | | | |
| | | *1* | *2* | *3* | *4* | *5* | *7* |
| | | Heating of sweet wort (min; °C) | | | | | |
| | | 0; 67 | 22; 99 | 37; 100 | 52; 100 | 82; 100 | 142;90 |
| | | | | | *µg*/*L* | | |
| Wild-type | | | | | | | |
| cv. Rosalina | DMSP | 1825 | 1665 | 1262 | 1005 | 611 | 376 |
| | DMS | 155 | 178 | 74 | 32 | 27 | 154 |
| Triple-Null | DMSP | 11 | - | - | - | - | 2 |
| | DMS | 4 | - | - | - | - | 4 |

**Table 12. Levels of T2N precursors (T2N pre) and free T2N in pilot scale worts produced using varying kilning and boiling regimes**

| Barley type | Boiling type *) | At start of heating | | Heated wort | |
|---|---|---|---|---|---|
| | | T2N (ppb) | T2N pre (ppb) | T2N (ppb) | T2N pre (ppb) |
| *Normal drying at 85 °C* | | | | | |
| Wild-type cv. Rosalina | Ves.op.; evap. | 0.39 | 5.3 | 0.21 | 4.2 |
| Triple-Null | Ves.op.; evap. | 0.24 | 1.8 | 0.08 | 1.8 |
| Wild-type cv. Rosalina | Ves.clo.; no evap. | 0.40 | 5.3 | 0.36 | 4.1 |
| Triple-Null | Ves.clo.; no evap. | 0.28 | 2.2 | 0.11 | 1.8 |
| *Low-temperature drying at 75°C* | | | | | |
| Wild-type cv. Rosalina | Ves.op.; evap. | 0.21 | 4.4 | 0.27 | 4.7 |
| Triple-Null | Ves.op.; evap. | 0.31 | 1.9 | 0.18 | 1.8 |

| | | | | | |
|---|---|---|---|---|---|
| *) Abbreviations: Vessel open (ves. op) or closed (ves. clo.); no evaporation (no. evap.) or evaporation-allowed (evap.) | | | | | |

**Table 13. DMS and free T2N in fresh and aged beer**

| Barley type | Boiling type *) | DMS (ppb) | Free T2N **) (ppb) | SO₂ (ppm) | Free T2N ***) (ppb) |
|---|---|---|---|---|---|
| *Normal drying at 85°C* | | | | | |
| Wild-type cv. Rosalina | Ves.op.; evap. | 117 | 0.011 | 5 | 0.055 |
| Triple-Null | Ves.op.; evap. | 1 | 0.011 | 5 | 0.018 |
| Wild-type cv. Rosalina | Ves.clo.; no evap. | 326 | 0.011 | 5 | 0.035 |
| Triple-Null | Ves.clo.; no evap. | 2 | 0.012 | 4 | 0.017 |
| *Low temperature drying at 75°C* | | | | | |
| Wild-type cv. Rosalina | Ves.op.; evap. | 174 | - | 10 | - |
| Triple-Null | Ves.op.; evap | 5 | - | 5 | - |

| | | | | | |
|---|---|---|---|---|---|
| *) Abbreviations: Vessel open (ves. op) or closed (ves. clo.); no evaporation (no. evap.) or evaporation-allowed (evap.) **) In fresh beer ***) In beer incubated for 2 weeks at 37°C | | | | | |

**Table 14 Sensory evaluation of fresh and aged beer produced from wild-type cv. Rosalina and Triple-Null malt**

| | | Beer incubation conditons | | | | |
|---|---|---|---|---|---|---|
| Barley type | Boiling type *) | Fresh | | 2 weeks at 37°C | | |
| | | Flavour score¹ | Comments | | Ageing score² | Papery score³ |
| *Normal drying of kernels at 85 °C* | | | | | | |
| Wild-type cv. Rosalina | Ves.op.; evap. | 3.4 | Slightly sulphidic | | | |
| | | | **Markedly DMS** | | | |
| Triple-Null | Ves.op.; evap. | 5.7 | Slightly after-bitter | | 2.4 | 1 |
| Wild-type cv. Rosalina | Ves.clo.; no evap. | 3.1 | **Strongly DMS** | | | |
| Triple-Null | Ves.clo.; no evap. | 5.0 | Slightly sour | | 2.7 | 2.0 |
| | | | Slightly hoppy | | | |
| | | | Slightly sharp-bitter | | | |
| | | | Slightly grainy | | | |
| *Low-temperature drying of kernels at 75°C* | | | | | | |
| Wild-type cv. Rosalina | Ves.op.; evap. | 4.8 | Slightly fruity **Markedly DMS** | | | |
| Triple-Null | Ves.op.; evap. | 5.2 | Slightly bitter | | 2.1 | 0.7 |
| | | | Slightly acidic | | | |

| | | | | | | |
|---|---|---|---|---|---|---|
| *) Abbreviations: Vessel open (ves.op) or closed (ves.clo.); no evaporation (no. evap.) or evaporation-allowed (evap.) ¹ On a scale from 1-9, where 9 is best ² On a scale from 0-5, where 0 designates a fresh beer, and 5 a very aged beer ³ On a scale from 0-5, where 5 designates an extremely papery beer | | | | | | |

**Table 15. THAs in beers produced from malt of cv Rosalina and Triple-Null mutant.**

| Barley type | Boiling type *) | 9.12.13-Hodea (mg/l) | 9.10.13-Hodea (mg/l) | 9.12.13-Hodea/ 9.10.13-Hodea |
|---|---|---|---|---|
| *Normal drying at 85 °C* | | | | |
| Wild-type cv. Rosalina | Ves.op.; evap. | 3.68 | 0.84 | 4.4 |
| Triple-Null | Ves.op.; evap. | 0.75 | 0.29 | 2.5 |
| Wild-type cv. Rosalina | Ves.clo.; no evap. | 3.28 | 0.78 | 4.2 |
| Triple-Null | Ves.clo.; no evap. | 0.51 | 0.23 | 2.2 |
| *Low- temperature drying at 75°C* | | | | |
| Wild-type cv. Rosalina | Ves.op.; evap. | 3.77 | 0.8 | 4.7 |
| Triple-Null | | 0.49 | 0.22 | 2.2 |

| | | | | |
|---|---|---|---|---|
| *) Abbreviations: Vessel open (ves.op) or closed (ves.clo.); no evaporation (no. evap.) or evaporation-allowed (evap.) | | | | |

**Table 16 Sequence Listing**

| **SEQ ID NO** | **Corresponds to** | **Description** |
|---|---|---|
| SEQ **ID** NO:1 | SEQ ID NO:1 of international patent application WO2005/087934 | Barley gDNA of cv. Barke spanning the start and stop codons of the gene encoding LOX-1 |
| SEQ ID NO:2 | SEQ ID NO:2 of international patent application WO2005/087934 | Barley gDNA of mutant D112 spanning the region between the start and stop codons of the corresponding gene encoding LOX-1 of cv. Barke |
| SEQ ID NO:3 | SEQ ID NO:3 of international patent application WO2005/087934 | Protein sequence of full-length LOX-1 protein of cv. Barke |
| SEQ ID NO:4 | SEQ ID NO:4 of international patent application WO2005/087934 | Protein sequence of inactive, truncated LOX-1 of mutant D112 |
| SEQ ID NO:5 | SEQ ID NO:1 of international patent application PCT/DK2009/050355 published as WO2010/075860 | The sequence of wild-type genomic DNA encoding LOX-2 from cv. Barke. |
| SEQ ID NO:6 | SEQ ID NO:2 of international patent application PCT/DK2009/050355 published as WO2010/075860 | The sequence of mutant LOX-2 genomic DNA from barley mutant A689. |
| SEQ ID NO:7 | SEQ ID NO:5 of international patent application PCT/DK2009/050355 published as WO2010/075860 | The sequence of full length LOX-2 protein of wild type barley, cv. Barke. |
| SEQ ID NO:8 | SEQ ID NO:6 of international patent application PCT/DK2009/050355 published as WO2010/075860 | The sequence of mutant LOX-2 protein lacking LOX-2 activity from barley mutant A689. |
| SEQ ID NO:9 | SEQ ID NO:3 of international | Barley gDNA of cv. Prestige, |
| | patent application PCT/DK2009/050315 published as WO2010/063288 | genomic sequence spanning the start and stop codons of the gene for MMT. |
| SEQ ID NO:10 | SEQ ID NO:8 of international patent application PCT/DK2009/050315 published as WO2010/063288 | Barley gDNA of Mutant 8063, Genomic sequence for MMT spanning the start and stop codons of the gene for MMT. |
| SEQ ID NO:11 | SEQ ID NO:16 of international patent application PCT/DK2009/050315 published as WO2010/063288 | Barley gDNA of cv. Sebastian, Genomic sequence for MMT spanning the start and stop codons of the gene for MMT. |
| SEQ ID NO:12 | SEQ ID NO:19 of international patent application PCT/DK2009/050315 published as WO2010/063288 | Barley gDNA of Mutant 14018, Genomic sequence for MMT spanning the start and stop codons of the gene for MMT. |
| SEQ ID NO:13 | SEQ ID NO:6 of international patent application PCT/DK2009/050315 published as WO2010/063288 | Sequence for barley MMT of cv. Prestige. |
| SEQ ID NO:14 | SEQ ID NO:18 of international patent application PCT/DK2009/050315 published as WO2010/063288 | Sequence for barley MMT of cv. Sebastian. |
| SEQ ID NO:15 | SEQ ID NO:22 of international patent application PCT/DK2009/050315 published as WO2010/063288 | Entire translated sequence derived from mis-spliced RNA of Mutant 14018. |
| SEQ ID NO:16 | SEQ ID NO:24 of international patent application PCT/DK2009/050315 published as WO2010/063288 | Entire translated sequence derived from mis-spliced RNA of barley Mutant 14018. |
| SEQ ID NO:17 | SEQ ID NO:26 of international patent application PCT/DK2009/050315 published as WO2010/063288 | Entire translated sequence derived from mis-spliced RNA of barley Mutant 14018. |

### REFERENCES CITED

### Patent Documents

U.S. Pat. No. 4.683.195 to Mullis, K.B. et al.
U.S. Pat. No. 4.800.159 to Mullis, K.B. et al.
PCT patent application WO 02/053721 to Douma, A.C. et al.
PCT patent application WO 2005/087934 to Breddam, K. et al.
European patent no. EP 1609866 to Hirota, N. et al.
PCT/DK2009/050315 (published as WO2010/063288) to Knudsen, S. et al.
PCT/DK2009/050355 (published as WO 2010/075860) to Skadhauge, B. et al.

### Other Publications

American Association of Cereal Chemists, "Approved methods of the American Association of Cereal Chemists." ISBN 0-913250-86-4 (1995).
American Society of Brewing Chemists, "Methods of analysis of the American Society of Brewing Chemists." ISBN 1-881696-01-4 (1992).
Baur, C. and Grosch. W., "Investigation about the taste of di-, tri- and tetrahydroxy fatty acids." Z. Lebensm. Unters. Forsch. 165: 82-84 (1977).
Baur, C. et al., "Enzymatic oxidation of linoleic acid: Formation of bittertasting fatty acids." Z. Lebensm. Unters. Forsch. 164:171-176 (1977).
B. Bonacchelli, C. De Brackeleire and F. Harmegnies "Wort boiling - the Meura's concept with wortstripping" Proceedings of the 31 st International Congress of the European Brewery Convention Venice, Italy, 6-10 May 2007, Contribution no. 58, ISBN 978-90-70143-24-4
Briggs, D.E. et al., "Malting and brewing science. Volume I Malt and sweet wort." Chapman and Hall. New York. USA. ISBN 0412165805 (1981).
Durai, S. et al., "Zinc finger nucleases: custom-designed molecular scissors for genome engineering of plant and mammalian cells." Nucleic Acids Res. 33:5978-5990 (2005).
European Brewery Convention. "Analytica- EBC." ISBN 3-418-00759-7 (1998).
Groenqvist, A. et al., "Carbonyl compounds during beer production in beer." Proceedings of the 24th EBC Congress, Oslo, pp. 421-428 (1993).
Hamberg, M., "Trihydroxyoctadecenoic acids in beer: Qualitative and quantitative analysis." J. Agric. Food Chem. 39:1568-1572 (1991).
Hansen, M. et al., "Antisense-mediated suppression of C-hordein biosynthesis in the barley grain results in correlated changes in the transcriptome, protein profile, and amino acid composition." J. Exp. Bot. 58:3987-3995 (2007).
Hough, J.S. et al., "Malting and brewing science. Volume II Hopped wort and beer." Chapman and Hall, New York, USA. ISBN 0412165902 (1982).
lida, S. and Terada, R., "Modification of endogenous natural genes by gene targeting in rice and other higher plants." Plant Mol. Biol. 59:205-219 (2005).
Institute of Brewing, "Institute of Brewing. Methods of analysis." ISBN 0-900489-10-3 (1997).
Jamieson, A.M. and van Gheluwe, J.E.A., "Identification of a compound responsible for cardboard flavor in beer." Proc. Am. Soc. Brew. Chem. 29:192-197 (1970).
Kleinhofs, A. et al., "Induction and selection of specific gene mutations in Hordeum and Pisum." Mut. Res. 51:29-35 (1978).
Kumar, S. et al., "Gene targeting in plants: fingers on the move." Trends Plant Sci. 11:159-161 (2006).
Kuroda, H. et al., "Characterization of factors involved in the production of 2(E)-nonenal during mashing." Biosci. Biotechnol. Biochem. 67:691-697 (2003).
Kuroda, H. et al., "Characterization of 9-fatty acid hydroperoxide lyase-like activity in germinating barley seeds that transforms 9(S)-hydroperoxy-10(E).12(Z)-octadecadienoic acid into 2(E)-nonenal." Biosci. Biotechnol. Biochem. 69:1661-1668 (2005).
Lermusieau, G. et al., "Nonoxidative mechanism for development of trans-2-nonenal in beer." J. Am. Soc. Brew. Chem. 57:29-33 (1999).
Liégeois, C. et al., "Release of deuterated (E)-2-nonenal during beer aging from labeled precursors synthesized before boiling." J. Agric. Food Chem. 50:7634-7638 (2002).
Maquat, L.E. and Carmichael, G.G., "Quality control of mRNA function." Cell 104:173-176 (2001).
Meilgaard, M.C., "Flavor chemistry of beer: Part II: Flavor and threshold of 239 aroma volatiles." Tech. Q. MBAA 12:151-167 (1975).
Mendell, J.T. and Dietz, H.C., "When the message goes awry: Disease-producing mutations that influence mRNA content and performance." Cell 107:411-414 (2002).
Nevo, E., "Resources for Breeding of Wild Barley." In: "Barley: Genetics. Biochemistry. Molecular Biology and Biotechnology." Shewry. P.R., ed., pp. 3-18. C.A.B. International. ISBN 0-85198-725-7 (1992).
Nyborg, M. et al., "Investigations of the protective mechanism of sulfite against beer staling and formation of adducts with trans-2-nonenal." J. Am. Soc. Brew. Chem. 57:24-28 (1999).
Rasmussen, S.K. and Hatzack, F., "Identification of two low-phytate barley (Hordeum vulgare L.) grain mutants by TLC and genetic analysis." Hereditas 129:107-112 (1998).
Robbins, M.P. et al., "Gene manipulation of condensed tannins in higher plants." Plant Physiol. 116:1133-1144 (1998).
Sambrook, J. and Russell. D.W., "Molecular Cloning. A Laboratory Manual. 3rd Ed.", Cold Spring Harbor Laboratory Press. Cold Spring Harbor. New York. ISBN 0-87969-577-3 (2001).
Schmitt, N.F. and van Mechelen. J.R., "Expression of lipoxygenase isoenzymes in developing barley grains." Plant Sci. 128:141-150 (1997).
Stahl, Y. et al., "Antisense downregulation of the barley limit dextrinase inhibitor modulates starch granule sizes distribution. starch composition and amylopectin structure". Plant J. 39:599-611 (2004).
Tzfira, T. and White. C., "Towards targeted mutagenesis and gene replacement in plants." Trends Biotechnol. 23:567-569 (2005).
von Bothmer, R. et al., "Diversity in barley (Hordeum vulgare)." In: "Diversity in Barley (Hordeum vulgare)." von Bothmer, R., van Hintum, T., Knüpffer, H., Sato. K., eds., pp. 129-136. ISBN 0-444-50587-7 (2003). Also available at http://www.genres.de/.
Wackerbauer, K. and Meyna, S., "Freie und triglyceride-gebundene Hydroxyfettsäuren in Gerste und Malz", Monatsschrift fur Brauwissenschaft, heft 3/4: 52-57 (2002).
Wu, J. et al., "Nonsense-mediated mRNA decay (NMD) silences the accumulation of aberrant trypsin proteinase inhibitor mRNA in Nicotiana attenuata." Plant J. 51:693-706 (2007).

## Claims

1. A method for preparing a barley based beverage with low levels of one or more off-flavours and/or precursors thereof, wherein the method involves reduced energy input, the method comprising the steps of:
(i) providing a barley plant or part thereof, wherein said barley plant comprises:
(a) a first mutation that results in a total loss of functional lipoxygenase (LOX)-1; and
(b) a second mutation resulting in a total loss of functional LOX-2; and
(c) a third mutation resulting in a total loss of functional S-adenosylmethionine:methionine S-methyltransferase (MMT);
(ii) optionally malting at least part of said barley, thereby obtaining malted barley;
(iii) mashing said barley and/or malted barley and optionally additional adjuncts, thereby obtaining a wort;
(iv) heating said wort optionally in the presence of additional ingredient(s), wherein at the most 4% of the wort volume is evaporated, thereby obtaining heated wort;
(v) processing said heated wort into a beverage;
thereby preparing the barley based beverage with low levels of one or more off-flavours and/or precursors thereof.

2. The method according to claim 1, wherein step (i) comprises providing barley kernels.

3. The method according to anyone of the preceding claims, wherein said barley is malted using a method comprising the steps of
a) steeping said barley;
b) germinating said barley;
c) kiln drying said barley
and wherein kiln drying of said barley is performed at a temperature of at the most 80°C, yet more preferably at a temperature of at the most 75°C, such as at temperature of at the most 70°C, for example at a temperature of at the most 65°C, such as at temperature of at the most 60°C, for example at a temperature of at the most 55°C, such as at a temperature of at the most 50 °C, for example at a temperature of at the most 45°C, such as at a temperature of at the most 40°C.

4. The method according to anyone of the preceding claims, wherein said wort is heated to a temperature of at the most 99.8°C, such as at the most 99.5°C, for example at the most 99°C.

5. The method according to anyone of the preceding claims, wherein step v) comprises fermentation of the wort.

6. The method according to anyone of the preceding claims, wherein the method does not involve heating to a temperature above 80°C for more than 30 min, preferably for more than 20 min.

7. A barley plant, or a part thereof, wherein said barley plant comprises:
a) a first mutation that results in a total loss of functional LOX-1, and
b) a second mutation resulting in a total loss of functional LOX-2 and
c) a third mutation resulting in a total loss of functional MMT.

8. The barley plant, or a part thereof, according to claims 7, wherein the gene encoding LOX-1 of said plant comprises a premature stop codon situated at the most 705, preferably at the most 665 codons downstream of the start codon.

9. The barley plant, or a part thereof, according to any one of claims 7 to 8, wherein the gene encoding LOX-2 of said plant comprises a premature stop codon situated at the most 707, preferably at the most 684 codons downstream of the start codon.

10. The barley plant, or a part thereof, according to any one of claims 7 to 8, wherein the mutation in the gene encoding MMT is a mutation within a 5' splice site of an intron, such as a G→A mutation of the terminal 5' base of an intron.

11. The barley plant, or a part thereof, according to anyone of claims 7 to 10, wherein the mutation results in a gene encoding a truncated form of MMT comprising an N-terminal fragment of wild-type MMT and optionally additional C-terminal sequences not found in wild-type MMT, wherein said N-terminal fragment comprises at the most the 500, more preferably at the most the 450, even more preferably at the most the 400, yet more preferably at the most the 350, even more preferably at the most the 320, yet more preferably at the most 311, or at the most 288 N-terminal amino acid residues of SEQ ID NO:13.

12. A malt composition comprising a processed barley plant, or a part thereof, wherein said barley plant is the barley plant according to any of claims 7 to 11.

13. A barley based beverage preparable from a barley plant, or a part thereof, according to any one of claims 7 to 11, wherein said beverage contains a level of dimethyl sulfide (DMS) below 10 ppb.

14. The beverage according to claim 13, wherein said beverage is prepared by the method according to anyone of claims 1 to 6.

15. The beverage according to any one of claims 13 to 14, wherein the beverage is beer.

16. The beverage according to any one of claims 13 to 15, wherein the beverage is a non-alcoholic malt beverage.

## Patentansprüche

1. Verfahren zur Herstellung eines Getränks auf Gerstenbasis mit niedrigen Werten eines oder mehrerer Fremdaromen und/oder Vorläufern davon, wobei das Verfahren einen reduzierten Energieeintrag beinhaltet, wobei das Verfahren die folgenden Schritte umfasst:
(i) das Bereitstellen einer Gerstenpflanze oder eines Teils davon, wobei die Gerstenpflanze Folgendes umfasst:
(a) eine erste Mutation, die zu einem Totalverlust der funktionellen Lipoxygenase (LOX)-1 führt; und
(b) eine zweite Mutation, die zu einem Totalverlust von funktioneller LOX-2 führt; und
(c) eine dritte Mutation, die zu einem Totalverlust der funktionellen S-Adenosylmethionin:Methionin-S-Methyltransferase (MMT) führt;
(ii) optional das Mälzen wenigstens eines Teils der Gerste, wodurch gemälzte Gerste erhalten wird;
(iii) das Einmaischen der Gerste und/oder der gemälzten Gerste und optional von zusätzlichen Zusätzen, wodurch eine Bierwürze erhalten wird;
(iv) das Erhitzen der Bierwürze, optional in Anwesenheit eines zusätzlichen Inhaltsstoffs/zusätzlicher Inhaltsstoffe, wobei höchstens 4 % des Volumens der Bierwürze evaporiert wird, wodurch eine erhitzte Bierwürze erhalten wird;
(v) das Verarbeiten der erhitzten Bierwürze in ein Getränk; wodurch das Getränk auf Gerstenbasis mit niedrigen Werten eines oder mehrerer Fremdaromen und/oder Vorläufern davon hergestellt wird.

2. Verfahren nach Anspruch 1, wobei Schritt (i) das Bereitstellen von Gerstenkörnern umfasst.

3. Verfahren nach einem der vorstehenden Ansprüche, wobei die Gerste unter Verwendung eines Verfahrens gemälzt wird, umfassend die folgenden Schritte:
a) Einlaugen der Gerste;
b) Auskeimen der Gerste;
c) Ofentrocknen der Gerste
und wobei das Ofentrocknen der Gerste bei einer Temperatur von höchstens 80 °C, jedoch eher bevorzugt bei einer Temperatur von höchstens 75 °C, wie z.B. bei einer Temperatur von höchstens 70 °C, z.B. bei einer Temperatur von höchstens 65 °C, wie z.B. bei einer Temperatur von höchstens 60 °C, z.B. bei einer Temperatur von höchstens 55 °C, wie z.B. bei einer Temperatur von höchstens 50 °C, z.B. bei einer Temperatur von höchstens 45 °C, wie z.B. bei einer Temperatur von höchstens 40 °C durchgeführt wird.

4. Verfahren nach einem der vorhergehenden Ansprüche, wobei die Bierwürze auf eine Temperatur von höchstens 99,8 °C, wie z.B. auf höchstens 99,5 °C, z.B. auf höchstens 99 °C erhitzt wird.

5. Verfahren nach einem der vorhergehenden Ansprüche, wobei Schritt v) die Gärung der Bierwürze umfasst.

6. Verfahren nach einem der vorhergehenden Ansprüche, wobei das Verfahren kein Erhitzen auf eine Temperatur über 80 °C für länger als 30 min, bevorzugt für länger als 20 min beinhaltet.

7. Gerstenpflanze oder ein Teil davon, wobei die Gerstenpflanze Folgendes umfasst:
a) eine erste Mutation, die zu einem Totalverlust von funktioneller LOX-1 führt und
(b) eine zweite Mutation, die zu einem Totalverlust von funktioneller LOX-2 führt und
(c) eine dritte Mutation, die zu einem Totalverlust von funktioneller MMT führt.

8. Gerstenpflanze oder ein Teil davon nach Anspruch 7, wobei das für LOX-1 codierende Gen der Pflanze ein vorzeitiges Stoppcodon umfasst, das dem Startcodon höchstens 705, bevorzugt höchstens 665 Codone nachgeordnet ist.

9. Gerstenpflanze oder ein Teil davon nach einem der Ansprüche 7 bis 8, wobei das für LOX-2 codierende Gen der Pflanze ein vorzeitiges Stoppcodon umfasst, das dem Startcodon höchstens 707, bevorzugt höchstens 684 Codone nachgeordnet ist.

10. Gerstenpflanze oder ein Teil davon nach einem der Ansprüche 7 bis 8, wobei die Mutation in dem für MMT codierenden Gen eine Mutation innerhalb einer 5'-Spleißstelle eines Introns ist, wie z.B. eine G→A-Mutation der terminalen 5'-Base eines Introns.

11. Gerstenpflanze oder ein Teil davon nach einem der Ansprüche 7 bis 10, wobei die Mutation zu einem Gen führt, das für eine verkürzte Form von MMT codiert, umfassend ein N-terminales Fragment des Wildtyp-MMTs und optional zusätzliche C-terminale Sequenzen, die nicht im Wildtyp-MMT zu finden sind, wobei das N-terminale Fragment höchstens die 500, eher bevorzugt höchstens die 450, noch eher bevorzugt höchstens die 400, noch eher bevorzugt höchstens die 350, noch eher bevorzugt höchstens die 320, noch eher bevorzugt höchstens die 311 oder höchstens die 288 N-terminalen-Aminosäurereste von SEQ ID NO: 13 umfasst.

12. Malzzusammensetzung, umfassend eine verarbeitete Gerstenpflanze oder einen Teil davon, wobei die Gerstenpflanz die Gerstenpflanze gemäß einem der Ansprüche 7 bis 11 ist.

13. Getränk auf Gerstenbasis, das aus einer Gerstenpflanz oder einem Teil davon gemäß einem der Ansprüche 7 bis 11 herstellbar ist, wobei das Getränk einen Wert an Dimethylsulfid (DMS) unter 10 ppb enthält.

14. Getränk nach Anspruch 13, wobei das Getränk durch das Verfahren gemäß einem der Ansprüche 1 bis 6 hergestellt wird.

15. Getränk nach einem der Ansprüche 13 bis 14, wobei es sich bei dem Getränk um Bier handelt.

16. Getränk nach einem der Ansprüche 13 bis 15, wobei es sich bei dem Getränk um ein alkoholfreies Malzgetränk handelt.

## Revendications

1. Procédé de préparation d'une boisson à base d'orge avec des taux bas d'un ou de plusieurs arômes indésirables et/ou de leurs précurseurs, dans lequel le procédé implique la réduction de l'apport énergétique, le procédé comprenant les étapes de :
(i) fourniture d'une plante d'orge ou de l'une de ses parties, dans laquelle ladite plante d'orge comprend :
(a) une première mutation qui entraîne une perte totale de lipoxygénase (LOX)-1 fonctionnelle ; et
(b) une deuxième mutation entraînant une perte totale de LOX-2 fonctionnelle ; et
(c) une troisième mutation entraînant une perte totale de méthionine S-méthyltransférase (MMT) fonctionnelle ;
(ii) maltage éventuel d'au moins une partie de ladite orge, obtenant de cette façon de l'orge malté ;
(iii) brassage dudit orge et/ou orge malté et éventuellement de produits d'addition supplémentaires, obtenant de cette façon un moût ;
(iv) chauffage dudit moût éventuellement en présence d'un ou de plusieurs ingrédients supplémentaires, dans lequel au plus 4 % du volume du moût est évaporé, obtenant de cette façon un moût chauffé ;
(v) traitement dudit moût chauffé en une boisson ;
préparant de cette façon la boisson à base d'orge avec des taux bas d'un ou de plusieurs arômes indésirables et/ou de leurs précurseurs.

2. Procédé selon la revendication 1, dans lequel l'étape (i) comprend la fourniture de grains d'orge.

3. Procédé selon l'une quelconque des revendications précédentes, dans lequel ledit orge est malté en utilisant un procédé comprenant les étapes de
(a) trempage dudit orge ;
(b) germination dudit orge ;
(c) touraillage dudit orge
et dans lequel le touraillage dudit orge est effectué à une température d'au plus 80 °C, de façon encore davantage préférée à une température d'au plus 75 °C, comme à une température d'au plus 70 °C, par exemple à une température d'au plus 65 °C, comme à une température d'au plus 60 °C, par exemple à une température d'au plus 55 °C, comme à une température d'au plus 50 °C, par exemple à une température d'au plus 45 °C, comme à une température d'au plus 40 °C.

4. Procédé selon l'une quelconque des revendications précédentes, dans lequel ledit moût est chauffé jusqu'à une température d'au plus 99,8 °C, comme d'au plus 99,5 °C, par exemple d'au plus 99 °C.

5. Procédé selon l'une quelconque des revendications précédentes, dans lequel l'étape v) comprend la fermentation du moût.

6. Procédé selon l'une quelconque des revendications précédentes, dans lequel le procédé n'implique pas le chauffage jusqu'à une température supérieure à 80 °C pendant plus de 30 min, de préférence pendant plus de 20 min.

7. Plante d'orge, ou l'une de ses parties, dans laquelle ladite plante d'orge comprend :
a) une première mutation qui entraîne une perte totale de LOX-1 fonctionnelle, et
b) une deuxième mutation entraînant une perte totale de LOX-2 fonctionnelle et
c) une troisième mutation entraînant une perte totale de MMT fonctionnelle.

8. Plante d'orge, ou l'une de ses parties, selon la revendication 7, dans laquelle le gène codant pour la LOX-1 de ladite plante comprend un codon d'arrêt prématuré situé au plus à 705, de préférence au plus à 665 codons en aval du codon de départ.

9. Plante d'orge, ou l'une de ses parties, selon l'une quelconque des revendications 7 à 8, dans laquelle le gène codant pour la LOX-2 de ladite plante comprend un codon d'arrêt prématuré situé au plus à 707, de préférence au plus à 684 codons en aval du codon de départ.

10. Plante d'orge, ou l'une de ses parties, selon l'une quelconque des revendications 7 à 8, dans laquelle la mutation dans le gène codant pour la MMT est une mutation au sein d'un site d'épissage en 5' d'un intron, comme une mutation G→A de la base terminale en 5' d'un intron.

11. Plante d'orge, ou l'une de ses parties, selon l'une quelconque des revendications 7 à 10, dans laquelle la mutation conduit à un gène codant pour une forme tronquée de la MMT comprenant un fragment N-terminal de la MMT de type sauvage et éventuellement des séquences C-terminales supplémentaires non trouvées dans la MMT de type sauvage, dans laquelle ledit fragment N-terminal comprend au plus les 500, de façon davantage préférée au plus les 450, de façon même davantage préférée au plus les 400, de façon encore davantage préférée au plus les 350, de façon même davantage préférée au plus les 320, de façon encore davantage préférée au plus les 311, ou au plus les 288 résidus d'acides aminés N-terminaux de SEQ ID NO : 13.

12. Composition de malt comprenant une plante d'orge, ou l'une de ses parties, traitée, dans laquelle ladite plante d'orge est la plante d'orge selon l'une quelconque des revendications 7 à 11.

13. Boisson à base d'orge pouvant être préparée à partir d'une plante d'orge, ou de l'une de ses parties, selon l'une quelconque des revendications 7 à 11, dans laquelle ladite boisson contient un taux de sulfure de diméthyle (DMS) inférieur à 10 ppb.

14. Boisson selon la revendication 13, dans laquelle ladite boisson est préparée par le procédé selon l'une quelconque des revendications 1 à 6.

15. Boisson selon l'une quelconque des revendications 13 à 14, dans laquelle la boisson est de la bière.

16. Boisson selon l'une quelconque des revendications 13 à 15, dans laquelle la boisson est une boisson de malt non alcoolisée.


### SEQUENCE LISTING

<110> Carlsberg Breweries A/S Heineken Supply Chain B.V.
<120> Energy saving brewing method
<130> P2253PC00
<160> 20
<170> PatentIn version 3.5
<210> 1
   <211> 4165
   <212> DNA
   <213> Hordeum vulgare cv. Barke
<400> 1
   atgctgctgg gagggctgat cgacaccctc acgggggcga acaagagcgc ccggctcaag      60
   ggcacggtgg tgctcatgcg caagaacgtg ctggacctca acgacttcgg cgccaccatc     120
   atcgacggca tcggcgagtt cctcggcaag ggcgtcacct gccagcttat cagctccacc     180
   gccgtcgacc aaggtaatca ctaccctcct ccggccttct cctctgttta caagatatag     240
   tatttctttc gtgtgggccg gcggccatgg atggatggat gtgtctggat cggctaaaga     300
   agataggata gctagccctg gccggtcgtc tttacctgag catgggcata tgccatcgaa     360
   aaaagagaca acagcatgca tgcatggtgc gcgcaccaga ccacgcagag caccggatgc     420
   tcgagacaaa gcaacacaac aagcaaggac gacacgtcaa aagcaacaca acaagcaagg     480
   acggcacgtc aaaagcaaca caaacctaaa ctaaagcaca aagacgtaag agcaagcaca     540
   caatcagcag gctataaaca gttgtcatca aaaacaacgc tggaagagag agagaaggaa     600
   ggaagtagta gccatgaaaa attaaatcac cgggcgttgc tctttgccca acaattaatc     660
   aagcaggata cgtggcatgt atagttcttg taagtaaact aagcatgtga tatgagaagg     720
   tacgtggtgg tgcagacaac ggcggtcgcg ggaaggtggg cgcggaggcg gagctggagc     780
   agtgggtgac gagcctgccg tcgctgacga cgggggagtc caagttcggc ctcaccttcg     840
   actgggaggt ggagaagctc ggggtgccgg gcgccatcgt cgtcaacaac taccacagct     900
   ccgagttcct gcttaaaacc atcaccctcc acgacgtccc cggccgcagc ggcaacctca     960
   ccttcgtcgc caactcatgg atctaccccg ccgccaacta ccgatacagc cgcgtcttct    1020
   tcgccaacga cgtgcgtgga ttttcctcta ctttcctctc ctttcatttt caccgccttc    1080
   gtcattcatg gtcgatcatt aagtcttgcc aggacaatag atgatgagct aggagtggtt    1140
   accacttagc agtacgtaca ttatttattc cgtgttggta gaaaaggata tggtttggtg    1200
   cagatcgaca caagattgaa tgaaagttgc accgtggcac cgtggcagcg tggtaggtga    1260
   aaataactgt tgcacggatc cacccacatg attgttttca tgaataaact ttttaaggat    1320
   gtgtctagcc acatctagat gcatgtcaca taattattgc ataccaaaac gattaaatta 1380
   agcataaaaa gaaaaggaaa aaaatactca catatctcga cgtaagatca atgatatagt 1440
   atttagatat gcaatattta tcttacatct aaacctttct tcattcctaa atataagaca 1500
   tttgtaagat ttcactatgg acaacatacg aaacaaaatc agtggatctc tctatgcatt 1560
   cattatgtag tctataataa aatctttaaa agatcgtata ttttgcaacg gagggagtaa 1620
   aacataactt tttaatagta atgttgcacg gctccacact cgcagacgta cctgccgagc 1680
   cagatgccgg cggcgctgaa gccgtaccgc gacgacgagc tccggaacct gcgtggcgac 1740
   gaccagcagg gcccgtacca ggagcacgac cgcatctacc gctacgacgt ctacaacgac 1800
   ctcggcgagg gccgccccat cctcggcggc aactccgacc acccttaccc gcgccgcggc 1860
   cgcacggagc gcaagcccaa cgccagcgac ccgagcctgg agagccggct gtcgctgctg 1920
   gagcagatct acgtgccgcg ggacgagaag ttcggccacc tcaagacgtc cgacttcctg 1980
   ggctactcca tcaaggccat cacgcagggc atcctgccgg ccgtgcgcac ctacgtggac 2040
   accacccccg gcgagttcga ctccttccag gacatcatca acctctatga gggcggcatc 2100
   aagctgccca aggtggccgc cctggaggag ctccgtaagc agttcccgct ccagctcatc 2160
   aaggacctcc tccccgtcgg cggcgactcc ctgcttaagc tccccgtgcc ccacatcatc 2220
   caggagaaca agcaggcgtg gaggaccgac gaggagttcg cacgggaggt gctcgccggc 2280
   gtcaacccgg tcatgatcac gcgtctcacg gtgagtcagc gattatttgt tcattgtgtg 2340
   tgtatggtgt ccatggtgag aaagtgcaga tcttgatttg cgttgggtcg catgcacgca 2400
   tgctgcatgc atgcaggagt tcccgccaaa aagtagtctg gaccctagca agtttggtga 2460
   ccacaccagc accatcacgg cggagcacat agagaagaac ctcgagggcc tcacggtgca 2520
   gcaggtaatt ggtccaagcc atcgacatca actatgattt acctaggagt aattggtagc 2580
   tgtagataat ttggcttcgt tgcaattaat ttgatgctgg ccgatcaagt gatcgtattg 2640
   ggtttgaaat ttgcaggcgc tggaaagcaa caggctgtac atccttgatc accatgaccg 2700
   gttcatgccg ttcctgatcg acgtcaacaa cctgcccggc aacttcatct acgccacgag 2760
   gaccctcttc ttcctgcgcg gcgacggcag gctcacgccg ctcgccatcg agctgagcga 2820
   gcccatcatc cagggcggcc ttaccacggc caagagcaag gtttacacgc cggtgcccag 2880
   cggctccgtc gaaggctggg tgtgggagct cgccaaggcc tacgtcgccg tcaatgactc 2940
   cgggtggcac cagctcgtca gccactggta cgttctccac ggtcgatgtg attcagtcag 3000
   tcgatgcaca acaactgatc gaaatatgat tgattgaaac gcgcaggctg aacactcacg 3060
   cggtgatgga gccgttcgtg atctcgacga accggcacct tagcgtgacg cacccggtgc 3120
   acaagctgct gagcccgcac taccgcgaca ccatgaccat caacgcgctg gcgcggcaga 3180
   cgctcatcaa cgccggcggc atcttcgaga tgacggtgtt cccgggcaag ttcgcgttgg 3240
   ggatgtcggc cgtggtgtac aaggactgga agttcaccga gcagggactg ccggacgatc 3300
   tcatcaagag gtacgtacct ggtaaatgtt atgaatgtgt aaaacaaatt gggcgtctcg 3360
   ctcactgaca ggaacgtggt aaaaaaaatg caggggcatg gcggtggagg acccgtcgag 3420
   cccgtacaag gtgcggttgc tggtgtcgga ctacccgtac gcggcggacg ggctggcgat 3480
   ctggcacgcc attgagcagt acgtgagcga gtacctggcc atctactacc cgaacgacgg 3540
   cgtgctgcag ggcgatacgg aggtgcaggc gtggtggaag gagacgcgcg aggtcgggca 3600
   cggcgacctc aaggacgccc catggtggcc caagatgcaa agtgtgccgg agctggccaa 3660
   ggcgtgcacc accatcatct ggatcgggtc ggcgctgcat gcggcagtca acttcgggca 3720
   gtacccctac gcggggttcc tcccgaaccg gccgacggtg agccggcgcc gcatgccgga 3780
   gcccggcacg gaggagtacg cggagctgga gcgcgacccg gagcgggcct tcatccacac 3840
   catcacgagc cagatccaga ccatcatcgg cgtgtcgctg ctggaggtgc tgtcgaagca 3900
   ctcctccgac gagctgtacc tcgggcagcg ggacacgccg gagtggacct cggacccaaa 3960
   ggccctggag gtgttcaagc ggttcagcga ccggctggtg gagatcgaga gcaaggtggt 4020
   gggcatgaac catgacccgg agctcaagaa ccgcaacggc ccggctaagt ttccctacat 4080
   gctgctctac cccaacacct ccgaccacaa gggcgccgct gccgggctta ccgccaaggg 4140
   catccccaac agcatctcca tctaa 4165
<210> 2
   <211> 4165
   <212> DNA
   <213> Hordeum vulgare mutant D112
<400> 2
   atgctgctgg gagggctgat cgacaccctc acgggggcga acaagagcgc ccggctcaag 60
   ggcacggtgg tgctcatgcg caagaacgtg ctggacctca acgacttcgg cgccaccatc 120
   atcgacggca tcggcgagtt cctcggcaag ggcgtcacct gccagcttat cagctccacc 180
   gccgtcgacc aaggtaatca ctaccctcct ccggccttct cctctgttta caagatatag 240
   tatttctttc gtgtgggccg gcggccatgg atggatggat gtgtctggat cggctaaaga 300
   agataggata gctagccctg gccggtcgtc tttacctgag catgggcata tgccatcgaa 360
   aaaagagaca acagcatgca tgcatggtgc gcgcaccaga ccacgcagag caccggatgc 420
   tcgagacaaa gcaacacaac aagcaaggac gacacgtcaa aagcaacaca acaagcaagg 480
   acggcacgtc aaaagcaaca caaacctaaa ctaaagcaca aagacgtaag agcaagcaca 540
   caatcagcag gctataaaca gttgtcatca aaaacaacgc tggaagagag agagaaggaa 600
   ggaagtagta gccatgaaaa attaaatcac cgggcgttgc tctttgccca acaattaatc 660
   aagcaggata cgtggcatgt atagttcttg taagtaaact aagcatgtga tatgagaagg 720
   tacgtggtgg tgcagacaac ggcggtcgcg ggaaggtggg cgcggaggcg gagctggagc 780
   agtgggtgac gagcctgccg tcgctgacga cgggggagtc caagttcggc ctcaccttcg 840
   actgggaggt ggagaagctc ggggtgccgg gcgccatcgt cgtcaacaac taccacagct 900
   ccgagttcct gcttaaaacc atcaccctcc acgacgtccc cggccgcagc ggcaacctca 960
   ccttcgtcgc caactcatgg atctaccccg ccgccaacta ccgatacagc cgcgtcttct 1020
   tcgccaacga cgtgcgtgga ttttcctcta ctttcctctc ctttcatttt caccgccttc 1080
   gtcattcatg gtcgatcatt aagtcttgcc aggacaatag atgatgagct aggagtggtt 1140
   accacttagc agtacgtaca ttatttattc cgtgttggta gaaaaggata tggtttggtg 1200
   cagatcgaca caagattgaa tgaaagttgc accgtggcac cgtggcagcg tggtaggtga 1260
   aaataactgt tgcacggatc cacccacatg attgttttca tgaataaact ttttaaggat 1320
   gtgtctagcc acatctagat gcatgtcaca taattattgc ataccaaaac gattaaatta 1380
   agcataaaaa gaaaaggaaa aaaatactca catatctcga cgtaagatca atgatatagt 1440
   atttagatat gcaatattta tcttacatct aaacctttct tcattcctaa atataagaca 1500
   tttgtaagat ttcactatgg acaacatacg aaacaaaatc agtggatctc tctatgcatt 1560
   cattatgtag tctataataa aatctttaaa agatcgtata ttttgcaacg gagggagtaa 1620
   aacataactt tttaatagta atgttgcacg gctccacact cgcagacgta cctgccgagc 1680
   cagatgccgg cggcgctgaa gccgtaccgc gacgacgagc tccggaacct gcgtggcgac 1740
   gaccagcagg gcccgtacca ggagcacgac cgcatctacc gctacgacgt ctacaacgac 1800
   ctcggcgagg gccgccccat cctcggcggc aactccgacc acccttaccc gcgccgcggc 1860
   cgcacggagc gcaagcccaa cgccagcgac ccgagcctgg agagccggct gtcgctgctg 1920
   gagcagatct acgtgccgcg ggacgagaag ttcggccacc tcaagacgtc cgacttcctg 1980
   ggctactcca tcaaggccat cacgcagggc atcctgccgg ccgtgcgcac ctacgtggac 2040
   accacccccg gcgagttcga ctccttccag gacatcatca acctctatga gggcggcatc 2100
   aagctgccca aggtggccgc cctggaggag ctccgtaagc agttcccgct ccagctcatc 2160
   aaggacctcc tccccgtcgg cggcgactcc ctgcttaagc tccccgtgcc ccacatcatc 2220
   caggagaaca agcaggcgtg gaggaccgac gaggagttcg cacgggaggt gctcgccggc 2280
   gtcaacccgg tcatgatcac gcgtctcacg gtgagtcagc gattatttgt tcattgtgtg 2340
   tgtatggtgt ccatggtgag aaagtgcaga tcttgatttg cgttgggtcg catgcacgca 2400
   tgctgcatgc atgcaggagt tcccgccaaa aagtagtctg gaccctagca agtttggtga 2460
   ccacaccagc accatcacgg cggagcacat agagaagaac ctcgagggcc tcacggtgca 2520
   gcaggtaatt ggtccaagcc atcgacatca actatgattt acctaggagt aattggtagc 2580
   tgtagataat ttggcttcgt tgcaattaat ttgatgctgg ccgatcaagt gatcgtattg    2640
   ggtttgaaat ttgcaggcgc tggaaagcaa caggctgtac atccttgatc accatgaccg    2700
   gttcatgccg ttcctgatcg acgtcaacaa cctgcccggc aacttcatct acgccacgag    2760
   gaccctcttc ttcctgcgcg gcgacggcag gctcacgccg ctcgccatcg agctgagcga    2820
   gcccatcatc cagggcggcc ttaccacggc caagagcaag gtttacacgc cggtgcccag    2880
   cggctccgtc gaaggctggg tgtgggagct cgccaaggcc tacgtcgccg tcaatgactc    2940
   cgggtggcac cagctcgtca gccactggta cgttctccac ggtcgatgtg attcagtcag    3000
   tcgatgcaca acaactgatc gaaatatgat tgattgaaac gcgcaggctg aacactcacg    3060
   cggtgatgga gccgttcgtg atctcgacga accggcacct tagcgtgacg cacccggtgc    3120
   acaagctgct gagcccgcac taccgcgaca ccatgaccat caacgcgctg gcgcggcaga    3180
   cgctcatcaa cgccggcggc atcttcgaga tgacggtgtt cccgggcaag ttcgcgttgg    3240
   ggatgtcggc cgtggtgtac aaggactgga agttcaccga gcagggactg ccggacgatc    3300
   tcatcaagag gtacgtacct ggtaaatgtt atgaatgtgt aaaacaaatt gggcgtctcg    3360
   ctcactgaca ggaacgtggt aaaaaaaatg caggggcatg gcggtggagg acccgtcgag    3420
   cccgtacaag gtgcggttgc tggtgtcgga ctacccgtac gcggcggacg ggctggcgat    3480
   ctggcacgcc attgagcagt acgtgagcga gtacctggcc atctactacc cgaacgacgg    3540
   cgtgctgcag ggcgatacgg aggtgcaggc gtgatggaag gagacgcgcg aggtcgggca    3600
   cggcgacctc aaggacgccc catggtggcc caagatgcaa agtgtgccgg agctggccaa    3660
   ggcgtgcacc accatcatct ggatcgggtc ggcgctgcat gcggcagtca acttcgggca    3720
   gtacccctac gcggggttcc tcccgaaccg gccgacggtg agccggcgcc gcatgccgga    3780
   gcccggcacg gaggagtacg cggagctgga gcgcgacccg gagcgggcct tcatccacac    3840
   catcacgagc cagatccaga ccatcatcgg cgtgtcgctg ctggaggtgc tgtcgaagca    3900
   ctcctccgac gagctgtacc tcgggcagcg ggacacgccg gagtggacct cggacccaaa    3960
   ggccctggag gtgttcaagc ggttcagcga ccggctggtg gagatcgaga gcaaggtggt    4020
   gggcatgaac catgacccgg agctcaagaa ccgcaacggc ccggctaagt ttccctacat    4080
   gctgctctac cccaacacct ccgaccacaa gggcgccgct gccgggctta ccgccaaggg    4140
   catccccaac agcatctcca tctaa                                          4165
<210> 3
   <211> 862
   <212> PRT
   <213> Hordeum vulgare cv. Barke
<400> 3
   Met Leu Leu Gly Gly Leu Ile Asp Thr Leu Thr Gly Ala Asn Lys Ser
   1               5                   10                  15
   Ala Arg Leu Lys Gly Thr Val Val Leu Met Arg Lys Asn Val Leu Asp
               20                  25                  30
   Leu Asn Asp Phe Gly Ala Thr Ile Ile Asp Gly Ile Gly Glu Phe Leu
           35                  40                  45
   Gly Lys Gly Val Thr Cys Gln Leu Ile Ser Ser Thr Ala Val Asp Gln
       50                  55                  60
   Asp Asn Gly Gly Arg Gly Lys Val Gly Ala Glu Ala Glu Leu Glu Gln
   65                  70                  75                  80
   Trp Val Thr Ser Leu Pro Ser Leu Thr Thr Gly Glu Ser Lys Phe Gly
                   85                  90                  95
   Leu Thr Phe Asp Trp Glu Val Glu Lys Leu Gly Val Pro Gly Ala Ile
               100                 105                 110
   Val Val Asn Asn Tyr His Ser Ser Glu Phe Leu Leu Lys Thr Ile Thr
           115                 120                 125
   Leu His Asp Val Pro Gly Arg Ser Gly Asn Leu Thr Phe Val Ala Asn
       130                 135                 140
   Ser Trp Ile Tyr Pro Ala Ala Asn Tyr Arg Tyr Ser Arg Val Phe Phe
   145                 150                 155                 160
   Ala Asn Asp Thr Tyr Leu Pro Ser Gln Met Pro Ala Ala Leu Lys Pro
                   165                 170                 175
   Tyr Arg Asp Asp Glu Leu Arg Asn Leu Arg Gly Asp Asp Gln Gln Gly
               180                 185                 190
   Pro Tyr Gln Glu His Asp Arg Ile Tyr Arg Tyr Asp Val Tyr Asn Asp
           195                 200                 205
   Leu Gly Glu Gly Arg Pro Ile Leu Gly Gly Asn Ser Asp His Pro Tyr
       210                 215                 220
   Pro Arg Arg Gly Arg Thr Glu Arg Lys Pro Asn Ala Ser Asp Pro Ser
   225                 230                 235                 240
   Leu Glu Ser Arg Leu Ser Leu Leu Glu Gln Ile Tyr Val Pro Arg Asp
                   245                 250                 255
   Glu Lys Phe Gly His Leu Lys Thr Ser Asp Phe Leu Gly Tyr Ser Ile
   260 265 270
   Lys Ala Ile Thr Gln Gly Ile Leu Pro Ala Val Arg Thr Tyr Val Asp
   275 280 285
   Thr Thr Pro Gly Glu Phe Asp Ser Phe Gln Asp Ile Ile Asn Leu Tyr
   290 295 300
   Glu Gly Gly Ile Lys Leu Pro Lys Val Ala Ala Leu Glu Glu Leu Arg
   305 310 315 320
   Lys Gln Phe Pro Leu Gln Leu Ile Lys Asp Leu Leu Pro Val Gly Gly
   325 330 335
   Asp Ser Leu Leu Lys Leu Pro Val Pro His Ile Ile Gln Glu Asn Lys
   340 345 350
   Gln Ala Trp Arg Thr Asp Glu Glu Phe Ala Arg Glu Val Leu Ala Gly
   355 360 365
   Val Asn Pro Val Met Ile Thr Arg Leu Thr Glu Phe Pro Pro Lys Ser
   370 375 380
   Ser Leu Asp Pro Ser Lys Phe Gly Asp His Thr Ser Thr Ile Thr Ala
   385 390 395 400
   Glu His Ile Glu Lys Asn Leu Glu Gly Leu Thr Val Gln Gln Ala Leu
   405 410 415
   Glu Ser Asn Arg Leu Tyr Ile Leu Asp His His Asp Arg Phe Met Pro
   420 425 430
   Phe Leu Ile Asp Val Asn Asn Leu Pro Gly Asn Phe Ile Tyr Ala Thr
   435 440 445
   Arg Thr Leu Phe Phe Leu Arg Gly Asp Gly Arg Leu Thr Pro Leu Ala
   450 455 460
   Ile Glu Leu Ser Glu Pro Ile Ile Gln Gly Gly Leu Thr Thr Ala Lys
   465 470 475 480
   Ser Lys Val Tyr Thr Pro Val Pro Ser Gly Ser Val Glu Gly Trp Val
   485 490 495
   Trp Glu Leu Ala Lys Ala Tyr Val Ala Val Asn Asp Ser Gly Trp His
   500 505 510
   Gln Leu Val Ser His Trp Leu Asn Thr His Ala Val Met Glu Pro Phe
           515                 520                 525
   Val Ile Ser Thr Asn Arg His Leu Ser Val Thr His Pro Val His Lys
       530                 535                 540
   Leu Leu Ser Pro His Tyr Arg Asp Thr Met Thr Ile Asn Ala Leu Ala
   545                 550                 555                 560
   Arg Gln Thr Leu Ile Asn Ala Gly Gly Ile Phe Glu Met Thr Val Phe
                   565                 570                 575
   Pro Gly Lys Phe Ala Leu Gly Met Ser Ala Val Val Tyr Lys Asp Trp
               580                 585                 590
   Lys Phe Thr Glu Gln Gly Leu Pro Asp Asp Leu Ile Lys Arg Gly Met
           595                 600                 605
   Ala Val Glu Asp Pro Ser Ser Pro Tyr Lys Val Arg Leu Leu Val Ser
       610                 615                 620
   Asp Tyr Pro Tyr Ala Ala Asp Gly Leu Ala Ile Trp His Ala Ile Glu
   625                 630                 635                 640
   Gln Tyr Val Ser Glu Tyr Leu Ala Ile Tyr Tyr Pro Asn Asp Gly Val
                   645                 650                 655
   Leu Gln Gly Asp Thr Glu Val Gln Ala Trp Trp Lys Glu Thr Arg Glu
               660                 665                 670
   Val Gly His Gly Asp Leu Lys Asp Ala Pro Trp Trp Pro Lys Met Gln
           675                 680                 685
   Ser Val Pro Glu Leu Ala Lys Ala Cys Thr Thr Ile Ile Trp Ile Gly
       690                 695                 700
   Ser Ala Leu His Ala Ala Val Asn Phe Gly Gln Tyr Pro Tyr Ala Gly
   705                 710                 715                 720
   Phe Leu Pro Asn Arg Pro Thr Val Ser Arg Arg Arg Met Pro Glu Pro
                   725                 730                 735
   Gly Thr Glu Glu Tyr Ala Glu Leu Glu Arg Asp Pro Glu Arg Ala Phe
               740                 745                 750
   Ile His Thr Ile Thr Ser Gln Ile Gln Thr Ile Ile Gly Val Ser Leu
           755                 760                 765
   Leu Glu Val Leu Ser Lys His Ser Ser Asp Glu Leu Tyr Leu Gly Gln
   770 775 780
   Arg Asp Thr Pro Glu Trp Thr Ser Asp Pro Lys Ala Leu Glu Val Phe
   785 790 795 800
   Lys Arg Phe Ser Asp Arg Leu Val Glu Ile Glu Ser Lys Val Val Gly
   805 810 815
   Met Asn His Asp Pro Glu Leu Lys Asn Arg Asn Gly Pro Ala Lys Phe
   820 825 830
   Pro Tyr Met Leu Leu Tyr Pro Asn Thr Ser Asp His Lys Gly Ala Ala
   835 840 845
   Ala Gly Leu Thr Ala Lys Gly Ile Pro Asn Ser Ile Ser Ile
   850 855 860
<210> 4
   <211> 665
   <212> PRT
   <213> Hordeum vulgare mutant D112
<400> 4
   Met Leu Leu Gly Gly Leu Ile Asp Thr Leu Thr Gly Ala Asn Lys Ser
   1 5 10 15
   Ala Arg Leu Lys Gly Thr Val Val Leu Met Arg Lys Asn Val Leu Asp
   20 25 30
   Leu Asn Asp Phe Gly Ala Thr Ile Ile Asp Gly Ile Gly Glu Phe Leu
   35 40 45
   Gly Lys Gly Val Thr Cys Gln Leu Ile Ser Ser Thr Ala Val Asp Gln
   50 55 60
   Asp Asn Gly Gly Arg Gly Lys Val Gly Ala Glu Ala Glu Leu Glu Gln
   65 70 75 80
   Trp Val Thr Ser Leu Pro Ser Leu Thr Thr Gly Glu Ser Lys Phe Gly
   85 90 95
   Leu Thr Phe Asp Trp Glu Val Glu Lys Leu Gly Val Pro Gly Ala Ile
   100 105 110
   Val Val Asn Asn Tyr His Ser Ser Glu Phe Leu Leu Lys Thr Ile Thr
   115 120 125
   Leu His Asp Val Pro Gly Arg Ser Gly Asn Leu Thr Phe Val Ala Asn
   130 135 140
   Ser Trp Ile Tyr Pro Ala Ala Asn Tyr Arg Tyr Ser Arg Val Phe Phe
   145 150 155 160
   Ala Asn Asp Thr Tyr Leu Pro Ser Gln Met Pro Ala Ala Leu Lys Pro
   165 170 175
   Tyr Arg Asp Asp Glu Leu Arg Asn Leu Arg Gly Asp Asp Gln Gln Gly
   180 185 190
   Pro Tyr Gln Glu His Asp Arg Ile Tyr Arg Tyr Asp Val Tyr Asn Asp
   195 200 205
   Leu Gly Glu Gly Arg Pro Ile Leu Gly Gly Asn Ser Asp His Pro Tyr
   210 215 220
   Pro Arg Arg Gly Arg Thr Glu Arg Lys Pro Asn Ala Ser Asp Pro Ser
   225 230 235 240
   Leu Glu Ser Arg Leu Ser Leu Leu Glu Gln Ile Tyr Val Pro Arg Asp
   245 250 255
   Glu Lys Phe Gly His Leu Lys Thr Ser Asp Phe Leu Gly Tyr Ser Ile
   260 265 270
   Lys Ala Ile Thr Gln Gly Ile Leu Pro Ala Val Arg Thr Tyr Val Asp
   275 280 285
   Thr Thr Pro Gly Glu Phe Asp Ser Phe Gln Asp Ile Ile Asn Leu Tyr
   290 295 300
   Glu Gly Gly Ile Lys Leu Pro Lys Val Ala Ala Leu Glu Glu Leu Arg
   305 310 315 320
   Lys Gln Phe Pro Leu Gln Leu Ile Lys Asp Leu Leu Pro Val Gly Gly
   325 330 335
   Asp Ser Leu Leu Lys Leu Pro Val Pro His Ile Ile Gln Glu Asn Lys
   340 345 350
   Gln Ala Trp Arg Thr Asp Glu Glu Phe Ala Arg Glu Val Leu Ala Gly
   355 360 365
   Val Asn Pro Val Met Ile Thr Arg Leu Thr Glu Phe Pro Pro Lys Ser
   370 375 380
   Ser Leu Asp Pro Ser Lys Phe Gly Asp His Thr Ser Thr Ile Thr Ala
   385 390 395 400
   Glu His Ile Glu Lys Asn Leu Glu Gly Leu Thr Val Gln Gln Ala Leu
   405 410 415
   Glu Ser Asn Arg Leu Tyr Ile Leu Asp His His Asp Arg Phe Met Pro
   420 425 430
   Phe Leu Ile Asp Val Asn Asn Leu Pro Gly Asn Phe Ile Tyr Ala Thr
   435 440 445
   Arg Thr Leu Phe Phe Leu Arg Gly Asp Gly Arg Leu Thr Pro Leu Ala
   450 455 460
   Ile Glu Leu Ser Glu Pro Ile Ile Gln Gly Gly Leu Thr Thr Ala Lys
   465 470 475 480
   Ser Lys Val Tyr Thr Pro Val Pro Ser Gly Ser Val Glu Gly Trp Val
   485 490 495
   Trp Glu Leu Ala Lys Ala Tyr Val Ala Val Asn Asp Ser Gly Trp His
   500 505 510
   Gln Leu Val Ser His Trp Leu Asn Thr His Ala Val Met Glu Pro Phe
   515 520 525
   Val Ile Ser Thr Asn Arg His Leu Ser Val Thr His Pro Val His Lys
   530 535 540
   Leu Leu Ser Pro His Tyr Arg Asp Thr Met Thr Ile Asn Ala Leu Ala
   545 550 555 560
   Arg Gln Thr Leu Ile Asn Ala Gly Gly Ile Phe Glu Met Thr Val Phe
   565 570 575
   Pro Gly Lys Phe Ala Leu Gly Met Ser Ala Val Val Tyr Lys Asp Trp
   580 585 590
   Lys Phe Thr Glu Gln Gly Leu Pro Asp Asp Leu Ile Lys Arg Gly Met
   595 600 605
   Ala Val Glu Asp Pro Ser Ser Pro Tyr Lys Val Arg Leu Leu Val Ser
   610 615 620
   Asp Tyr Pro Tyr Ala Ala Asp Gly Leu Ala Ile Trp His Ala Ile Glu
   625 630 635 640
   Gln Tyr Val Ser Glu Tyr Leu Ala Ile Tyr Tyr Pro Asn Asp Gly Val
   645 650 655
   Leu Gln Gly Asp Thr Glu Val Gln Ala
   660 665
<210> 5
   <211> 3229
   <212> DNA
   <213> Hordeum vulgare cv: Barke
<400> 5
   atgtttggcg tcggcggcat cgtgagcgac ctgacggggg gcctccgggg cgcccacctc     60
   aagggctccg tcgtcctcat gcgcaagaac gcgctcgact tcaacgactt cggcgccacc    120
   gtcatggacg gcgtcaccga gctcctcggc cgcggcgtca cctgccagct catcagctcc    180
   accaacgtcg accacagtga gcactcactc gccactcccc gttttgtaat ccctgccact    240
   gtgatacatg gaaaacggaa gcagatccgc atcctcacgc ccgaaccaag caaataatat    300
   atataaagaa ctaaaatgca cgtatggtta cggatgcatg cttatgcttg agcttgagct    360
   tgagcttgag agacagggac gtgcaaaaaa taacttaata atggagtaac taatgtgaga    420
   catgacgcac ggaggggttt accttactac taattaattg tcgagcagac aacggtgggc    480
   gcgggaaggt gggcgcggag gcgaacctgg agcagtggct cctgccgacg aacctgccgt    540
   tcatcaccac cggcgagaac aagttcgccg tcaccttcga ctggtcggtg gacaagctgg    600
   gggtgccggg ggccatcatc gtcaagaaca accacgcctc cgagttcttc ctcaagacca    660
   tcaccctcga caacgtgccc ggccgcggca ccatcgtctt cgtcgccaac tcatgggtct    720
   acccgcaggc caagtaccgc tacaaccgcg tcttcttcgc caacgacgtg agtattttat    780
   acgagtacca ctccatggta gctagtacga tggatttcgc ttgctcgatg cctgactggt    840
   cggttccgtt gggacatacg tgccgcagac gtacctgccg caccagatgc cggcggcgct    900
   gaagccgtac cgcgacgacg agctccggaa cctgaggggc gacgaccagc aggggcccta    960
   cctggaccac gaccgcgtct accgctacga cgtctacaac gacctcggcg actcccgcga   1020
   cgtcctcggc ggctccaagg acctccccta cccgcgccgc tgccgcaccg gccggaagcc   1080
   ctcggacagc agtgcgtgtc tcctcccttc tccttccttt cgatctcccc ataacgtgta   1140
   cttggtctga caagcatgtg tggccgacgc agagcccgac cacgagagcc ggctgctgcc   1200
   gctggtgcag aacgtctacg tgccgcgcga cgagctcttc ggccacctca agcagtcgga   1260
   cttcctgggc tacacgctca aggcgctggt ggacgggatc ataccggcca tccgcaccta   1320
   cgtcgacctc tcccccggcg agttcgactc cttcgccgac atcctcaagc tctacgaggg   1380
   cggcatcaag ctgcccaaca tcccggccct cgaggaggtg cgcaagcgct tcccgctcca 1440
   gctcgtcaag gacctcatcc ccaagggcgg cgacttcctc ctcaagctcc ccaagccgga 1500
   gatcatcaag gtagaccaga aagcgtggat gactgacgag gagttcgcca gggagatgct 1560
   cgccggcgtc aaccccatga tgatcaaacg cctcaccgtg agtgacccac tccatctacc 1620
   ggccattgaa caaaatcgtc catacatgtc actaatcaat actcacaccg ttttgaccgc 1680
   gtgtgcagga gttccctccc aagagcactc tggatccgag caagtacggc gaccacacca 1740
   gcaccatgac cgaggagcac gtggccaaga gcctggaggg cctcaccgtg cagcaggcgc 1800
   tcgccggcaa caggctctac atcgtagacc agcacgacaa cctgatgccg ttcctgatcg 1860
   acatcaacaa cctcgacgcc agcttcgtgt acgccacaag gacgctgctc ttcctgcgag 1920
   gggacggcac gctggcgccg gtcgccatcg agctgagctc gccgctgatc cagggcgagc 1980
   tgaccaccgc caagagcgcc gtgtacacgc cgcagcacgc cggcgtggag ggctggatat 2040
   ggcagctcgc caaggcctac gcctccgtga acgactacgg gtggcaccag ctcatcagcc 2100
   actggctcaa cacgcacgcc gtcatggagc ccttcgtcat cgccaccaac aggcagctca 2160
   gcgtcaccca cccggtctac aagctcctgc acccgcacta ccgcgacacc atgaacatca 2220
   acgcgcgggc gcgcgggctg ctcatcaacg ccggcggcgt catcgagatg accgtgttcc 2280
   cgcacaagca cgccatgccc atgtcctcca tggtctacaa gcactggaac ttcaccgaac 2340
   aagctctccc cgccgatcta atcaagaggt gcaacatgtt tacattatat aattgacgaa 2400
   acggtccttg atttgatcaa aatgattaat cgatcttgat ggttgatgat gatgtagggg 2460
   catggcggtg gaggacgcat cgagcccgca caaggtgcgg ctgctgatca aggactaccc 2520
   gtacgcgacc gacgggctgg ccgtgtggga cgccatcgag cagtgggtgt cggactacct 2580
   gaccatctac taccccaacg acggcgtgct gcagggcgac gtggagctgc aggcgtggtg 2640
   gaaggaggtg agggaggtcg ggcacggcga cctcaaggac gcggcgtggt ggccaaagat 2700
   gcagacggtg gcggagctga tcaaggcgtg cgccaccatc atctggaccg ggtcggcgct 2760
   ccacgcggcc gtcaacttcg ggcagtaccc ctactcgggc taccacccca acaagccgtc 2820
   ggcgagccgg aggccgatgc cggtgcaggg gagcgaggag tacgcggagc tggagcgaga 2880
   cccggagaag gccttcatcc gcaccatcac cagccagttc catgccctgg tgggcatctc 2940
   gctcatggag atcctctcca agcactcctc cgacgaggtc tacctgggcc agcacgacac 3000
   gccggcgtgg acgtcggacg ccaaggcgct ggaggcgttc aagcggttcg gggcgaagct 3060
   ggagggcatc gagaagcagg tggtggccat gaactcggac ccgcagctaa agaaccgcac 3120
   cgggccggcc aagttcccat acatgctgct ctacccaaac acctccgacc acacgggaca 3180
   ggccgagggg ctcaccgcca ggggcatccc gaacagcata tccatctga 3229
<210> 6
   <211> 3229
   <212> DNA
   <213> Hordeum vulgare Mutant A689
<400> 6
   atgtttggcg tcggcggcat cgtgagcgac ctgacggggg gcctccgggg cgcccacctc     60
   aagggctccg tcgtcctcat gcgcaagaac gcgctcgact tcaacgactt cggcgccacc    120
   gtcatggacg gcgtcaccga gctcctcggc cgcggcgtca cctgccagct catcagctcc    180
   accaacgtcg accacagtga gcactcactc gccactcccc gttttgtaat ccctgccact    240
   gtgatacatg gaaaacggaa gcagatccgc atcctcacgc ccgaaccaag caaataatat    300
   atataaagaa ctaaaatgca cgtatggtta cggatgcatg cttatgcttg agcttgagct    360
   tgagcttgag agacagggac gtgcaaaaaa taacttaata atggagtaac taatgtgaga    420
   catgacgcac ggaggggttt accttactac taattaattg tcgagcagac aacggtgggc   480
   gcgggaaggt gggcgcggag gcgaacctgg agcagtggct cctgccgacg aacctgccgt    540
   tcatcaccac cggcgagaac aagttcgccg tcaccttcga ctggtcggtg gacaagctgg    600
   gggtgccggg ggccatcatc gtcaagaaca accacgcctc cgagttcttc ctcaagacca    660
   tcaccctcga caacgtgccc ggccgcggca ccatcgtctt cgtcgccaac tcatgggtct    720
   acccgcaggc caagtaccgc tacaaccgcg tcttcttcgc caacgacgtg agtattttat    780
   acgagtacca ctccatggta gctagtacga tggatttcgc ttgctcgatg cctgactggt    840
   cggttccgtt gggacatacg tgccgcagac gtacctgccg caccagatgc cggcggcgct    900
   gaagccgtac cgcgacgacg agctccggaa cctgaggggc gacgaccagc aggggcccta    960
   cctggaccac gaccgcgtct accgctacga cgtctacaac gacctcggcg actcccgcga   1020
   cgtcctcggc ggctccaagg acctccccta cccgcgccgc tgccgcaccg gccggaagcc   1080
   ctcggacagc agtgcgtgtc tcctcccttc tccttccttt cgatctcccc ataacgtgta   1140
   cttggtctga caagcatgtg tggccgacgc agagcccgac cacgagagcc ggctgctgcc   1200
   gctggtgcag aacgtctacg tgccgcgcga cgagctcttc ggccacctca agcagtcgga   1260
   cttcctgggc tacacgctca aggcgctggt ggacgggatc ataccggcca tccgcaccta   1320
   cgtcgacctc tcccccggcg agttcgactc cttcgccgac atcctcaagc tctacgaggg   1380
   cggcatcaag ctgcccaaca tcccggccct cgaggaggtg cgcaagcgct tcccgctcca   1440
   gctcgtcaag gacctcatcc ccaagggcgg cgacttcctc ctcaagctcc ccaagccgga   1500
   gatcatcaag gtagaccaga aagcgtggat gactgacgag gagttcgcca gggagatgct   1560
   cgccggcgtc aaccccatga tgatcaaacg cctcaccgtg agtgacccac tccatctacc   1620
   ggccattgaa caaaatcgtc catacatgtc actaatcaat actcacaccg ttttgaccgc   1680
   gtgtgcagga gttccctccc aagagcactc tggatccgag caagtacggc gaccacacca 1740
   gcaccatgac cgaggagcac gtggccaaga gcctggaggg cctcaccgtg cagcaggcgc 1800
   tcgccggcaa caggctctac atcgtagacc agcacgacaa cctgatgccg ttcctgatcg 1860
   acatcaacaa cctcgacgcc agcttcgtgt acgccacaag gacgctgctc ttcctgcgag 1920
   gggacggcac gctggcgccg gtcgccatcg agctgagctc gccgctgatc cagggcgagc 1980
   tgaccaccgc caagagcgcc gtgtacacgc cgcagcacgc cggcgtggag ggctggatat 2040
   ggcagctcgc caaggcctac gcctccgtga acgactacgg gtggcaccag ctcatcagcc 2100
   actggctcaa cacgcacgcc gtcatggagc ccttcgtcat cgccaccaac aggcagctca 2160
   gcgtcaccca cccggtctac aagctcctgc acccgcacta ccgcgacacc atgaacatca 2220
   acgcgcgggc gcgcgggctg ctcatcaacg ccggcggcgt catcgagatg accgtgttcc 2280
   cgcacaagca cgccatgccc atgtcctcca tggtctacaa gcactggaac ttcaccgaac 2340
   aagctctccc cgccgatcta atcaagaggt gcaacatgtt tacattatat aattgacgaa 2400
   acggtccttg atttgatcaa aatgattaat cgatcttgat ggttgatgat gatgtagggg 2460
   catggcggtg gaggacgcat cgagcccgca caaggtgcgg ctgctgatca aggactaccc 2520
   gtacgcgacc gacgggctgg ccgtgtggga cgccatcgag cagtgggtgt cggactacct 2580
   gaccatctac taccccaacg acggcgtgct gcagggcgac gtggagctgc aggcgtggtg 2640
   gaaggaggtg agggaggtcg ggcacggcga cctcaaggac gcggcgtgat ggccaaagat 2700
   gcagacggtg gcggagctga tcaaggcgtg cgccaccatc atctggaccg ggtcggcgct 2760
   ccacgcggcc gtcaacttcg ggcagtaccc ctactcgggc taccacccca acaagccgtc 2820
   ggcgagccgg aggccgatgc cggtgcaggg gagcgaggag tacgcggagc tggagcgaga 2880
   cccggagaag gccttcatcc gcaccatcac cagccagttc catgccctgg tgggcatctc 2940
   gctcatggag atcctctcca agcactcctc cgacgaggtc tacctgggcc agcacgacac 3000
   gccggcgtgg acgtcggacg ccaaggcgct ggaggcgttc aagcggttcg gggcgaagct 3060
   ggagggcatc gagaagcagg tggtggccat gaactcggac ccgcagctaa agaaccgcac 3120
   cgggccggcc aagttcccat acatgctgct ctacccaaac acctccgacc acacgggaca 3180
   ggccgagggg ctcaccgcca ggggcatccc gaacagcata tccatctga 3229
<210> 7
   <211> 864
   <212> PRT
   <213> Hordeum vulgare cv: Barke
<400> 7
   Met Leu Gly Val Gly Gly Ile Val Ser Asp Leu Thr Gly Gly Ile Arg
   1               5                   10                  15
   Gly Ala His Leu Lys Gly Ser Val Val Leu Met Arg Lys Asn Ala Leu
   20 25 30
   Asp Phe Asn Asp Phe Gly Ala His Val Met Asp Gly Val Thr Glu Leu
   35 40 45
   Leu Gly Arg Gly Val Thr Cys Gln Leu Ile Ser Ser Thr Asn Val Asp
   50 55 60
   His Asn Asn Gly Gly Arg Gly Lys Val Gly Ala Glu Ala Asn Leu Glu
   65 70 75 80
   Gln Trp Leu Leu Pro Thr Asn Leu Pro Phe Ile Thr Thr Gly Glu Asn
   85 90 95
   Lys Phe Ala Val Thr Phe Asp Trp Ser Val Asp Lys Leu Gly Val Pro
   100 105 110
   Gly Ala Ile Ile Val Lys Asn Asn His Ala Ser Glu Phe Phe Leu Lys
   115 120 125
   Thr Ile Thr Leu Asp Asn Val Pro Gly Arg Gly Thr Ile Val Phe Val
   130 135 140
   Ala Asn Ser Trp Val Tyr Pro Gln Ala Lys Tyr Arg Tyr Asn Arg Val
   145 150 155 160
   Phe Phe Ala Asn Asp Thr Tyr Leu Pro His Gln Met Pro Ala Ala Leu
   165 170 175
   Lys Pro Tyr Arg Asp Asp Glu Leu Arg Asn Leu Arg Gly Asp Asp Gln
   180 185 190
   Gln Gly Pro Tyr Leu Asp His Asp Arg Val Tyr Arg Tyr Asp Val Tyr
   195 200 205
   Asn Asp Leu Gly Asp Ser Arg Asp Val Leu Gly Gly Ser Lys Asp Leu
   210 215 220
   Pro Tyr Pro Arg Arg Cys Arg Thr Gly Arg Lys Pro Ser Asp Ser Lys
   225 230 235 240
   Pro Asp His Glu Ser Arg Leu Leu Leu Leu Val Gln Asn Val Tyr Val
   245 250 255
   Leu Arg Asp Glu Leu Phe Gly His Leu Lys Gln Ser Asp Leu Leu Gly
   260 265 270
   Tyr Thr Leu Lys Gly Trp Leu Asp Gly Ile Ile Leu Ala Ile Arg Thr
           275                 280                 285
   Tyr Val Asp Leu Ser Pro Gly Glu Phe Asp Ser Phe Ala Asp Ile Leu
       290                 295                 300
   Lys Leu Tyr Glu Gly Gly Ile Lys Leu Pro Asn Ile Pro Ala Leu Glu
   305                 310                 315                 320
   Glu Val Arg Lys Arg Phe Pro Leu Gln Leu Val Lys Asp Leu Ile Pro
                   325                 330                 335
   Lys Gly Gly Asp Phe Leu Leu Lys Leu Pro Lys Pro Glu Ile Ile Lys
               340                 345                 350
   Val Asp Gln Lys Ala Trp Met Thr Asp Glu Glu Phe Ala Arg Glu Met
           355                 360                 365
   Leu Ala Gly Val Asn Pro Met Met Ile Lys Arg Leu Thr Glu Phe Pro
       370                 375                 380
   Pro Lys Ser Thr Leu Asp Pro Ser Lys Tyr Gly Asp His Thr Ser Thr
   385                 390                 395                 400
   Met Thr Glu Glu His Val Ala Lys Ser Leu Glu Gly Leu Thr Val Gln
                   405                 410                 415
   Gln Ala Leu Ala Gly Asn Arg Leu Tyr Ile Val Asp Gln His Asp Asn
               420                 425                 430
   Leu Met Pro Phe Leu Ile Asp Ile Asn Asn Leu Asp Ala Ser Phe Val
           435                 440                 445
   Tyr Ala Thr Arg Thr Leu Leu Phe Leu Arg Gly Asp Gly Thr Leu Ala
       450                 455                 460
   Pro Val Ala Ile Glu Leu Ser Ser Pro Leu Ile Gln Gly Glu Leu Thr
   465                 470                 475                 480
   Thr Ala Lys Ser Ala Val Tyr Thr Pro Gln His Ala Gly Val Glu Gly
                   485                 490                 495
   Trp Ile Trp Gln Leu Ala Lys Ala Tyr Ala Ser Val Asn Asp Tyr Gly
               500                 505                 510
   Trp His Gln Leu Ile Ser His Trp Leu Asn Thr His Ala Val Met Glu
   515 520 525
   Pro Phe Val Ile Ala Thr Asn Arg Gln Leu Ser Val Thr His Pro Val
   530 535 540
   Tyr Lys Leu Leu His Pro His Tyr Arg Asp Thr Met Asn Ile Asn Ala
   545 550 555 560
   Arg Ala Arg Gly Leu Leu Ile Asn Ala Gly Gly Val Ile Glu Met Thr
   565 570 575
   Val Phe Pro His Lys His Ala Met Pro Met Ser Ser Met Val Tyr Lys
   580 585 590
   His Trp Asn Phe Thr Glu Gln Ala Leu Pro Ala Asp Leu Ile Lys Arg
   595 600 605
   Gly Met Ala Val Glu Asp Ala Ser Ser Pro His Lys Val Arg Leu Leu
   610 615 620
   Ile Lys Asp Tyr Pro Tyr Ala Thr Asp Gly Leu Ala Val Trp Asp Ala
   625 630 635 640
   Ile Glu Gln Trp Val Ser Asp Tyr Leu Thr Ile Tyr Tyr Pro Asn Asp
   645 650 655
   Gly Val Leu Gln Gly Asp Val Glu Leu Gln Ala Trp Trp Lys Glu Val
   660 665 670
   Arg Glu Val Gly His Gly Asp Leu Lys Asp Ala Ala Trp Trp Pro Lys
   675 680 685
   Met Gln Thr Val Ala Glu Leu Ile Lys Ala Cys Ala Thr Ile Ile Trp
   690 695 700
   Thr Gly Ser Ala Leu His Ala Ala Val Asn Phe Gly Gln Tyr Pro Tyr
   705 710 715 720
   Ser Gly Tyr His Pro Asn Lys Pro Ser Ala Ser Arg Arg Pro Met Pro
   725 730 735
   Val Gln Gly Ser Glu Glu Tyr Ala Glu Leu Glu Arg Asp Pro Glu Lys
   740 745 750
   Ala Phe Ile Arg Thr Ile Thr Ser Gln Phe His Ala Leu Val Gly Ile
   755 760 765
   Ser Leu Met Glu Ile Leu Ser Lys His Ser Ser Asp Glu Val Tyr Leu
   770 775 780
   Gly Gln His Asp Thr Pro Ala Trp Thr Ser Asp Ala Lys Ala Leu Glu
   785 790 795 800
   Ala Phe Lys Arg Phe Gly Ala Lys Leu Glu Gly Ile Glu Lys Gln Val
   805 810 815
   Val Ala Met Asn Ser Asp Pro Gln Leu Lys Asn Arg Thr Gly Pro Ala
   820 825 830
   Lys Phe Pro Tyr Met Leu Leu Tyr Pro Asn Thr Ser Asp His Thr Gly
   835 840 845
   Gln Ala Glu Gly Leu Thr Ala Arg Gly Ile Pro Asn Ser Ile Ser Ile
   850 855 860
<210> 8
   <211> 684
   <212> PRT
   <213> Hordeum vulgare mutant A689
<400> 8
   Met Leu Gly Val Gly Gly Ile Val Ser Asp Leu Thr Gly Gly Ile Arg
   1 5 10 15
   Gly Ala His Leu Lys Gly Ser Val Val Leu Met Arg Lys Asn Ala Leu
   20 25 30
   Asp Phe Asn Asp Phe Gly Ala His Val Met Asp Gly Val Thr Glu Leu
   35 40 45
   Leu Gly Arg Gly Val Thr Cys Gln Leu Ile Ser Ser Thr Asn Val Asp
   50 55 60
   His Asn Asn Gly Gly Arg Gly Lys Val Gly Ala Glu Ala Asn Leu Glu
   65 70 75 80
   Gln Trp Leu Leu Pro Thr Asn Leu Pro Phe Ile Thr Thr Gly Glu Asn
   85 90 95
   Lys Phe Ala Val Thr Phe Asp Trp Ser Val Asp Lys Leu Gly Val Pro
   100 105 110
   Gly Ala Ile Ile Val Lys Asn Asn His Ala Ser Glu Phe Phe Leu Lys
   115 120 125
   Thr Ile Thr Leu Asp Asn Val Pro Gly Arg Gly Thr Ile Val Phe Val
       130                 135                 140
   Ala Asn Ser Trp Val Tyr Pro Gln Ala Lys Tyr Arg Tyr Asn Arg Val
   145                 150                 155                 160
   Phe Phe Ala Asn Asp Thr Tyr Leu Pro His Gln Met Pro Ala Ala Leu
                   165                 170                 175
   Lys Pro Tyr Arg Asp Asp Glu Leu Arg Asn Leu Arg Gly Asp Asp Gln
               180                 185                 190
   Gln Gly Pro Tyr Leu Asp His Asp Arg Val Tyr Arg Tyr Asp Val Tyr
           195                 200                 205
   Asn Asp Leu Gly Asp Ser Arg Asp Val Leu Gly Gly Ser Lys Asp Leu
       210                 215                 220
   Pro Tyr Pro Arg Arg Cys Arg Thr Gly Arg Lys Pro Ser Asp Ser Lys
   225                 230                 235                 240
   Pro Asp His Glu Ser Arg Leu Leu Leu Leu Val Gln Asn Val Tyr Val
                   245                 250                 255
   Leu Arg Asp Glu Leu Phe Gly His Leu Lys Gln Ser Asp Leu Leu Gly
               260                 265                 270
   Tyr Thr Leu Lys Gly Trp Leu Asp Gly Ile Ile Leu Ala Ile Arg Thr
           275                 280                 285
   Tyr Val Asp Leu Ser Pro Gly Glu Phe Asp Ser Phe Ala Asp Ile Leu
       290                 295                 300
   Lys Leu Tyr Glu Gly Gly Ile Lys Leu Pro Asn Ile Pro Ala Leu Glu
   305                 310                 315                 320
   Glu Val Arg Lys Arg Phe Pro Leu Gln Leu Val Lys Asp Leu Ile Pro
                   325                 330                 335
   Lys Gly Gly Asp Phe Leu Leu Lys Leu Pro Lys Pro Glu Ile Ile Lys
               340                 345                 350
   Val Asp Gln Lys Ala Trp Met Thr Asp Glu Glu Phe Ala Arg Glu Met
           355                 360                 365
   Leu Ala Gly Val Asn Pro Met Met Ile Lys Arg Leu Thr Glu Phe Pro
       370                 375                 380
   Pro Lys Ser Thr Leu Asp Pro Ser Lys Tyr Gly Asp His Thr Ser Thr
   385 390 395 400
   Met Thr Glu Glu His Val Ala Lys Ser Leu Glu Gly Leu Thr Val Gln
   405 410 415
   Gln Ala Leu Ala Gly Asn Arg Leu Tyr Ile Val Asp Gln His Asp Asn
   420 425 430
   Leu Met Pro Phe Leu Ile Asp Ile Asn Asn Leu Asp Ala Ser Phe Val
   435 440 445
   Tyr Ala Thr Arg Thr Leu Leu Phe Leu Arg Gly Asp Gly Thr Leu Ala
   450 455 460
   Pro Val Ala Ile Glu Leu Ser Ser Pro Leu Ile Gln Gly Glu Leu Thr
   465 470 475 480
   Thr Ala Lys Ser Ala Val Tyr Thr Pro Gln His Ala Gly Val Glu Gly
   485 490 495
   Trp Ile Trp Gln Leu Ala Lys Ala Tyr Ala Ser Val Asn Asp Tyr Gly
   500 505 510
   Trp His Gln Leu Ile Ser His Trp Leu Asn Thr His Ala Val Met Glu
   515 520 525
   Pro Phe Val Ile Ala Thr Asn Arg Gln Leu Ser Val Thr His Pro Val
   530 535 540
   Tyr Lys Leu Leu His Pro His Tyr Arg Asp Thr Met Asn Ile Asn Ala
   545 550 555 560
   Arg Ala Arg Gly Leu Leu Ile Asn Ala Gly Gly Val Ile Glu Met Thr
   565 570 575
   Val Phe Pro His Lys His Ala Met Pro Met Ser Ser Met Val Tyr Lys
   580 585 590
   His Trp Asn Phe Thr Glu Gln Ala Leu Pro Ala Asp Leu Ile Lys Arg
   595 600 605
   Gly Met Ala Val Glu Asp Ala Ser Ser Pro His Lys Val Arg Leu Leu
   610 615 620
   Ile Lys Asp Tyr Pro Tyr Ala Thr Asp Gly Leu Ala Val Trp Asp Ala
   625 630 635 640
   Ile Glu Gln Trp Val Ser Asp Tyr Leu Thr Ile Tyr Tyr Pro Asn Asp
                   645                 650                 655
   Gly Val Leu Gln Gly Asp Val Glu Leu Gln Ala Trp Trp Lys Glu Val
               660                 665                 670
   Arg Glu Val Gly His Gly Asp Leu Lys Asp Ala Ala
           675                 680
<210> 9
   <211> 6459
   <212> DNA
   <213> Barley cv. Prestige
<400> 9
   atggctgcgg cggcggggga cgtggaggcg ttcctggcgg cgtgccaggc gtcgggcgac      60
   gcggcgtacg gcgccgccaa ggccgtgctg gagcggctcg aggcgccggc cacgcgcgcc     120
   gaggccaggc ggctcctcgg cgccgtgcga cggcgcttcg ccgccggcgg cccggccgcg     180
   gggctcgagt gcttccgcac cttccacttc cgcatccacg acgtcgtcct cgacccccac     240
   ctccaaggtt gcccggcccc ttccctacac acccgttgtc gacccgcatc tctttcgccg     300
   atctggccgt caaaagcacg cggcttggta gaaatcaagc ctgcaatcct gatccgttta     360
   tggctggcca gtcgatcagt aatttggcca taactggagt ataaccttgg tctctaatct     420
   ctacctgacc atataccgag ttggttttct ttcttcttgt ttccgtattt gtgtagtttt     480
   ttcttttctt tcgagcatga tgttctttga attaatgcgt accagactcc agtaattcga     540
   cattttgaat tttggcgagt gttcttggaa tttataacac aacgaggctt tgatcaagtg     600
   gtttatgtag aggagtgttt ttgttcttgt gcaccgtata caattctcta tttcccaaca     660
   attttgatgg cctctaagca tcctgtagtc atgtctactg tgtaagctac agatttattc     720
   atgtctatgt gtaagctgca aatggagaga aaagctatct atttggttgt tccagcttgt     780
   tctttggcag aacaatcctg cccatcctat caccataagt ataaaagcac gacaaatgag     840
   tggggcaagc atgctgccaa gctaatacac gacataagct acatattttg aggggcatgt     900
   tatctttttt tttcccttct actcagtttc ttctttggga gaacaatcct actcaaccta     960
   taatcataag aataaaagca agacagatga gtgctgcaga ctattggcat atataacaac    1020
   taaataggac atctgtccgc tatatcttta gttaataatt gtatatagac gcagtctttg    1080
   tgctggaaaa actgcaacta aatattttct tacattatat ggaatctggg tgtgatatga    1140
   cttctttgtt acgttttgtg tgcataaagc attaacttct gtctttagtt ggcgcagcgg    1200
   taaaaacacc cattgcttaa tattttattt gctttccgta gcttgataaa atttcaactg    1260
   cttctaggat tccagcaaag aaagaagcta acaatgatgg agatacccag cattttcatt    1320
   ccagaagact ggtcattcac tttctacgag ggtctcaacc ggcatccaga ttccatcttc 1380
   agggataaga cagtagcaga gctgggatgt ggcaatggtt ggatatccat tgcacttgca 1440
   gaaaagtggt gcccttcgaa ggttggcacc tcttgttccg tagatattta tcttatctcg 1500
   tttgttgcaa acatgggacc tgcagaagtt agacatttac tcaggttact ttatatgaaa 1560
   cttttaggtg tctgccagta gtctgctggt ggtctaattt tcttggtata cctgatgccg 1620
   tcgagcatat tgctttcaaa ttttgggcaa ggcattacca ccacatattg tttctacaat 1680
   gctgaacaat tgctctcctt tgaaaggaag aaaaacaaga atgacatgca ccttagtagt 1740
   ttaagccaca aataccagcg aatcaaatta gtttgcagtc agcttggcat taccttactt 1800
   gagccttggt tgttcttttg aaggtttatg gtctggatat aaacccaaga gctatcaaga 1860
   ttgcatggat aaacctttac ttgaatgcac tagacgacga tggtctccca atctatgatg 1920
   cggaggggaa aacattgctt gacagagtcg aattctatga atctgatctt ctttcttact 1980
   gtagagataa caagatagaa cttgatcgca ttgttggatg cataccacag gtacggtcag 2040
   gtttttacca atttcctgtg aatggggatt atagtcgatc agaacttgat caaaatgccc 2100
   ttaatatctg cctttcagat tcttaacccc aatccagagg caatgtcaaa gattgtaact 2160
   gaaaattcaa gtgaggagtt cttgtactcc ttgagcaact actgtgctct ccaggtgagt 2220
   tgagatctat ttaaactcaa gccattcagt ttacctgtta ctaaatggtt acccatgtca 2280
   gagtctccaa atctttttct tttctcaaac agcaaagaga gaagaaaact tttaagttct 2340
   atcctgaaat tgactttaca atgcttgttc ataatctgct tacgaaatat gcgtttgaac 2400
   atttctcttt tccttgtagg catgtggtca gacctttata taagaaaatg aagtttttgt 2460
   agaaataatg tatgctttgt acttatgaca tggttccacc agtataatca atttaagtct 2520
   aggtagttag gaacctagga tggagagcac cgacagtgta taatatatat atgtcgatag 2580
   ggggttagca gtccaaatcc acctcaagtt caacctattg cataactttt ggtcttacaa 2640
   cctgtatgga caaatgtgat cagcacccca gtctttccta taaaaatgtc tgctggaata 2700
   tggaattatt aacagcggta tttattttta ccctgtttaa ttttttcctt tgctaaaaga 2760
   atgataatcc ttatgccacg aggttacatt gtattactca agtcaatatt tgttactatg 2820
   gctgattgta cgattccagc ttccggttgt taattttgtt atgtttgtga actttgctgc 2880
   attcagggtt ttgttgagga ccaatttggc ctcgggttga ttgctcgggc tgttgaagaa 2940
   gggatatctg tgataaagcc tagtggtctt atggtattca acatgggagg ccggccagga 3000
   caaggtgtct gtgagcgcct atttctacgc cgtggatttc gcatcaataa gctctggcaa 3060
   acaaaaatta tgcaggtagc aattctttga gtgactagat gttaactaat cccagtgttt 3120
   ttccatgcca gcaacagcat tatatcctgg ttagaggaat atgctcttca tgttgcacac 3180
   caatcttcag ctgggcctag aattttcatc taccggctta catttttaca ttacagaacc 3240
   aatttttgtt gaggatcatt accaactagt tgggtctttg caggctgctg acacagacat 3300
   ctccgcttta gttgaaattg agaaaaatag ccgacatcgc ttcgagttct ttatggatct 3360
   tgttggggat cagcctgtgt gtgcgcgcac agcatgggca tacatgaaat ctggtggccg 3420
   catttcacat gctttgtctg tgtatagctg tcaacttcgc cagcccaacc aggtacctat 3480
   actctctgat tagatcttta caacaataat atagtaatgt caggaataat aataatttgg 3540
   agaatttcag gtgaagaaaa tatttgagtt ccttaaagac ggattccatg aagtcagcag 3600
   ctccctcgat ttgtcctttg atgatgattc tgttgctgat gaaaaaattc ctttcctagc 3660
   atacctagct agtttcttgc aagagaataa gtctaatcct tgtgagcctc cagctggatg 3720
   tttaaatttc cggaatcttg ttgctggatt tatgaagagt taccaccaca tcccattaac 3780
   tcctgatgta agacttggtg tctattgcct acaattatgt ttgcttatta gaaattcata 3840
   agatcaacct atttgatgct tctcacgtat gcttcatgtg acacttcctt ttcctctggt 3900
   gcaccagaat gttgttgtgt tcccatcccg tgctgttgca atcgaaaatg ctcttcggtt 3960
   gttctcacct ggacttgcaa ttgttgacga acacctaacc agacacttgc ccaagcaatg 4020
   gttaacatct ttagcaattg aggtactttg accgatactc ccctctttct ttctgtgttt 4080
   ggaactgtgg aaaatacatg tgttctgtga agaaaaagtt atgctgacaa gaatttcgat 4140
   gttattgcca ttcttctaaa tttcaggaaa gtaaccatgc taaagataca gtaactgtaa 4200
   tcgaagcacc acgccaatca gatttgctga ttgagttgat caggaaactg aagcctcagg 4260
   ttgttgttac tggcatggct cagtttgagg ctatcaccag tgctgctttc gtgaacttat 4320
   taagtgtaac gaaagatgtt ggttcccgat tattactaga tatttcagaa catctggaat 4380
   tgtctagtct gccaagctca aatggtgtat tgaaatatct tgctgggaag accctgcctt 4440
   cacatgcggc tatattgtgt ggcttagtta agaatcaggt gtgtgtcaat cagcctgaac 4500
   tctagttgaa ctgttgtgca tactatatag aatatcttga cttttatatg tactttagaa 4560
   acactgttta aatgtactca tttctttttg cttcatttta cttgcaggtt tattctgatc 4620
   tggaagttgc ttttgctatc tctgaagatc caactgttta taaggcattg tcacaaacta 4680
   ttgagctatt ggaaggacat acttctgtga tcagccagca ctattatggt tgtcttttcc 4740
   atgagctgct ggcatttcaa attggtgacc ggcatccaca acaagaggta aacatggctt 4800
   gcctcttcca gttctccatc tcactcagtt ctgtccacaa ggtgccgaat gatctgttca 4860
   agtggacact cccctcagca cgggcaagct agtccatgaa tttggattag ttccctctta 4920
   gctgggtact tcgattacac cacaatgagc tcctcaacgt ggtctggttt atgtttttca 4980
   tgttttccct ctaatgtttg gttgctcttt ttcagagaga acctgcagaa gtgatatcta 5040
   aggagatgat agggttttca agttcagcta tgtccaccct agaaggagct gagtttttcg 5100
   ttcctggttc catggaatcc ggtgtcatac atatggatct ggaccgcagc ttcttgccag 5160
   taccttctgc agtaaacgcc tccattttcg aaagttttgt tcgtcagaac atcactgatt 5220
   ctgaaaccga tgtccgttcc agcattcagc agctggtgaa agatagctat ggtttctcag 5280
   caggcggtgc ttctgaaatt atatacggga acacctgtct cgcgctcttc aacaagcttg 5340
   ttctttgctg catgcaagaa cagggcacct tgcttttccc cttgggaacc aacgggcatt 5400
   acgtcaacgc agcaaagttt gtgaatgcaa ccaccttgac tattccaacg aaggcagatt 5460
   caggcttcaa gatcgaacca agtgctctag ccgacacact agagaaggtg tctcagccgt 5520
   gggtctatat ttctggcccc acaatcaacc ctactggctt cctgtacagt gacgacgata 5580
   tagcagagct gctttctgtc tgtgcgacat acggagccag ggtggtgata gatacctcct 5640
   cctctggtct ggagttccaa gccaccggct gcagccagtg gaatttggaa agatgtcttt 5700
   ctaatgtcaa gtcttcaaag ccctcgttct ccgttgtcct gctcggagag ctgtcctttg 5760
   agctgaccac ggctgggctt gatttcgggt ttctgattat gagcgactcg tccttggttg 5820
   acacatttta cagtttccca agcttgagtc ggccacacag cacgttgaag tacacgttca 5880
   ggaagctgtt gggtcttaag aaccagaagg atcagcattt ctctgatctc atccttgagc 5940
   agaaggagac gttgaagaat cgtgccgacc agttgatcaa ggtatgcctt ttgggatatc 6000
   ctgtgtttag gctctctgtt ttcttcccct gatcagctct ccgatcccct tacatcctta 6060
   ggctaatttc agtacttcaa gtttgccacg catttctgac atattctttc ctcttgtttt 6120
   attttcctgt gatgtgatga acagacgctt gagagctgcg gctgggacgc tgtgggctgc 6180
   catggcggca tctcgatgct tgcaaaaccg accgcctaca ttggcaaatc gctcaaggtg 6240
   gacggctttg agggcaagct ggacagccac aacatgaggg aagccctcct gaggtccacc 6300
   gggctgtgca ttagcagcag cgggtggaca ggggtgccgg actactgccg cttcagcttt 6360
   gctctggaga gcggcgactt cgaccgggcc atggagtgca tcgcccggtt cagggagctg 6420
   gtccttggtg gcggtgctaa ggtgaatggt agcaactag 6459
<210> 10
   <211> 6459
   <212> DNA
   <213> Barley, Mutant 8063
<400> 10
   atggctgcgg cggcggggga cgtggaggcg ttcctggcgg cgtgccaggc gtcgggcgac 60
   gcggcgtacg gcgccgccaa ggccgtgctg gagcggctcg aggcgccggc cacgcgcgcc 120
   gaggccaggc ggctcctcgg cgccgtgcga cggcgcttcg ccgccggcgg cccggccgcg 180
   gggctcgagt gcttccgcac cttccacttc cgcatccacg acgtcgtcct cgacccccac 240
   ctccaaggtt gcccggcccc ttccctacac acccgttgtc gacccgcatc tctttcgccg 300
   atctggccgt caaaagcacg cggcttggta gaaatcaagc ctgcaatcct gatccgttta 360
   tggctggcca gtcgatcagt aatttggcca taactggagt ataaccttgg tctctaatct 420
   ctacctgacc atataccgag ttggttttct ttcttcttgt ttccgtattt gtgtagtttt 480
   ttcttttctt tcgagcatga tgttctttga attaatgcgt accagactcc agtaattcga 540
   cattttgaat tttggcgagt gttcttggaa tttataacac aacgaggctt tgatcaagtg 600
   gtttatgtag aggagtgttt ttgttcttgt gcaccgtata caattctcta tttcccaaca 660
   attttgatgg cctctaagca tcctgtagtc atgtctactg tgtaagctac agatttattc 720
   atgtctatgt gtaagctgca aatggagaga aaagctatct atttggttgt tccagcttgt 780
   tctttggcag aacaatcctg cccatcctat caccataagt ataaaagcac gacaaatgag 840
   tggggcaagc atgctgccaa gctaatacac gacataagct acatattttg aggggcatgt 900
   tatctttttt tttcccttct actcagtttc ttctttggga gaacaatcct actcaaccta 960
   taatcataag aataaaagca agacagatga gtgctgcaga ctattggcat atataacaac 1020
   taaataggac atctgtccgc tatatcttta gttaataatt gtatatagac gcagtctttg 1080
   tgctggaaaa actgcaacta aatattttct tacattatat ggaatctggg tgtgatatga 1140
   cttctttgtt acgttttgtg tgcataaagc attaacttct gtctttagtt ggcgcagcgg 1200
   taaaaacacc cattgcttaa tattttattt gctttccgta gcttgataaa atttcaactg 1260
   cttctaggat tccagcaaag aaagaagcta acaatgatgg agatacccag cattttcatt 1320
   ccagaagact ggtcattcac tttctacgag ggtctcaacc ggcatccaga ttccatcttc 1380
   agggataaga cagtagcaga gctgggatgt ggcaatggtt ggatatccat tgcacttgca 1440
   gaaaagtggt gcccttcgaa ggttggcacc tcttgttccg tagatattta tcttatctcg 1500
   tttgttgcaa acatgggacc tgcagaagtt agacatttac tcaggttact ttatatgaaa 1560
   cttttaggtg tctgccagta gtctgctggt ggtctaattt tcttggtata cctgatgccg 1620
   tcgagcatat tgctttcaaa ttttgggcaa ggcattacca ccacatattg tttctacaat 1680
   gctgaacaat tgctctcctt tgaaaggaag aaaaacaaga atgacatgca ccttagtagt 1740
   ttaagccaca aataccagcg aatcaaatta gtttgcagtc agcttggcat taccttactt 1800
   gagccttggt tgttcttttg aaggtttatg gtctggatat aaacccaaga gctatcaaga 1860
   ttgcatggat aaacctttac ttgaatgcac tagacgacga tggtctccca atctatgatg 1920
   cggaggggaa aacattgctt gacagagtcg aattctatga atctgatctt ctttcttact 1980
   gtagagataa caagatagaa cttgatcgca ttgttggatg cataccacag gtacggtcag 2040
   gtttttacca atttcctgtg aatggggatt atagtcgatc agaacttgat caaaatgccc 2100
   ttaatatctg cctttcagat tcttaacccc aatccagagg caatgtcaaa gattgtaact 2160
   gaaaattcaa gtgaggagtt cttgtactcc ttgagcaact actgtgctct ccaggtgagt 2220
   tgagatctat ttaaactcaa gccattcagt ttacctgtta ctaaatggtt acccatgtca 2280
   gagtctccaa atctttttct tttctcaaac agcaaagaga gaagaaaact tttaagttct 2340
   atcctgaaat tgactttaca atgcttgttc ataatctgct tacgaaatat gcgtttgaac 2400
   atttctcttt tccttgtagg catgtggtca gacctttata taagaaaatg aagtttttgt 2460
   agaaataatg tatgctttgt acttatgaca tggttccacc agtataatca atttaagtct 2520
   aggtagttag gaacctagga tggagagcac cgacagtgta taatatatat atgtcgatag 2580
   ggggttagca gtccaaatcc acctcaagtt caacctattg cataactttt ggtcttacaa 2640
   cctgtatgga caaatgtgat cagcacccca gtctttccta taaaaatgtc tgctggaata 2700
   tggaattatt aacagcggta tttattttta ccctgtttaa ttttttcctt tgctaaaaga 2760
   atgataatcc ttatgccacg aggttacatt gtattactca agtcaatatt tgttactatg 2820
   gctgattgta cgattccagc ttccggttgt taattttgtt atgtttgtga actttgctgc 2880
   attcagggtt ttgttgagga ccaatttggc ctcgggttga ttgctcgggc tgttgaagaa 2940
   gggatatctg tgataaagcc tagtggtctt atggtattca acatgggagg ccggccagga 3000
   caaggtgtct gtgagcgcct atttctacgc cgtggatttc gcatcaataa gctctggcaa 3060
   acaaaaatta tgcagatagc aattctttga gtgactagat gttaactaat cccagtgttt 3120
   ttccatgcca gcaacagcat tatatcctgg ttagaggaat atgctcttca tgttgcacac 3180
   caatcttcag ctgggcctag aattttcatc taccggctta catttttaca ttacagaacc 3240
   aatttttgtt gaggatcatt accaactagt tgggtctttg caggctgctg acacagacat 3300
   ctccgcttta gttgaaattg agaaaaatag ccgacatcgc ttcgagttct ttatggatct 3360
   tgttggggat cagcctgtgt gtgcgcgcac agcatgggca tacatgaaat ctggtggccg 3420
   catttcacat gctttgtctg tgtatagctg tcaacttcgc cagcccaacc aggtacctat 3480
   actctctgat tagatcttta caacaataat atagtaatgt caggaataat aataatttgg 3540
   agaatttcag gtgaagaaaa tatttgagtt ccttaaagac ggattccatg aagtcagcag 3600
   ctccctcgat ttgtcctttg atgatgattc tgttgctgat gaaaaaattc ctttcctagc 3660
   atacctagct agtttcttgc aagagaataa gtctaatcct tgtgagcctc cagctggatg 3720
   tttaaatttc cggaatcttg ttgctggatt tatgaagagt taccaccaca tcccattaac 3780
   tcctgatgta agacttggtg tctattgcct acaattatgt ttgcttatta gaaattcata 3840
   agatcaacct atttgatgct tctcacgtat gcttcatgtg acacttcctt ttcctctggt 3900
   gcaccagaat gttgttgtgt tcccatcccg tgctgttgca atcgaaaatg ctcttcggtt 3960
   gttctcacct ggacttgcaa ttgttgacga acacctaacc agacacttgc ccaagcaatg 4020
   gttaacatct ttagcaattg aggtactttg accgatactc ccctctttct ttctgtgttt 4080
   ggaactgtgg aaaatacatg tgttctgtga agaaaaagtt atgctgacaa gaatttcgat 4140
   gttattgcca ttcttctaaa tttcaggaaa gtaaccatgc taaagataca gtaactgtaa 4200
   tcgaagcacc acgccaatca gatttgctga ttgagttgat caggaaactg aagcctcagg 4260
   ttgttgttac tggcatggct cagtttgagg ctatcaccag tgctgctttc gtgaacttat 4320
   taagtgtaac gaaagatgtt ggttcccgat tattactaga tatttcagaa catctggaat 4380
   tgtctagtct gccaagctca aatggtgtat tgaaatatct tgctgggaag accctgcctt 4440
   cacatgcggc tatattgtgt ggcttagtta agaatcaggt gtgtgtcaat cagcctgaac 4500
   tctagttgaa ctgttgtgca tactatatag aatatcttga cttttatatg tactttagaa 4560
   acactgttta aatgtactca tttctttttg cttcatttta cttgcaggtt tattctgatc 4620
   tggaagttgc ttttgctatc tctgaagatc caactgttta taaggcattg tcacaaacta 4680
   ttgagctatt ggaaggacat acttctgtga tcagccagca ctattatggt tgtcttttcc 4740
   atgagctgct ggcatttcaa attggtgacc ggcatccaca acaagaggta aacatggctt 4800
   gcctcttcca gttctccatc tcactcagtt ctgtccacaa ggtgccgaat gatctgttca 4860
   agtggacact cccctcagca cgggcaagct agtccatgaa tttggattag ttccctctta 4920
   gctgggtact tcgattacac cacaatgagc tcctcaacgt ggtctggttt atgtttttca 4980
   tgttttccct ctaatgtttg gttgctcttt ttcagagaga acctgcagaa gtgatatcta 5040
   aggagatgat agggttttca agttcagcta tgtccaccct agaaggagct gagtttttcg 5100
   ttcctggttc catggaatcc ggtgtcatac atatggatct ggaccgcagc ttcttgccag 5160
   taccttctgc agtaaacgcc tccattttcg aaagttttgt tcgtcagaac atcactgatt 5220
   ctgaaaccga tgtccgttcc agcattcagc agctggtgaa agatagctat ggtttctcag 5280
   caggcggtgc ttctgaaatt atatacggga acacctgtct cgcgctcttc aacaagcttg 5340
   ttctttgctg catgcaagaa cagggcacct tgcttttccc cttgggaacc aacgggcatt 5400
   acgtcaacgc agcaaagttt gtgaatgcaa ccaccttgac tattccaacg aaggcagatt 5460
   caggcttcaa gatcgaacca agtgctctag ccgacacact agagaaggtg tctcagccgt 5520
   gggtctatat ttctggcccc acaatcaacc ctactggctt cctgtacagt gacgacgata 5580
   tagcagagct gctttctgtc tgtgcgacat acggagccag ggtggtgata gatacctcct 5640
   cctctggtct ggagttccaa gccaccggct gcagccagtg gaatttggaa agatgtcttt 5700
   ctaatgtcaa gtcttcaaag ccctcgttct ccgttgtcct gctcggagag ctgtcctttg 5760
   agctgaccac ggctgggctt gatttcgggt ttctgattat gagcgactcg tccttggttg 5820
   acacatttta cagtttccca agcttgagtc ggccacacag cacgttgaag tacacgttca 5880
   ggaagctgtt gggtcttaag aaccagaagg atcagcattt ctctgatctc atccttgagc 5940
   agaaggagac gttgaagaat cgtgccgacc agttgatcaa ggtatgcctt ttgggatatc 6000
   ctgtgtttag gctctctgtt ttcttcccct gatcagctct ccgatcccct tacatcctta 6060
   ggctaatttc agtacttcaa gtttgccacg catttctgac atattctttc ctcttgtttt 6120
   attttcctgt gatgtgatga acagacgctt gagagctgcg gctgggacgc tgtgggctgc 6180
   catggcggca tctcgatgct tgcaaaaccg accgcctaca ttggcaaatc gctcaaggtg 6240
   gacggctttg agggcaagct ggacagccac aacatgaggg aagccctcct gaggtccacc 6300
   gggctgtgca ttagcagcag cgggtggaca ggggtgccgg actactgccg cttcagcttt 6360
   gctctggaga gcggcgactt cgaccgggcc atggagtgca tcgcccggtt cagggagctg 6420
   gtccttggtg gcggtgctaa ggtgaatggt agcaactag 6459
<210> 11
   <211> 6459
   <212> DNA
   <213> Barley cv. Sebastian
<400> 11
   atggctgcgg cggcggggga cgtggaggcg ttcctggcgg cgtgccaggc gtcgggcgac 60
   gcggcgtacg gcgccgccaa ggccgtgctg gagcggctcg aggcgccggc cacgcgcgcc 120
   gaggccaggc ggctcctcgg cgccgtgcga cggcgcttcg ccgccggcgg cccggccgcg 180
   gggctcgagt gcttccgcac cttccacttc cgcatccacg acgtcgtcct cgacccccac 240
   ctccaaggtt gcccggcccc ttccctacac acccgttgtc gacccgcatc tctttcgccg 300
   atctggccgt caaaagcacg cggcttggta gaaatcaagc ctgcaatcct gatccgttta 360
   tggctggcca gtcgatcagt aatttggcca taactggagt ataaccttgg tctctaatct 420
   ctacctgacc atataccgag ttggttttct ttcttcttgt ttccgtattt gtgtagtttt 480
   ttcttttctt tcgagcatga tgttctttga attaatgcgt accagactcc agtaattcga 540
   cattttgaat tttggcgagt gttcttggaa tttataacac aacgaggctt tgatcaagtg 600
   gtttatgtag aggagtgttt ttgttcttgt gcaccgtata caattctcta tttcccaaca 660
   attttgatgg cctctaagca tcctgtagtc atgtctactg tgtaagctac agatttattc 720
   atgtctatgt gtaagctgca aatggagaga aaagctatct atttggttgt tccagcttgt 780
   tctttggcag aacaatcctg cccatcctat caccataagt ataaaagcac gacaaatgag 840
   tggggcaagc atgctgccaa gctaatacac gacataagct acatattttg aggggcatgt 900
   tatctttttt tttcccttct actcagtttc ttctttggga gaacaatcct actcaaccta 960
   taatcataag aataaaagca agacagatga gtgctgcaga ctattggcat atataacaac 1020
   taaataggac atctgtccgc tatatcttta gttaataatt gtatatagac gcagtctttg 1080
   tgctggaaaa actgcaacta aatattttct tacattatat ggaatctggg tgtgatatga 1140
   cttctttgtt acgttttgtg tgcataaagc attaacttct gtctttagtt ggcgcagcgg 1200
   taaaaacacc cattgcttaa tattttattt gctttccgta gcttgataaa atttcaactg 1260
   cttctaggat tccagcaaag aaagaagcta acaatgatgg agatacccag cattttcatt 1320
   ccagaagact ggtcattcac tttctacgag ggtctcaacc ggcatccaga ttccatcttc 1380
   agggataaga cagtagcaga gctgggatgt ggcaatggtt ggatatccat tgcacttgca 1440
   gaaaagtggt gcccttcgaa ggttggcacc tcttgttccg tagatattta tcttatctcg 1500
   tttgttgcaa acatgggacc tgcagaagtt agacatttac tcaggttact ttatatgaaa 1560
   cttttaggtg tctgccagta gtctgctggt ggtctaattt tcttggtata cctgatgccg 1620
   tcgagcatat tgctttcaaa ttttgggcaa ggcattacca ccacatattg tttctacaat 1680
   gctgaacaat tgctctcctt tgaaaggaag aaaaacaaga atgacatgca ccttagtagt 1740
   ttaagccaca aataccagcg aatcaaatta gtttgcagtc agcttggcat taccttactt 1800
   gagccttggt tgttcttttg aaggtttatg gtctggatat aaacccaaga gctatcaaga 1860
   ttgcatggat aaacctttac ttgaatgcac tagacgacga tggtctccca atctatgatg 1920
   cggaggggaa aacattgctt gacagagtcg aattctatga atctgatctt ctttcttact 1980
   gtagagataa caagatagaa cttgatcgca ttgttggatg cataccacag gtacggtcag 2040
   gtttttacca atttcctgtg aatggggatt atagtcgatc agaacttgat caaaatgccc 2100
   ttaatatctg cctttcagat tcttaacccc aatccagagg caatgtcaaa gattgtaact 2160
   gaaaattcaa gtgaggagtt cttgtactcc ttgagcaact actgtgctct ccaggtgagt 2220
   tgagatctat ttaaactcaa gccattcagt ttacctgtta ctaaatggtt acccatgtca 2280
   gagtctccaa atctttttct tttctcaaac agcaaagaga gaagaaaact tttaagttct 2340
   atcctgaaat tgactttaca atgcttgttc ataatctgct tacgaaatat gcgtttgaac 2400
   atttctcttt tccttgtagg catgtggtca gacctttata taagaaaatg aagtttttgt 2460
   agaaataatg tatgctttgt acttatgaca tggttccacc agtataatca atttaagtct 2520
   aggtagttag gaacctagga tggagagcac cgacagtgta taatatatat atgtcgatag 2580
   ggggttagca gtccaaatcc acctcaagtt caacctattg cataactttt ggtcttacaa 2640
   cctgtatgga caaatgtgat cagcacccca gtctttccta taaaaatgtc tgctggaata 2700
   tggaattatt aacagcggta tttattttta ccctgtttaa ttttttcctt tgctaaaaga 2760
   atgataatcc ttatgccacg aggttacatt gtattactca agtcaatatt tgttactatg 2820
   gctgattgta cgattccagc ttccggttgt taattttgtt atgtttgtga actttgctgc 2880
   attcagggtt ttgttgagga ccaatttggc ctcgggttga ttgctcgggc tgttgaagaa 2940
   gggatatctg tgataaagcc tagtggtctt atggtattca acatgggagg ccggccagga 3000
   caaggtgtct gtgagcgcct atttctacgc cgtggatttc gcatcaataa gctctggcaa 3060
   acaaaaatta tgcaggtagc aattctttga gtgactagat gttaactaat cccagtgttt 3120
   ttccatgcca gcaacagcat tatatcctgg ttagaggaat atgctcttca tgttgcacac 3180
   caatcttcag ctgggcctag aattttcatc taccggctta catttttaca ttacagaacc 3240
   aatttttgtt gaggatcatt accaactagt tgggtctttg caggctgctg acacagacat 3300
   ctccgcttta gttgaaattg agaaaaatag ccgacatcgc ttcgagttct ttatggatct 3360
   tgttggggat cagcctgtgt gtgcgcgcac agcatgggca tacatgaaat ctggtggccg 3420
   catttcacat gctttgtctg tgtatagctg tcaacttcgc cagcccaacc aggtacctat 3480
   actctctgat tagatcttta caacaataat atagtaatgt caggaataat aataatttgg 3540
   agaatttcag gtgaagaaaa tatttgagtt ccttaaagac ggattccatg aagtcagcag 3600
   ctccctcgat ttgtcctttg atgatgattc tgttgctgat gaaaaaattc ctttcctagc 3660
   atacctagct agtttcttgc aagagaataa gtctaatcct tgtgagcctc cagctggatg 3720
   tttaaatttc cggaatcttg ttgctggatt tatgaagagt taccaccaca tcccattaac 3780
   tcctgatgta agacttggtg tctattgcct acaattatgt ttgcttatta gaaattcata 3840
   agatcaacct atttgatgct tctcacgtat gcttcatgtg acacttcctt ttcctctggt 3900
   gcaccagaat gttgttgtgt tcccatcccg tgctgttgca atcgaaaatg ctcttcggtt 3960
   gttctcacct ggacttgcaa ttgttgacga acacctaacc agacacttgc ccaagcaatg 4020
   gttaacatct ttagcaattg aggtactttg accgatactc ccctctttct ttctgtgttt 4080
   ggaactgtgg aaaatacatg tgttctgtga agaaaaagtt atgctgacaa gaatttcgat 4140
   gttattgcca ttcttctaaa tttcaggaaa gtaaccatgc taaagataca gtaactgtaa 4200
   tcgaagcacc acgccaatca gatttgctga ttgagttgat caggaaactg aagcctcagg 4260
   ttgttgttac tggcatggct cagtttgagg ctatcaccag tgctgctttc gtgaacttat 4320
   taagtgtaac gaaagatgtt ggttcccgat tattactaga tatttcagaa catctggaat 4380
   tgtctagtct gccaagctca aatggtgtat tgaaatatct tgctgggaag accctgcctt 4440
   cacatgcggc tatattgtgt ggcttagtta agaatcaggt gtgtgtcaat cagcctgaac 4500
   tctagttgaa ctgttgtgca tactatatag aatatcttga cttttatatg tactttagaa 4560
   acactgttta aatgtactca tttctttttg cttcatttta cttgcaggtt tattctgatc 4620
   tggaagttgc ttttgctatc tctgaagatc caactgttta taaggcattg tcacaaacta 4680
   ttgagctatt ggaaggacat acttctgtga tcagccagca ctattatggt tgtcttttcc 4740
   atgagctgct ggcatttcaa attggtgacc ggcatccaca acaagaggta aacatggctt 4800
   gcctcttcca gttctccatc tcactcagtt ctgtccacaa ggtgccgaat gatctgttca 4860
   agtggacact cccctcagca cgggcaagct agtccatgaa tttggattag ttccctctta 4920
   gctgggtact tcgattacac cacaatgagc tcctcaacgt ggtctggttt atgtttttca 4980
   tgttttccct ctaatgtttg gttgctcttt ttcagagaga acctgcagaa gtgatatcta 5040
   aggagatgat agggttttca agttcagcta tgtccaccct agaaggagct gagtttttcg 5100
   ttcctggttc catggaatcc ggtgtcatac atatggatct ggaccgcagc ttcttgccag 5160
   taccttctgc agtaaacgcc tccattttcg aaagttttgt tcgtcagaac atcactgatt 5220
   ctgaaaccga tgtccgttcc agcattcagc agctggtgaa agatagctat ggtttctcag 5280
   caggcggtgc ttctgaaatt atatacggga acacctgtct cgcgctcttc aacaagcttg 5340
   ttctttgctg catgcaagaa cagggcacct tgcttttccc cttgggaacc aacgggcatt 5400
   acgtcaacgc agcaaagttt gtgaatgcaa ccaccttgac tattccaacg aaggcagatt 5460
   caggcttcaa gatcgaacca agtgctctag ccgacacact agagaaggtg tctcagccgt 5520
   gggtctatat ttctggcccc acaatcaacc ctactggctt cctgtacagt gacgacgata 5580
   tagcagagct gctttctgtc tgtgcgacat acggagccag ggtggtgata gatacctcct 5640
   cctctggtct ggagttccaa gccaccggct gcagccagtg gaatttggaa agatgtcttt 5700
   ctaatgtcaa gtcttcaaag ccctcgttct ccgttgtcct gctcggagag ctgtcctttg 5760
   agctgaccac ggctgggctt gatttcgggt ttctgattat gagcgactcg tccttggttg 5820
   acacatttta cagtttccca agcttgagtc ggccacacag cacgttgaag tacacgttca 5880
   ggaagctgtt gggtcttaag aaccagaagg atcagcattt ctctgatctc atccttgagc 5940
   agaaggagac gttgaagaat cgtgccgacc agttgatcaa ggtatgcctt ttgggatatc 6000
   ctgtgtttag gctctctgtt ttcttcccct gatcagctct ccgatcccct tacatcctta 6060
   ggctaatttc agtacttcaa gtttgccacg catttctgac atattctttc ctcttgtttt 6120
   attttcctgt gatgtgatga acagacgctt gagagctgcg gctgggacgc tgtgggctgc 6180
   catggcggca tctcgatgct tgcaaaaccg accgcctaca ttggcaaatc gctcaaggtg 6240
   gacggctttg agggcaagct ggacagccac aacatgaggg aagccctcct gaggtccacc 6300
   gggctgtgca ttagcagcag cgggtggaca ggggtgccgg actactgccg cttcagcttt 6360
   gctctggaga gcggcgactt cgaccgggcc atggagtgca tcgcccggtt cagggagctg 6420
   gtccttggtg gcggtgctaa ggtgaatggt agcaactag 6459
<210> 12
   <211> 6459
   <212> DNA
   <213> Barley, Mutant 14018
<400> 12
   atggctgcgg cggcggggga cgtggaggcg ttcctggcgg cgtgccaggc gtcgggcgac 60
   gcggcgtacg gcgccgccaa ggccgtgctg gagcggctcg aggcgccggc cacgcgcgcc 120
   gaggccaggc ggctcctcgg cgccgtgcga cggcgcttcg ccgccggcgg cccggccgcg 180
   gggctcgagt gcttccgcac cttccacttc cgcatccacg acgtcgtcct cgacccccac 240
   ctccaaggtt gcccggcccc ttccctacac acccgttgtc gacccgcatc tctttcgccg 300
   atctggccgt caaaagcacg cggcttggta gaaatcaagc ctgcaatcct gatccgttta 360
   tggctggcca gtcgatcagt aatttggcca taactggagt ataaccttgg tctctaatct 420
   ctacctgacc atataccgag ttggttttct ttcttcttgt ttccgtattt gtgtagtttt 480
   ttcttttctt tcgagcatga tgttctttga attaatgcgt accagactcc agtaattcga 540
   cattttgaat tttggcgagt gttcttggaa tttataacac aacgaggctt tgatcaagtg 600
   gtttatgtag aggagtgttt ttgttcttgt gcaccgtata caattctcta tttcccaaca 660
   attttgatgg cctctaagca tcctgtagtc atgtctactg tgtaagctac agatttattc 720
   atgtctatgt gtaagctgca aatggagaga aaagctatct atttggttgt tccagcttgt 780
   tctttggcag aacaatcctg cccatcctat caccataagt ataaaagcac gacaaatgag 840
   tggggcaagc atgctgccaa gctaatacac gacataagct acatattttg aggggcatgt 900
   tatctttttt tttcccttct actcagtttc ttctttggga gaacaatcct actcaaccta 960
   taatcataag aataaaagca agacagatga gtgctgcaga ctattggcat atataacaac 1020
   taaataggac atctgtccgc tatatcttta gttaataatt gtatatagac gcagtctttg 1080
   tgctggaaaa actgcaacta aatattttct tacattatat ggaatctggg tgtgatatga 1140
   cttctttgtt acgttttgtg tgcataaagc attaacttct gtctttagtt ggcgcagcgg 1200
   taaaaacacc cattgcttaa tattttattt gctttccgta gcttgataaa atttcaactg 1260
   cttctaggat tccagcaaag aaagaagcta acaatgatgg agatacccag cattttcatt 1320
   ccagaagact ggtcattcac tttctacgag ggtctcaacc ggcatccaga ttccatcttc 1380
   agggataaga cagtagcaga gctgggatgt ggcaatggtt ggatatccat tgcacttgca 1440
   gaaaagtggt gcccttcgaa gattggcacc tcttgttccg tagatattta tcttatctcg 1500
   tttgttgcaa acatgggacc tgcagaagtt agacatttac tcaggttact ttatatgaaa 1560
   cttttaggtg tctgccagta gtctgctggt ggtctaattt tcttggtata cctgatgccg 1620
   tcgagcatat tgctttcaaa ttttgggcaa ggcattacca ccacatattg tttctacaat 1680
   gctgaacaat tgctctcctt tgaaaggaag aaaaacaaga atgacatgca ccttagtagt 1740
   ttaagccaca aataccagcg aatcaaatta gtttgcagtc agcttggcat taccttactt 1800
   gagccttggt tgttcttttg aaggtttatg gtctggatat aaacccaaga gctatcaaga 1860
   ttgcatggat aaacctttac ttgaatgcac tagacgacga tggtctccca atctatgatg 1920
   cggaggggaa aacattgctt gacagagtcg aattctatga atctgatctt ctttcttact 1980
   gtagagataa caagatagaa cttgatcgca ttgttggatg cataccacag gtacggtcag 2040
   gtttttacca atttcctgtg aatggggatt atagtcgatc agaacttgat caaaatgccc     2100
   ttaatatctg cctttcagat tcttaacccc aatccagagg caatgtcaaa gattgtaact     2160
   gaaaattcaa gtgaggagtt cttgtactcc ttgagcaact actgtgctct ccaggtgagt     2220
   tgagatctat ttaaactcaa gccattcagt ttacctgtta ctaaatggtt acccatgtca     2280
   gagtctccaa atctttttct tttctcaaac agcaaagaga gaagaaaact tttaagttct     2340
   atcctgaaat tgactttaca atgcttgttc ataatctgct tacgaaatat gcgtttgaac     2400
   atttctcttt tccttgtagg catgtggtca gacctttata taagaaaatg aagtttttgt     2460
   agaaataatg tatgctttgt acttatgaca tggttccacc agtataatca atttaagtct     2520
   aggtagttag gaacctagga tggagagcac cgacagtgta taatatatat atgtcgatag     2580
   ggggttagca gtccaaatcc acctcaagtt caacctattg cataactttt ggtcttacaa     2640
   cctgtatgga caaatgtgat cagcacccca gtctttccta taaaaatgtc tgctggaata     2700
   tggaattatt aacagcggta tttattttta ccctgtttaa ttttttcctt tgctaaaaga     2760
   atgataatcc ttatgccacg aggttacatt gtattactca agtcaatatt tgttactatg     2820
   gctgattgta cgattccagc ttccggttgt taattttgtt atgtttgtga actttgctgc     2880
   attcagggtt ttgttgagga ccaatttggc ctcgggttga ttgctcgggc tgttgaagaa     2940
   gggatatctg tgataaagcc tagtggtctt atggtattca acatgggagg ccggccagga     3000
   caaggtgtct gtgagcgcct atttctacgc cgtggatttc gcatcaataa gctctggcaa     3060
   acaaaaatta tgcaggtagc aattctttga gtgactagat gttaactaat cccagtgttt     3120
   ttccatgcca gcaacagcat tatatcctgg ttagaggaat atgctcttca tgttgcacac     3180
   caatcttcag ctgggcctag aattttcatc taccggctta catttttaca ttacagaacc     3240
   aatttttgtt gaggatcatt accaactagt tgggtctttg caggctgctg acacagacat     3300
   ctccgcttta gttgaaattg agaaaaatag ccgacatcgc ttcgagttct ttatggatct     3360
   tgttggggat cagcctgtgt gtgcgcgcac agcatgggca tacatgaaat ctggtggccg     3420
   catttcacat gctttgtctg tgtatagctg tcaacttcgc cagcccaacc aggtacctat     3480
   actctctgat tagatcttta caacaataat atagtaatgt caggaataat aataatttgg     3540
   agaatttcag gtgaagaaaa tatttgagtt ccttaaagac ggattccatg aagtcagcag     3600
   ctccctcgat ttgtcctttg atgatgattc tgttgctgat gaaaaaattc ctttcctagc     3660
   atacctagct agtttcttgc aagagaataa gtctaatcct tgtgagcctc cagctggatg     3720
   tttaaatttc cggaatcttg ttgctggatt tatgaagagt taccaccaca tcccattaac     3780
   tcctgatgta agacttggtg tctattgcct acaattatgt ttgcttatta gaaattcata     3840
   agatcaacct atttgatgct tctcacgtat gcttcatgtg acacttcctt ttcctctggt     3900
   gcaccagaat gttgttgtgt tcccatcccg tgctgttgca atcgaaaatg ctcttcggtt 3960
   gttctcacct ggacttgcaa ttgttgacga acacctaacc agacacttgc ccaagcaatg 4020
   gttaacatct ttagcaattg aggtactttg accgatactc ccctctttct ttctgtgttt 4080
   ggaactgtgg aaaatacatg tgttctgtga agaaaaagtt atgctgacaa gaatttcgat 4140
   gttattgcca ttcttctaaa tttcaggaaa gtaaccatgc taaagataca gtaactgtaa 4200
   tcgaagcacc acgccaatca gatttgctga ttgagttgat caggaaactg aagcctcagg 4260
   ttgttgttac tggcatggct cagtttgagg ctatcaccag tgctgctttc gtgaacttat 4320
   taagtgtaac gaaagatgtt ggttcccgat tattactaga tatttcagaa catctggaat 4380
   tgtctagtct gccaagctca aatggtgtat tgaaatatct tgctgggaag accctgcctt 4440
   cacatgcggc tatattgtgt ggcttagtta agaatcaggt gtgtgtcaat cagcctgaac 4500
   tctagttgaa ctgttgtgca tactatatag aatatcttga cttttatatg tactttagaa 4560
   acactgttta aatgtactca tttctttttg cttcatttta cttgcaggtt tattctgatc 4620
   tggaagttgc ttttgctatc tctgaagatc caactgttta taaggcattg tcacaaacta 4680
   ttgagctatt ggaaggacat acttctgtga tcagccagca ctattatggt tgtcttttcc 4740
   atgagctgct ggcatttcaa attggtgacc ggcatccaca acaagaggta aacatggctt 4800
   gcctcttcca gttctccatc tcactcagtt ctgtccacaa ggtgccgaat gatctgttca 4860
   agtggacact cccctcagca cgggcaagct agtccatgaa tttggattag ttccctctta 4920
   gctgggtact tcgattacac cacaatgagc tcctcaacgt ggtctggttt atgtttttca 4980
   tgttttccct ctaatgtttg gttgctcttt ttcagagaga acctgcagaa gtgatatcta 5040
   aggagatgat agggttttca agttcagcta tgtccaccct agaaggagct gagtttttcg 5100
   ttcctggttc catggaatcc ggtgtcatac atatggatct ggaccgcagc ttcttgccag 5160
   taccttctgc agtaaacgcc tccattttcg aaagttttgt tcgtcagaac atcactgatt 5220
   ctgaaaccga tgtccgttcc agcattcagc agctggtgaa agatagctat ggtttctcag 5280
   caggcggtgc ttctgaaatt atatacggga acacctgtct cgcgctcttc aacaagcttg 5340
   ttctttgctg catgcaagaa cagggcacct tgcttttccc cttgggaacc aacgggcatt 5400
   acgtcaacgc agcaaagttt gtgaatgcaa ccaccttgac tattccaacg aaggcagatt 5460
   caggcttcaa gatcgaacca agtgctctag ccgacacact agagaaggtg tctcagccgt 5520
   gggtctatat ttctggcccc acaatcaacc ctactggctt cctgtacagt gacgacgata 5580
   tagcagagct gctttctgtc tgtgcgacat acggagccag ggtggtgata gatacctcct 5640
   cctctggtct ggagttccaa gccaccggct gcagccagtg gaatttggaa agatgtcttt 5700
   ctaatgtcaa gtcttcaaag ccctcgttct ccgttgtcct gctcggagag ctgtcctttg 5760
   agctgaccac ggctgggctt gatttcgggt ttctgattat gagcgactcg tccttggttg 5820
   acacatttta cagtttccca agcttgagtc ggccacacag cacgttgaag tacacgttca 5880
   ggaagctgtt gggtcttaag aaccagaagg atcagcattt ctctgatctc atccttgagc 5940
   agaaggagac gttgaagaat cgtgccgacc agttgatcaa ggtatgcctt ttgggatatc 6000
   ctgtgtttag gctctctgtt ttcttcccct gatcagctct ccgatcccct tacatcctta 6060
   ggctaatttc agtacttcaa gtttgccacg catttctgac atattctttc ctcttgtttt 6120
   attttcctgt gatgtgatga acagacgctt gagagctgcg gctgggacgc tgtgggctgc 6180
   catggcggca tctcgatgct tgcaaaaccg accgcctaca ttggcaaatc gctcaaggtg 6240
   gacggctttg agggcaagct ggacagccac aacatgaggg aagccctcct gaggtccacc 6300
   gggctgtgca ttagcagcag cgggtggaca ggggtgccgg actactgccg cttcagcttt 6360
   gctctggaga gcggcgactt cgaccgggcc atggagtgca tcgcccggtt cagggagctg 6420
   gtccttggtg gcggtgctaa ggtgaatggt agcaactag 6459
<210> 13
   <211> 1088
   <212> PRT
   <213> Barley cv. Prestige
<400> 13
   Met Ala Ala Ala Ala Gly Asp Val Glu Ala Phe Leu Ala Ala Cys Gln
   1 5 10 15
   Ala Ser Gly Asp Ala Ala Tyr Gly Ala Ala Lys Ala Val Leu Glu Arg
   20 25 30
   Leu Glu Ala Pro Ala Thr Arg Ala Glu Ala Arg Arg Leu Leu Gly Ala
   35 40 45
   Val Arg Arg Arg Phe Ala Ala Gly Gly Pro Ala Ala Gly Leu Glu Cys
   50 55 60
   Phe Arg Thr Phe His Phe Arg Ile His Asp Val Val Leu Asp Pro His
   65 70 75 80
   Leu Gln Gly Phe Gln Gln Arg Lys Lys Leu Thr Met Met Glu Ile Pro
   85 90 95
   Ser Ile Phe Ile Pro Glu Asp Trp Ser Phe Thr Phe Tyr Glu Gly Leu
   100 105 110
   Asn Arg His Pro Asp Ser Ile Phe Arg Asp Lys Thr Val Ala Glu Leu
   115 120 125
   Gly Cys Gly Asn Gly Trp Ile Ser Ile Ala Leu Ala Glu Lys Trp Cys
   130 135 140
   Pro Ser Lys Val Tyr Gly Leu Asp Ile Asn Pro Arg Ala Ile Lys Ile
   145 150 155 160
   Ala Trp Ile Asn Leu Tyr Leu Asn Ala Leu Asp Asp Asp Gly Leu Pro
   165 170 175
   Ile Tyr Asp Ala Glu Gly Lys Thr Leu Leu Asp Arg Val Glu Phe Tyr
   180 185 190
   Glu Ser Asp Leu Leu Ser Tyr Cys Arg Asp Asn Lys Ile Glu Leu Asp
   195 200 205
   Arg Ile Val Gly Cys Ile Pro Gln Ile Leu Asn Pro Asn Pro Glu Ala
   210 215 220
   Met Ser Lys Ile Val Thr Glu Asn Ser Ser Glu Glu Phe Leu Tyr Ser
   225 230 235 240
   Leu Ser Asn Tyr Cys Ala Leu Gln Gly Phe Val Glu Asp Gln Phe Gly
   245 250 255
   Leu Gly Leu Ile Ala Arg Ala Val Glu Glu Gly Ile Ser Val Ile Lys
   260 265 270
   Pro Ser Gly Leu Met Val Phe Asn Met Gly Gly Arg Pro Gly Gln Gly
   275 280 285
   Val Cys Glu Arg Leu Phe Leu Arg Arg Gly Phe Arg Ile Asn Lys Leu
   290 295 300
   Trp Gln Thr Lys Ile Met Gln Ala Ala Asp Thr Asp Ile Ser Ala Leu
   305 310 315 320
   Val Glu Ile Glu Lys Asn Ser Arg His Arg Phe Glu Phe Phe Met Asp
   325 330 335
   Leu Val Gly Asp Gln Pro Val Cys Ala Arg Thr Ala Trp Ala Tyr Met
   340 345 350
   Lys Ser Gly Gly Arg Ile Ser His Ala Leu Ser Val Tyr Ser Cys Gln
   355 360 365
   Leu Arg Gln Pro Asn Gln Val Lys Lys Ile Phe Glu Phe Leu Lys Asp
   370 375 380
   Gly Phe His Glu Val Ser Ser Ser Leu Asp Leu Ser Phe Asp Asp Asp
   385 390 395 400
   Ser Val Ala Asp Glu Lys Ile Pro Phe Leu Ala Tyr Leu Ala Ser Phe
   405 410 415
   Leu Gln Glu Asn Lys Ser Asn Pro Cys Glu Pro Pro Ala Gly Cys Leu
   420 425 430
   Asn Phe Arg Asn Leu Val Ala Gly Phe Met Lys Ser Tyr His His Ile
   435 440 445
   Pro Leu Thr Pro Asp Asn Val Val Val Phe Pro Ser Arg Ala Val Ala
   450 455 460
   Ile Glu Asn Ala Leu Arg Leu Phe Ser Pro Gly Leu Ala Ile Val Asp
   465 470 475 480
   Glu His Leu Thr Arg His Leu Pro Lys Gln Trp Leu Thr Ser Leu Ala
   485 490 495
   Ile Glu Glu Ser Asn His Ala Lys Asp Thr Val Thr Val Ile Glu Ala
   500 505 510
   Pro Arg Gln Ser Asp Leu Leu Ile Glu Leu Ile Arg Lys Leu Lys Pro
   515 520 525
   Gln Val Val Val Thr Gly Met Ala Gln Phe Glu Ala Ile Thr Ser Ala
   530 535 540
   Ala Phe Val Asn Leu Leu Ser Val Thr Lys Asp Val Gly Ser Arg Leu
   545 550 555 560
   Leu Leu Asp Ile Ser Glu His Leu Glu Leu Ser Ser Leu Pro Ser Ser
   565 570 575
   Asn Gly Val Leu Lys Tyr Leu Ala Gly Lys Thr Leu Pro Ser His Ala
   580 585 590
   Ala Ile Leu Cys Gly Leu Val Lys Asn Gln Val Tyr Ser Asp Leu Glu
   595 600 605
   Val Ala Phe Ala Ile Ser Glu Asp Pro Thr Val Tyr Lys Ala Leu Ser
   610 615 620
   Gln Thr Ile Glu Leu Leu Glu Gly His Thr Ser Val Ile Ser Gln His
   625 630 635 640
   Tyr Tyr Gly Cys Leu Phe His Glu Leu Leu Ala Phe Gln Ile Gly Asp
   645 650 655
   Arg His Pro Gln Gln Glu Arg Glu Pro Ala Glu Val Ile Ser Lys Glu
   660 665 670
   Met Ile Gly Phe Ser Ser Ser Ala Met Ser Thr Leu Glu Gly Ala Glu
   675 680 685
   Phe Phe Val Pro Gly Ser Met Glu Ser Gly Val Ile His Met Asp Leu
   690 695 700
   Asp Arg Ser Phe Leu Pro Val Pro Ser Ala Val Asn Ala Ser Ile Phe
   705 710 715 720
   Glu Ser Phe Val Arg Gln Asn Ile Thr Asp Ser Glu Thr Asp Val Arg
   725 730 735
   Ser Ser Ile Gln Gln Leu Val Lys Asp Ser Tyr Gly Phe Ser Ala Gly
   740 745 750
   Gly Ala Ser Glu Ile Ile Tyr Gly Asn Thr Cys Leu Ala Leu Phe Asn
   755 760 765
   Lys Leu Val Leu Cys Cys Met Gln Glu Gln Gly Thr Leu Leu Phe Pro
   770 775 780
   Leu Gly Thr Asn Gly His Tyr Val Asn Ala Ala Lys Phe Val Asn Ala
   785 790 795 800
   Thr Thr Leu Thr Ile Pro Thr Lys Ala Asp Ser Gly Phe Lys Ile Glu
   805 810 815
   Pro Ser Ala Leu Ala Asp Thr Leu Glu Lys Val Ser Gln Pro Trp Val
   820 825 830
   Tyr Ile Ser Gly Pro Thr Ile Asn Pro Thr Gly Phe Leu Tyr Ser Asp
   835 840 845
   Asp Asp Ile Ala Glu Leu Leu Ser Val Cys Ala Thr Tyr Gly Ala Arg
   850 855 860
   Val Val Ile Asp Thr Ser Ser Ser Gly Leu Glu Phe Gln Ala Thr Gly
   865 870 875 880
   Cys Ser Gln Trp Asn Leu Glu Arg Cys Leu Ser Asn Val Lys Ser Ser
   885 890 895
   Lys Pro Ser Phe Ser Val Val Leu Leu Gly Glu Leu Ser Phe Glu Leu
   900 905 910
   Thr Thr Ala Gly Leu Asp Phe Gly Phe Leu Ile Met Ser Asp Ser Ser
   915 920 925
   Leu Val Asp Thr Phe Tyr Ser Phe Pro Ser Leu Ser Arg Pro His Ser
   930 935 940
   Thr Leu Lys Tyr Thr Phe Arg Lys Leu Leu Gly Leu Lys Asn Gln Lys
   945 950 955 960
   Asp Gln His Phe Ser Asp Leu Ile Leu Glu Gln Lys Glu Thr Leu Lys
   965 970 975
   Asn Arg Ala Asp Gln Leu Ile Lys Thr Leu Glu Ser Cys Gly Trp Asp
   980 985 990
   Ala Val Gly Cys His Gly Gly Ile Ser Met Leu Ala Lys Pro Thr Ala
   995 1000 1005
   Tyr Ile Gly Lys Ser Leu Lys Val Asp Gly Phe Glu Gly Lys Leu
   1010 1015 1020
   Asp Ser His Asn Met Arg Glu Ala Leu Leu Arg Ser Thr Gly Leu
   1025 1030 1035
   Cys Ile Ser Ser Ser Gly Trp Thr Gly Val Pro Asp Tyr Cys Arg
   1040 1045 1050
   Phe Ser Phe Ala Leu Glu Ser Gly Asp Phe Asp Arg Ala Met Glu
   1055 1060 1065
   Cys Ile Ala Arg Phe Arg Glu Leu Val Leu Gly Gly Gly Ala Lys
   1070 1075 1080
   Val Asn Gly Ser Asn
   1085
<210> 14
   <211> 1088
   <212> PRT
   <213> Barley cv. Sebastian
<400> 14
   Met Ala Ala Ala Ala Gly Asp Val Glu Ala Phe Leu Ala Ala Cys Gln
   1               5                   10                  15
   Ala Ser Gly Asp Ala Ala Tyr Gly Ala Ala Lys Ala Val Leu Glu Arg
               20                  25                  30
   Leu Glu Ala Pro Ala Thr Arg Ala Glu Ala Arg Arg Leu Leu Gly Ala
           35                  40                  45
   Val Arg Arg Arg Phe Ala Ala Gly Gly Pro Ala Ala Gly Leu Glu Cys
       50                  55                  60
   Phe Arg Thr Phe His Phe Arg Ile His Asp Val Val Leu Asp Pro His
   65                  70                  75                  80
   Leu Gln Gly Phe Gln Gln Arg Lys Lys Leu Thr Met Met Glu Ile Pro
                   85                  90                  95
   Ser Ile Phe Ile Pro Glu Asp Trp Ser Phe Thr Phe Tyr Glu Gly Leu
               100                 105                 110
   Asn Arg His Pro Asp Ser Ile Phe Arg Asp Lys Thr Val Ala Glu Leu
           115                 120                 125
   Gly Cys Gly Asn Gly Trp Ile Ser Ile Ala Leu Ala Glu Lys Trp Cys
       130                 135                 140
   Pro Ser Lys Val Tyr Gly Leu Asp Ile Asn Pro Arg Pro Ile Lys Ile
   145                 150                 155                 160
   Ala Trp Ile Asn Leu Tyr Leu Asn Ala Leu Asp Asp Asp Gly Leu Pro
                   165                 170                 175
   Ile Tyr Asp Ala Glu Gly Lys Thr Leu Leu Asp Arg Val Glu Phe Tyr
               180                 185                 190
   Glu Ser Asp Leu Leu Ser Tyr Cys Arg Asp Asn Lys Ile Glu Leu Asp
           195                 200                 205
   Arg Ile Val Gly Cys Ile Pro Gln Ile Leu Asn Pro Asn Pro Glu Ala
       210                 215                 220
   Met Ser Lys Ile Val Thr Glu Asn Ser Ser Glu Glu Phe Leu Tyr Ser
   225                 230                 235                 240
   Leu Ser Asn Tyr Cys Ala Leu Gln Gly Phe Val Glu Asp Gln Phe Gly
                   245                 250                 255
   Leu Gly Leu Ile Ala Arg Ala Val Glu Glu Gly Ile Ser Val Ile Lys
   260 265 270
   Pro Ser Gly Leu Met Val Phe Asn Met Gly Gly Arg Pro Gly Gln Gly
   275 280 285
   Val Cys Glu Arg Leu Phe Leu Arg Arg Gly Phe Arg Ile Asn Lys Leu
   290 295 300
   Trp Gln Thr Lys Ile Met Gln Ala Ala Asp Thr Asp Ile Ser Ala Leu
   305 310 315 320
   Val Glu Ile Glu Lys Asn Ser Arg His Arg Phe Glu Phe Phe Met Asp
   325 330 335
   Leu Val Gly Asp Gln Pro Val Cys Ala Arg Thr Ala Trp Ala Tyr Met
   340 345 350
   Lys Ser Gly Gly Arg Ile Ser His Ala Leu Ser Val Tyr Ser Cys Gln
   355 360 365
   Leu Arg Gln Pro Asn Gln Val Lys Lys Ile Phe Glu Phe Leu Lys Asp
   370 375 380
   Gly Phe His Glu Val Ser Ser Ser Leu Asp Leu Ser Phe Asp Asp Asp
   385 390 395 400
   Ser Val Ala Asp Glu Lys Ile Pro Phe Leu Ala Tyr Leu Ala Ser Phe
   405 410 415
   Leu Gln Glu Asn Lys Ser Asn Pro Cys Glu Pro Pro Ala Gly Cys Leu
   420 425 430
   Asn Phe Arg Asn Leu Val Ala Gly Phe Met Lys Ser Tyr His His Ile
   435 440 445
   Pro Leu Thr Pro Asp Asn Val Val Val Phe Pro Ser Arg Ala Val Ala
   450 455 460
   Ile Glu Asn Ala Leu Arg Leu Phe Ser Pro Gly Leu Ala Ile Val Asp
   465 470 475 480
   Glu His Leu Thr Arg His Leu Pro Lys Gln Trp Leu Thr Ser Leu Ala
   485 490 495
   Ile Glu Glu Ser Asn His Ala Lys Asp Thr Val Thr Val Ile Glu Ala
   500 505 510
   Pro Arg Gln Ser Asp Leu Leu Ile Glu Leu Ile Arg Lys Leu Lys Pro
   515 520 525
   Gln Val Val Val Thr Gly Met Ala Gln Phe Glu Ala Ile Thr Ser Ala
   530 535 540
   Ala Phe Val Asn Leu Leu Ser Val Thr Lys Asp Val Gly Ser Arg Leu
   545 550 555 560
   Leu Leu Asp Ile Ser Glu His Leu Glu Leu Ser Ser Leu Pro Ser Ser
   565 570 575
   Asn Gly Val Leu Lys Tyr Leu Ala Gly Lys Thr Leu Pro Ser His Ala
   580 585 590
   Ala Ile Leu Cys Gly Leu Val Lys Asn Gln Val Tyr Ser Asp Leu Glu
   595 600 605
   Val Ala Phe Ala Ile Ser Glu Asp Pro Thr Val Tyr Lys Ala Leu Ser
   610 615 620
   Gln Thr Ile Glu Leu Leu Glu Gly His Thr Ser Val Ile Ser Gln His
   625 630 635 640
   Tyr Tyr Gly Cys Leu Phe His Glu Leu Leu Ala Phe Gln Ile Gly Asp
   645 650 655
   Arg His Pro Gln Gln Glu Arg Glu Pro Ala Glu Val Ile Ser Lys Glu
   660 665 670
   Met Ile Gly Phe Ser Ser Ser Ala Met Ser Thr Leu Glu Gly Ala Glu
   675 680 685
   Phe Phe Val Pro Gly Ser Met Glu Ser Gly Val Ile His Met Asp Leu
   690 695 700
   Asp Arg Ser Phe Leu Pro Val Pro Ser Ala Val Asn Ala Ser Ile Phe
   705 710 715 720
   Glu Ser Phe Val Arg Gln Asn Ile Thr Asp Ser Glu Thr Asp Val Arg
   725 730 735
   Ser Ser Ile Gln Gln Leu Val Lys Asp Ser Tyr Gly Phe Ser Ala Gly
   740 745 750
   Gly Ala Ser Glu Ile Ile Tyr Gly Asn Thr Cys Leu Ala Leu Phe Asn
   755 760 765
   Lys Leu Val Leu Cys Cys Met Gln Glu Gln Gly Thr Leu Leu Phe Pro
       770                 775                 780
   Leu Gly Thr Asn Gly His Tyr Val Asn Ala Ala Lys Phe Val Asn Ala
   785                 790                 795                 800
   Thr Thr Leu Thr Ile Pro Thr Lys Ala Asp Ser Gly Phe Lys Ile Glu
                   805                 810                 815
   Pro Ser Ala Leu Ala Asp Thr Leu Glu Lys Val Ser Gln Pro Trp Val
               820                 825                 830
   Tyr Ile Ser Gly Pro Thr Ile Asn Pro Thr Gly Phe Leu Tyr Ser Asp
           835                 840                 845
   Asp Asp Ile Ala Glu Leu Leu Ser Val Cys Ala Thr Tyr Gly Ala Arg
       850                 855                 860
   Val Val Ile Asp Thr Ser Ser Ser Gly Leu Glu Phe Gln Ala Thr Gly
   865                 870                 875                 880
   Cys Ser Gln Trp Asn Leu Glu Arg Cys Leu Ser Asn Val Lys Ser Ser
                   885                 890                 895
   Lys Pro Ser Phe Ser Val Val Leu Leu Gly Glu Leu Ser Phe Glu Leu
               900                 905                 910
   Thr Thr Ala Gly Leu Asp Phe Gly Phe Leu Ile Met Ser Asp Ser Ser
           915                 920                 925
   Leu Val Asp Thr Phe Tyr Ser Phe Pro Ser Leu Ser Arg Pro His Ser
       930                 935                 940
   Thr Leu Lys Tyr Thr Phe Arg Lys Leu Leu Gly Leu Lys Asn Gln Lys
   945                 950                 955                 960
   Asp Gln His Phe Ser Asp Leu Ile Leu Glu Gln Lys Glu Thr Leu Lys
                   965                 970                 975
   Asn Arg Ala Asp Gln Leu Ile Lys Met Leu Glu Ser Cys Gly Trp Asp
               980                 985                 990
   Ala Val Gly Cys His Gly Gly Ile  Ser Met Leu Ala Lys  Pro Thr Ala
           995                 1000                1005
   Tyr Ile  Gly Lys Ser Leu Lys  Val Asp Gly Phe Glu  Gly Lys Leu
   1010 1015 1020
   Asp Ser His Asn Met Arg Glu Ala Leu Leu Arg Ser Thr Gly Leu
   1025 1030 1035
   Cys Ile Ser Ser Ser Gly Trp Thr Gly Val Pro Asp Tyr Cys Arg
   1040 1045 1050
   Phe Ser Phe Ala Leu Glu Ser Gly Asp Phe Asp Arg Ala Met Glu
   1055 1060 1065
   Cys Ile Ala Arg Phe Arg Glu Leu Val Leu Gly Gly Gly Ala Lys
   1070 1075 1080
   Val Asn Gly Ser Asn
   1085
<210> 15
   <211> 186
   <212> PRT
   <213> Barley, Mutant 14018
<400> 15
   Met Ala Ala Ala Ala Gly Asp Val Glu Ala Phe Leu Ala Ala Cys Gln
   1               5                   10                  15
   Ala Ser Gly Asp Ala Ala Tyr Gly Ala Ala Lys Ala Val Leu Glu Arg
               20                  25                  30
   Leu Glu Ala Pro Ala Thr Arg Ala Glu Ala Arg Arg Leu Leu Gly Ala
           35                  40                  45
   Val Arg Arg Arg Phe Ala Ala Gly Gly Pro Ala Ala Gly Leu Glu Cys
       50                  55                  60
   Phe Arg Thr Phe His Phe Arg Ile His Asp Val Val Leu Asp Pro His
   65                  70                  75                  80
   Leu Gln Gly Phe Gln Gln Arg Lys Lys Leu Thr Met Met Glu Ile Pro
                   85                  90                  95
   Ser Ile Phe Ile Pro Glu Asp Trp Ser Phe Thr Phe Tyr Glu Gly Leu
               100                 105                 110
   Asn Arg His Pro Asp Ser Ile Phe Arg Asp Lys Thr Val Ala Glu Leu
           115                 120                 125
   Gly Cys Gly Asn Gly Trp Ile Ser Ile Ala Leu Ala Glu Lys Trp Cys
       130                 135                 140
   Pro Ser Lys Ile Gly Thr Ser Cys Ser Val Asp Ile Tyr Leu Ile Ser
   145                 150                 155                 160
   Phe Val Ala Asn Met Gly Pro Ala Glu Val Arg His Leu Leu Arg Leu
                   165                 170                 175
   Leu Tyr Met Lys Leu Leu Gly Val Cys Gln
               180                 185
<210> 16
   <211> 180
   <212> PRT
   <213> Barley, Mutant 14018
<400> 16
   Met Ala Ala Ala Ala Gly Asp Val Glu Ala Phe Leu Ala Ala Cys Gln
   1               5                   10                  15
   Ala Ser Gly Asp Ala Ala Tyr Gly Ala Ala Lys Ala Val Leu Glu Arg
               20                  25                  30
   Leu Glu Ala Pro Ala Thr Arg Ala Glu Ala Arg Arg Leu Leu Gly Ala
           35                  40                  45
   Val Arg Arg Arg Phe Ala Ala Gly Gly Pro Ala Ala Gly Leu Glu Cys
       50                  55                  60
   Phe Arg Thr Phe His Phe Arg Ile His Asp Val Val Leu Asp Pro His
   65                  70                  75                  80
   Leu Gln Gly Phe Gln Gln Arg Lys Lys Leu Thr Met Met Glu Ile Pro
                   85                  90                  95
   Ser Ile Phe Ile Pro Glu Asp Trp Ser Phe Thr Phe Tyr Glu Gly Leu
               100                 105                 110
   Asn Arg His Pro Asp Ser Ile Phe Arg Asp Lys Thr Val Ala Glu Leu
           115                 120                 125
   Gly Cys Gly Asn Gly Trp Ile Ser Ile Ala Leu Ala Glu Lys Trp Cys
       130                 135                 140
   Pro Ser Lys Ile Gly Thr Ser Cys Ser Val Asp Ile Tyr Leu Ile Ser
   145                 150                 155                 160
   Phe Val Ala Asn Met Gly Pro Ala Glu Val Arg His Leu Leu Arg Phe
                   165                 170                 175
   Met Val Trp Ile
               180
<210> 17
   <211> 163
   <212> PRT
   <213> Barley, Mutant 14018
<400> 17
   Met Ala Ala Ala Ala Gly Asp Val Glu Ala Phe Leu Ala Ala Cys Gln
   1               5                   10                  15
   Ala Ser Gly Asp Ala Ala Tyr Gly Ala Ala Lys Ala Val Leu Glu Arg
               20                  25                  30
   Leu Glu Ala Pro Ala Thr Arg Ala Glu Ala Arg Arg Leu Leu Gly Ala
           35                  40                  45
   Val Arg Arg Arg Phe Ala Ala Gly Gly Pro Ala Ala Gly Leu Glu Cys
       50                  55                  60
   Phe Arg Thr Phe His Phe Arg Ile His Asp Val Val Leu Asp Pro His
   65                  70                  75                  80
   Leu Gln Gly Phe Gln Gln Arg Lys Lys Leu Thr Met Met Glu Ile Pro
                   85                  90                  95
   Ser Ile Phe Ile Pro Glu Asp Trp Ser Phe Thr Phe Tyr Glu Gly Leu
               100                 105                 110
   Asn Arg His Pro Asp Ser Ile Phe Arg Asp Lys Thr Val Ala Glu Leu
           115                 120                 125
   Gly Cys Gly Asn Gly Leu Trp Ser Gly Tyr Lys Pro Lys Ser Tyr Gln
       130                 135                 140
   Asp Cys Met Asp Lys Pro Leu Leu Glu Cys Thr Arg Arg Arg Trp Ser
   145                 150                 155                 160
   Pro Asn Leu
<210> 18
   <211> 315
   <212> PRT
   <213> Barley, Mutant 8063
<400> 18
   Met Ala Ala Ala Ala Gly Asp Val Glu Ala Phe Leu Ala Ala Cys Gln
   1 5 10 15
   Ala Ser Gly Asp Ala Ala Tyr Gly Ala Ala Lys Ala Val Leu Glu Arg
   20 25 30
   Leu Glu Ala Pro Ala Thr Arg Ala Glu Ala Arg Arg Leu Leu Gly Ala
   35 40 45
   Val Arg Arg Arg Phe Ala Ala Gly Gly Pro Ala Ala Gly Leu Glu Cys
   50 55 60
   Phe Arg Thr Phe His Phe Arg Ile His Asp Val Val Leu Asp Pro His
   65 70 75 80
   Leu Gln Gly Phe Gln Gln Arg Lys Lys Leu Thr Met Met Glu Ile Pro
   85 90 95
   Ser Ile Phe Ile Pro Glu Asp Trp Ser Phe Thr Phe Tyr Glu Gly Leu
   100 105 110
   Asn Arg His Pro Asp Ser Ile Phe Arg Asp Lys Thr Val Ala Glu Leu
   115 120 125
   Gly Cys Gly Asn Gly Trp Ile Ser Ile Ala Leu Ala Glu Lys Trp Cys
   130 135 140
   Pro Ser Lys Val Tyr Gly Leu Asp Ile Asn Pro Arg Ala Ile Lys Ile
   145 150 155 160
   Ala Trp Ile Asn Leu Tyr Leu Asn Ala Leu Asp Asp Asp Gly Leu Pro
   165 170 175
   Ile Tyr Asp Ala Glu Gly Lys Thr Leu Leu Asp Arg Val Glu Phe Tyr
   180 185 190
   Glu Ser Asp Leu Leu Ser Tyr Cys Arg Asp Asn Lys Ile Glu Leu Asp
   195 200 205
   Arg Ile Val Gly Cys Ile Pro Gln Ile Leu Asn Pro Asn Pro Glu Ala
   210 215 220
   Met Ser Lys Ile Val Thr Glu Asn Ser Ser Glu Glu Phe Leu Tyr Ser
   225 230 235 240
   Leu Ser Asn Tyr Cys Ala Leu Gln Gly Phe Val Glu Asp Gln Phe Gly
   245 250 255
   Leu Gly Leu Ile Ala Arg Ala Val Glu Glu Gly Ile Ser Val Ile Lys
   260 265 270
   Pro Ser Gly Leu Met Val Phe Asn Met Gly Gly Arg Pro Gly Gln Gly
   275 280 285
   Val Cys Glu Arg Leu Phe Leu Arg Arg Gly Phe Arg Ile Asn Lys Leu
   290 295 300
   Trp Gln Thr Lys Ile Met Gln Ile Ala Ile Leu
   305 310 315
<210> 19
   <211> 315
   <212> PRT
   <213> Barley, Mutant 8063
<400> 19
   Met Ala Ala Ala Ala Gly Asp Val Glu Ala Phe Leu Ala Ala Cys Gln
   1               5                   10                  15
   Ala Ser Gly Asp Ala Ala Tyr Gly Ala Ala Lys Ala Val Leu Glu Arg
               20                  25                  30
   Leu Glu Ala Pro Ala Thr Arg Ala Glu Ala Arg Arg Leu Leu Gly Ala
           35                  40                  45
   Val Arg Arg Arg Phe Ala Ala Gly Gly Pro Ala Ala Gly Leu Glu Cys
       50                  55                  60
   Phe Arg Thr Phe His Phe Arg Ile His Asp Val Val Leu Asp Pro His
   65                  70                  75                  80
   Leu Gln Gly Phe Gln Gln Arg Lys Lys Leu Thr Met Met Glu Ile Pro
                   85                  90                  95
   Ser Ile Phe Ile Pro Glu Asp Trp Ser Phe Thr Phe Tyr Glu Gly Leu
               100                 105                 110
   Asn Arg His Pro Asp Ser Ile Phe Arg Asp Lys Thr Val Ala Glu Leu
           115                 120                 125
   Gly Cys Gly Asn Gly Trp Ile Ser Ile Ala Leu Ala Glu Lys Trp Cys
       130                 135                 140
   Pro Ser Lys Val Tyr Gly Leu Asp Ile Asn Pro Arg Ala Ile Lys Ile
   145                 150                 155                 160
   Ala Trp Ile Asn Leu Tyr Leu Asn Ala Leu Asp Asp Asp Gly Leu Pro
                   165                 170                 175
   Ile Tyr Asp Ala Glu Gly Lys Thr Leu Leu Asp Arg Val Glu Phe Tyr
               180                 185                 190
   Glu Ser Asp Leu Leu Ser Tyr Cys Arg Asp Asn Lys Ile Glu Leu Asp
           195                 200                 205
   Arg Ile Val Gly Cys Ile Pro Gln Ile Leu Asn Pro Asn Pro Glu Ala
       210                 215                 220
   Met Ser Lys Ile Val Thr Glu Asn Ser Ser Glu Glu Phe Leu Tyr Ser
   225                 230                 235                 240
   Leu Ser Asn Tyr Cys Ala Leu Gln Gly Phe Val Glu Asp Gln Phe Gly
                   245                 250                 255
   Leu Gly Leu Ile Ala Arg Ala Val Glu Glu Gly Ile Ser Val Ile Lys
               260                 265                 270
   Pro Ser Gly Leu Met Val Phe Asn Met Gly Gly Arg Pro Gly Gln Gly
           275                 280                 285
   Val Cys Glu Arg Leu Phe Leu Arg Arg Gly Phe Arg Ile Asn Lys Leu
       290                 295                 300
   Trp Gln Thr Lys Ile Met Gln Ile Ala Ile Leu
   305                 310                 315
<210> 20
   <211> 289
   <212> PRT
   <213> Barley, Mutant 8063
<400> 20
   Met Ala Ala Ala Ala Gly Asp Val Glu Ala Phe Leu Ala Ala Cys Gln
   1               5                   10                  15
   Ala Ser Gly Asp Ala Ala Tyr Gly Ala Ala Lys Ala Val Leu Glu Arg
               20                  25                  30
   Leu Glu Ala Pro Ala Thr Arg Ala Glu Ala Arg Arg Leu Leu Gly Ala
           35                  40                  45
   Val Arg Arg Arg Phe Ala Ala Gly Gly Pro Ala Ala Gly Leu Glu Cys
   50 55 60
   Phe Arg Thr Phe His Phe Arg Ile His Asp Val Val Leu Asp Pro His
   65 70 75 80
   Leu Gln Gly Phe Gln Gln Arg Lys Lys Leu Thr Met Met Glu Ile Pro
   85 90 95
   Ser Ile Phe Ile Pro Glu Asp Trp Ser Phe Thr Phe Tyr Glu Gly Leu
   100 105 110
   Asn Arg His Pro Asp Ser Ile Phe Arg Asp Lys Thr Val Ala Glu Leu
   115 120 125
   Gly Cys Gly Asn Gly Trp Ile Ser Ile Ala Leu Ala Glu Lys Trp Cys
   130 135 140
   Pro Ser Lys Val Tyr Gly Leu Asp Ile Asn Pro Arg Ala Ile Lys Ile
   145 150 155 160
   Ala Trp Ile Asn Leu Tyr Leu Asn Ala Leu Asp Asp Asp Gly Leu Pro
   165 170 175
   Ile Tyr Asp Ala Glu Gly Lys Thr Leu Leu Asp Arg Val Glu Phe Tyr
   180 185 190
   Glu Ser Asp Leu Leu Ser Tyr Cys Arg Asp Asn Lys Ile Glu Leu Asp
   195 200 205
   Arg Ile Val Gly Cys Ile Pro Gln Ile Leu Asn Pro Asn Pro Glu Ala
   210 215 220
   Met Ser Lys Ile Val Thr Glu Asn Ser Ser Glu Glu Phe Leu Tyr Ser
   225 230 235 240
   Leu Ser Asn Tyr Cys Ala Leu Gln Gly Phe Val Glu Asp Gln Phe Gly
   245 250 255
   Leu Gly Leu Ile Ala Arg Ala Val Glu Glu Gly Ile Ser Val Ile Lys
   260 265 270
   Pro Ser Gly Leu Met Val Phe Asn Met Gly Gly Arg Pro Gly Gln Gly
   275 280 285
   Cys